# EUROPEAN PATENT APPLICATION

(11) **EP 3 693 377 A1**
(43) Date of publication of application: **12.08.2020**
(21) Application number: 18864861.2
(22) Date of filing: 03.10.2018
(51) Int. Cl.: C07K 1/06

(54) **METHOD FOR PRODUCING PEPTIDE COMPOUND**

(30) Priority: 03.10.2017 JP 2017193309; 17.07.2018 JP 2018134575; 26.09.2018 JP 2018180501
(71) Applicant: Nissan Chemical Corporation, Tokyo 103-6119 (JP); PeptiDream Inc., Kawasaki-shi, Kanagawa 210-0821 (JP)
(72) Inventor: NAGAYA, Akihiro, Funabashi-shi Chiba 274-8507 (JP); HANDA, Michiharu, Funabashi-shi Chiba 274-8507 (JP); YASUDA, Naohiko, Funabashi-shi Chiba 274-8507 (JP); YOSHINO, Madoka, Funabashi-shi Chiba 274-8507 (JP); KOBAYASHI, Yutaka, Kawasaki-shi Kanagawa 210-0821 (JP); MASUYA, Keiichi, Kawasaki-shi Kanagawa 210-0821 (JP)
(74) Representative: dompatent von Kreisler Selting Werner - Partnerschaft von Patent- und Rechtsanwälten mbB
(86) International application number: PCT/JP2018/037032
(87) International publication number: WO 2019/069978

(57) **Abstract**

An object of the present invention is to provide a method for producing a peptide with high efficiency.

A method for producing a peptide which comprises the following step (1) and (2).
(1) A step of condensing an N-protected amino acid or an N-protected peptide to an N-terminus of a C-protected amino acid or a C-protected peptide represented by the formula (II): 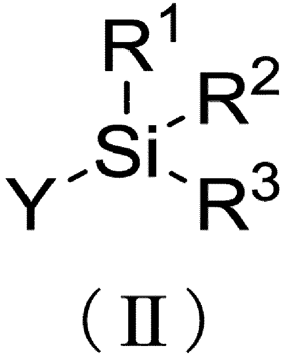 [wherein,
Y represents an amino acid an N-terminus of which is unprotected or a peptide an N-terminus of which is unprotected,
R¹, R² and R³ each independently represent an aliphatic hydrocarbon group which may have a substituent(s), an aromatic hydrocarbon group which may have a substituent(s) or -OR⁴ (R⁴ represents an aliphatic hydrocarbon group which may have a substituent(s) or an aromatic hydrocarbon group which may have a substituent(s).), two of R¹, R² and R³ may form a 5- to 7-membered ring together with the Si atom to which they are bonded,
a total number of the carbon atoms in R¹R²R³Si group is 8 or more, and
the R¹R²R³Si group is bonded to a C-terminus of an amino acid or a peptide in Y.].

(2) A step of removing the protective group at the N-terminus of the peptide obtained in the step (1).

## Description

### TECHNICAL FIELD

The present invention relates to a novel producing method of a peptide using a silyl protective group.

### BACKGROUND ART

A silyl protective group can be easily attachable/detachable as a protective group for a carboxy group, so that it has been used in many organic synthetic reactions (for example, see Non-Patent Document 1 and Patent Document 1).

Representative examples for use in peptide synthesis may be mentioned a method in which a C-terminal side is temporarily protected by a silyl protective group and an N-terminal side is bound to a solid phase support, and then, the silyl protective group is deprotected to elongate the peptide chain from the C-terminal side (for example, see Patent Document 1 and Patent Document 2).

Incidentally, in the method of elongating the peptide chain from the C-terminal side, it has been known that racemization proceeds due to formation of azlactone (oxazolone) when the carboxy group is activated (for example, see Non-Patent Document 2).

### PRIOR ART DOCUMENTS

### PATENT DOCUMENTS

Patent Document 1: JP Hei.4-502908A
Patent Document 2: WO 93/05065A

### NON-PATENT DOCUMENT

Non-Patent Document 1: Science of Synthesis, 2002, vol. 4, pp. 293-303
Non-Patent Document 2: The second series of Pharmaceutical Research and Development, 1991, vol. 14, pp. 3-10

### SUMMARY OF THE INVENTION

### PROBLEMS TO BE SOLVED BY THE INVENTION

An object of the present invention is to provide a novel method for producing a peptide using a silyl protective group at a C-terminal side and elongating the peptide chain from an N-terminal side.

The silyl protective group bonded to the C-terminal side used in the above-mentioned Patent Document 1 or Non-Patent Document 1, for example, a trimethylsilyl group or a t-butyldimethylsilyl group has been known to be easily deprotected in a solvent such as water and an alcohol, etc., so that it is not suitable for the method of elongating a peptide chain from an N-terminal side which is subjecting to a deprotection step of the protective group at the N-terminus or a purification step while maintaining the bonding of the C-terminus and the silyl protective group.

Also, the tri-t-butoxysilyl group bonded to the C-terminal side used in the above-mentioned Patent Document 2 has been known to be deprotected under weakly acidic conditions, and its use is limited in the peptide synthesis, in particular, it has not been investigated about a method of elongating a peptide chain from an N-terminal side which is subjecting to a deprotection step of the protective group at the N-terminus or a purification step while maintaining the bonding of the C-terminus and the silyl protective group.

### MEANS TO SOLVE THE PROBLEMS

Thus, the present inventors have intensively studied and as a result, they have found that the above-mentioned problems can be solved by using a silyl protective group having a specific structure whereby they have accomplished the present invention. That is, the present invention has the following characteristics.
[1] A method for producing an amino acid or a peptide which comprises the following step.
   A step of removing a protective group at an N-terminus of an amino acid or a peptide represented by the formula (I):

   **P-AA-O-SiR¹R²R³** **(I)**

   [wherein, AA represents a group derived from an amino acid or a peptide, P represents a protective group at the N-terminus, R¹R²R³Si represents a protective group at the C-terminus,
   R¹, R² and R³ each independently represent an aliphatic hydrocarbon group which may have a substituent(s), an aromatic hydrocarbon group which may have a substituent(s) or -OR⁴ (R⁴ represents an aliphatic hydrocarbon group which may have a substituent(s) or an aromatic hydrocarbon group which may have a substituent(s)),
   two of R¹, R² and R³ may form a 5- to 7-membered ring together with the Si atom to which they are bonded, and
   a total number of the carbon atoms in the R¹R²R³Si group is 8 or more.].
[2] A method for producing a peptide which comprises the following steps (1) and (2).
   (1) A step of condensing an N-protected amino acid or an N-protected peptide to an N-terminus of a C-protected amino acid or a C-protected peptide represented by the formula (II): 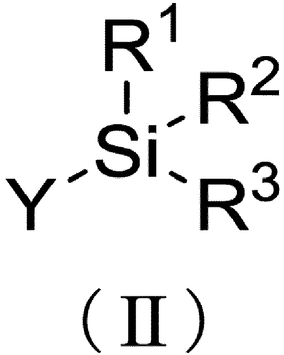 [wherein,
      Y represents an amino acid an N-terminus of which is unprotected or a peptide an N-terminus of which is unprotected,
      R¹, R² and R³ each independently represent an aliphatic hydrocarbon group which may have a substituent(s), an aromatic hydrocarbon group which may have a substituent(s) or -OR⁴ (R⁴ represents an aliphatic hydrocarbon group which may have a substituent(s) or an aromatic hydrocarbon group which may have a substituent(s).), two of R¹, R² and R³ may form a 5- to 7-membered ring together with the Si atom to which they are bonded,
      a total number of the carbon atoms in R¹R²R³Si group is 8 or more, and
      the R¹R²R³Si group is bonded to a C-terminus of an amino acid or a peptide in Y.].
   (2) A step of removing the protective group at the N-terminus of the peptide obtained in the step (1).
[3] The method for producing a peptide described in [2], which comprises the following steps (1) and (2).
   (1) A step of condensing an N-protected amino acid or an N-protected peptide to an N-terminus of a C-protected amino acid or a C-protected peptide represented by the formula (II): 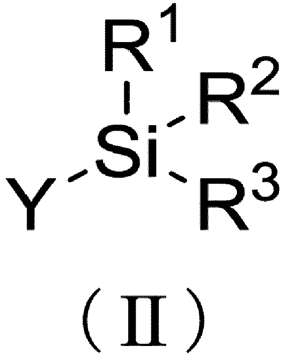 [wherein,
      Y represents an amino acid an N-terminus of which is unprotected or a peptide an N-terminus of which is unprotected,
      R¹, R² and R³ each independently represent an aliphatic hydrocarbon group which may have a substituent(s),
      a total number of the carbon atoms in R¹R²R³Si group is 8 or more, and the R¹R²R³Si group is bonded to a C-terminus of an amino acid or a peptide in Y.].
   (2) A step of removing the protective group at the N-terminus of the peptide obtained in the step (1).
[4] A method for producing a peptide which comprises the following steps (1) to (3).
   (1) A step of condensing an N-protected amino acid or an N-protected peptide to an N-terminus of a C-protected amino acid or a C-protected peptide represented by the formula (II): 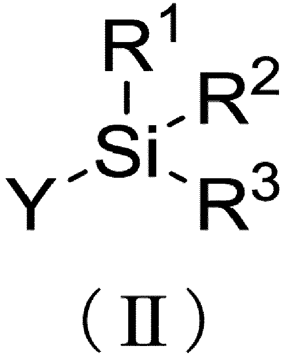 [wherein,
      Y represents an amino acid an N-terminus of which is unprotected or a peptide an N-terminus of which is unprotected,
      R¹, R² and R³ each independently represent an aliphatic hydrocarbon group which may have a substituent(s), an aromatic hydrocarbon group which may have a substituent(s) or -OR⁴ (R⁴ represents an aliphatic hydrocarbon group which may have a substituent(s) or an aromatic hydrocarbon group which may have a substituent(s).), two of R¹, R² and R³ may form a 5- to 7-membered ring together with the Si atom to which they are bonded,
      a total number of the carbon atoms in R¹R²R³Si group is 8 or more, and
      the R¹R²R³Si group is bonded to a C-terminus of an amino acid or a peptide in Y.].
   (2) A step of removing the protective group at the N-terminus of the peptide obtained in the step (1).
   (3) A step of condensing an N-protected amino acid or an N-protected peptide to the N-terminus of the peptide obtained in the step (2).
[5] The method for producing a peptide described in [4], which comprises the following steps (1) to (3).
   (1) A step of condensing an N-protected amino acid or an N-protected peptide to an N-terminus of a C-protected amino acid or a C-protected peptide represented by the formula (II): 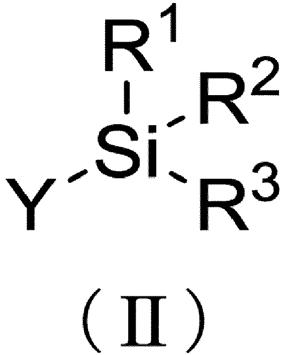 [wherein,
      Y represents an amino acid an N-terminus of which is unprotected or a peptide an N-terminus of which is unprotected,
      R¹, R² and R³ each independently represent an aliphatic hydrocarbon group which may have a substituent(s),
      a total number of the carbon atoms in R¹R²R³Si group is 8 or more, and the R¹R²R³Si group is bonded to a C-terminus of an amino acid or a peptide in Y.].
   (2) A step of removing the protective group at the N-terminus of the peptide obtained in the step (1).
   (3) A step of condensing an N-protected amino acid or an N-protected peptide to the N-terminus of the peptide obtained in the step (2).
[6] The method for producing a peptide described in [4] or [5], which comprises a step of removing the protective group at the C-terminus of the peptide obtained in the step (3).
[7] The method for producing a peptide described in [4] or [5], which further comprises one or more repeating of the following steps (4) and (5).
   (4) A step of removing the protective group at the N-terminus of the peptide obtained in the step (3) or the step (5).
   (5) A step of condensing an N-protected amino acid or an N-protected peptide to an N-terminus of the peptide obtained in the step (4).
[8] The method for producing a peptide described in [7], which comprises a step of removing the protective group at the C-terminus of the peptide obtained in the step (5).
[9] The producing method described in any one of [1] to [8], wherein a total number of carbon atoms in the R¹R²R³Si group is 10 to 100.
[10] The producing method described in any one of [1] to [8], wherein a total number of carbon atoms in the R¹R²R³Si group is 10 to 40.
[11] The producing method described in any one of [1] to [10], wherein among R¹, R² and R³, two or three of them each independently represent a secondary or tertiary aliphatic hydrocarbon group.
[12] The producing method described in [11], wherein among R¹, R² and R³, two of them each independently represent a secondary aliphatic hydrocarbon group, and the remaining one represents a secondary or tertiary aliphatic hydrocarbon group which may have a substituent(s) and a group different from the above two.
[13] The producing method described in [11], wherein among R¹, R² and R³, two of them each independently represent a tertiary aliphatic hydrocarbon group.
[14] The producing method described in any one of [1] to [10], wherein among R¹, R² and R³, two or three of them each independently represent a secondary or tertiary C₃₋₆ alkyl group or a C₃₋₆ cycloalkyl group.
[15] The producing method described in [14], wherein among R¹, R² and R³, two of them each independently represent a secondary C₃₋₆ alkyl group or a C₃₋₆ cycloalkyl group, and the remaining one represents a secondary or tertiary C₃₋₆ alkyl group or a C₃₋₆ cycloalkyl group, which may have a substituent(s), and is different from the above two.
[16] The producing method described in [14], wherein among R¹, R² and R³, two of them each independently represent a tertiary C₄₋₆ alkyl group.
[17] The producing method described in any one of [1] to [10], wherein among R¹, R² and R³, two or three of them each independently represent a t-butyl group, an i-propyl group, an s-butyl group, a cyclopentyl group or a cyclohexyl group.
[18] The producing method described in [17], wherein among R¹, R² and R³, one of them represents a t-butyl group or a cumyl group, and the remaining two are each independently represent a t-butyl group, an i-propyl group, an s-butyl group, a cyclopentyl group or a cyclohexyl group.
[19] The producing method described in [17], wherein among R¹, R² and R³, two of them are t-butyl groups, and the remaining one is an i-butyl group, a benzyl group, an octadecyl group or a (trimethylsilyl)methyl group.
[20] The producing method described in any one of [1] to [10], wherein the R¹R²R³Si group is a di-s-butyl-t-butylsilyl group, a di-t-butylisobutylsilyl group, a dit-butyloctadecylsilyl group, a benzyl-di-t-butylsilyl group, a tri-t-butylsilyl group, a di-i-propyl-t-butylsilyl group, a di-i-propylcumylsilyl group, a dicyclopentylcumylsilyl group, a di-cyclohexylcumylsilyl group, a di-s-butylcumylsilyl group or a di-t-butyl{(trimethylsilyl)methyl}silyl group.
[21] The producing method described in any one of [1], [2], [4] and [6] to [10], wherein the R¹R²R³Si group is a di-t-butylphenethoxysilyl group or a di-t-butylphenylsilyl group.
[22] The producing method described in any one of [1], [2], [4] and [6] to [10], wherein R¹, R² and R³ are -OR⁴ (R⁴ represents a tertiary aliphatic hydrocarbon group.).
[23] The producing method described in any one of [1], [2], [4] and [6] to [10], wherein the R¹R²R³Si group is a tri-t-butoxysilyl group.
[24] The producing method described in any one of [1] to [23], wherein the amino acid or the peptide is constituted by an α-amino acid.
[25] The producing method described in any one of [1] to [24], wherein the protective group at the N-terminus of the N-protected amino acid or the N-protected peptide is a carbamate protective group.
[26] The producing method described in any one of [1] to [24], wherein the protective group at the N-terminus of the N-protected amino acid or the N-protected peptide is a benzyloxycarbonyl group, a 9-fluorenylmethoxycarbonyl group or a t-butoxycarbonyl group.
[27] Use of a group represented by the following formula (III): 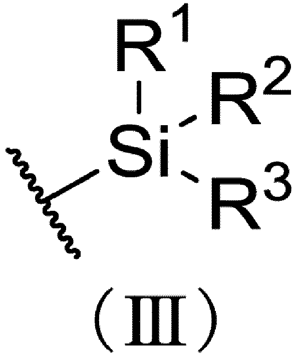 [wherein, R¹, R² and R³ each independently represent an aliphatic hydrocarbon group which may have a substituent(s), an aromatic hydrocarbon group which may have a substituent(s) or -OR⁴ (R⁴ represents an aliphatic hydrocarbon group which may have a substituent(s), or an aromatic hydrocarbon group which may have a substituent(s).), two of R¹, R² and R³ may form a 5- to 7-membered ring together with the Si atom to which they are bonded, and a total number of the carbon atoms in R¹R²R³Si group is 8 or more, and a wavy line is a bonding position to a residue at a C-terminus of the amino acid or the peptide]
   as a protective group of a C-terminus of an amino acid or a peptide for an N-terminal elongation reaction of the peptide.

### EFFECTS OF THE INVENTION

According to the present invention, a novel method for producing a peptide using a silyl protective group could be provided.

### EMBODIMENTS TO CARRY OUT THE INVENTION

In the following, the present invention will be explained in detail.

In the present specification, "n-" means normal, "i-" means iso, "s-" and "sec-" mean secondary, "t-" and "tert-" mean tertiary, "c-" means cyclo, "p-" means para, "Bu" means butyl, "Pr" means propyl, "Pen" means pentyl, "Hex" means hexyl, "Bn" means benzyl, "Ph" means phenyl, "Boc" means t-butoxycarbonyl, "Cbz" means benzyloxycarbonyl, "Fmoc" means 9-fluorenylmethoxycarbonyl, "Ts" means p-toluenesulfonyl, "Trt" means trityl, "Ac" means acetyl, "Tf" means trifluoromethanesulfonyl, "TMS" means trimethylsilyl, "TBS" means t-butyldimethylsilyl, "TIPS" means triisopropylsilyl, "BIBS" means di-t-butylisobutylsilyl, "IPBS" means di-i-propyl-t-butylsilyl, "IPCS" means di-i-propylcumylsilyl, "CPCS" means di-cyclopentylcumylsilyl, "CHCS" means di-cyclohexylcumylsilyl, and "SBCS" means disec-butylcumylsilyl. Incidentally, "t-Bu" and "tBu" both mean "tertiary butyl", and "s-Bu" and "sBu" both mean "secondary butyl".

In the present specification, the term "C₁₈H₃₇" or "octadecyl" means a linear octadecyl group, i.e., a group having a structure of CH₃(CH₂)₁₇-, otherwise specifically explained.

The "halogen atom" means a fluorine atom, a chlorine atom, a bromine atom or an iodine atom.

The "C₁₋₆ alkyl group" means a linear or branched alkyl group having 1 to 6 carbon atoms, and specific examples may be mentioned a methyl group, an ethyl group, an n-propyl group, an isopropyl group, an n-butyl group, an isobutyl group, an s-butyl group, a t-butyl group, an n-pentyl group, an n-hexyl group, etc. Also, the "C₃₋₆ alkyl group" means a linear or branched alkyl group having 3 to 6 carbon atoms, and the "C₄₋₆ alkyl group" means the same having 4 to 6 carbon atoms.

The "C₄₋₄₀ alkyl group" means a linear or branched alkyl group having 1 to 40 carbon atoms, and specific examples may be mentioned a methyl group, an ethyl group, an n-propyl group, an isopropyl group, an n-butyl group, an isobutyl group, an s-butyl group, a t-butyl group, an n-pentyl group, an n-hexyl group, an octyl group, a decyl group, a dodecyl group, a hexadecyl group, an octadecyl group, a docosyl group, a triacontyl group, a tetracontyl group, a 3,7,11,15-tetramethylhexadecyl group (hereinafter sometimes referred to as a 2,3-dihydrophytyl group.), etc.

The "C₁₋₆ alkoxy group" means a linear or branched alkoxy group having 1 to 6 carbon atoms, and specific examples may be mentioned a methoxy group, an ethoxy group, an n-propoxy group, an isopropoxy group, an n-butoxy group, an isobutoxy group, a t-butoxy group, an n-pentyloxy group, an n-hexyloxy group, etc.

The "C₁₋₄₀ alkoxy group" means a linear or branched alkoxy group having 1 to 40 carbon atoms, and specific examples may be mentioned a methoxy group, an ethoxy group, an n-propoxy group, an isopropoxy group, an n-butoxy group, an isobutoxy group, a t-butoxy group, an n-pentyloxy group, an n-hexyloxy group, an octyloxy group, a decyloxy group, a dodecyloxy group, a hexadecyloxy group, an octadecyloxy group, a docosyloxy group, a triacontyloxy group, a tetracontyloxy group, a 3,7,11,15-tetramethylhexadecyloxy group (hereinafter sometimes referred to as a 2,3-dihydrophytyloxy group.), etc.

The "C₁₋₆ alkoxycarbonyl group" means a linear or branched alkoxycarbonyl group having 1 to 6 carbon atoms, and specific examples may be mentioned a methoxycarbonyl group, an ethoxycarbonyl group, an n-propoxycarbonyl group, an isopropoxycarbonyl group, an n-butoxycarbonyl group, an isobutoxycarbonyl group, a t-butoxycarbonyl group, an n-pentyloxycarbonyl group, an n-hexyloxycarbonyl group, etc.

The "C₂₋₆ alkenyl group" means a linear or branched alkenyl group having 2 to 6 carbon atoms, and specific examples may be mentioned a vinyl group, a 1-propenyl group, an allyl group, an isopropenyl group, a butenyl group, an isobutenyl group, etc.

The "C₂₋₆ alkynyl group" means a linear or branched alkynyl group having 2 to 6 carbon atoms, and specific examples may be mentioned an ethynyl group, a 1-propynyl group, etc.

The "C₃₋₆ cycloalkyl group" means a cycloalkyl group having 3 to 6 carbon atoms, and specific examples may be mentioned a cyclopropyl group, a cyclobutyl group, a cyclopentyl group, a cyclohexyl group, etc.

The "C₃₋₆ cycloalkoxy group" means a cycloalkoxy group having 3 to 6 carbon atoms, and specific examples may be mentioned a cyclopropoxy group, a cyclobutoxy group, a cyclopentyloxy group, a cyclohexyloxy group, etc.

The "C₆₋₁₄ aryl group" means an aromatic hydrocarbon group having 6 to 14 carbon atoms, and specific examples thereof may be mentioned a phenyl group, a 1-naphthyl group, a 2-naphthyl group, a 1-anthracenyl group, a 2-anthracenyl group, a 9-anthracenyl group, a biphenyl group, etc.

The "C₆₋₁₄ aryloxy group" means an aryloxy group having 6 to 14 carbon atoms, and specific examples may be mentioned a phenoxy group, a 1-naphthyloxy group, a 2-naphthyloxy group, a 1-anthracenyloxy group, a 2-anthracenyloxy group, a 9-anthracenyloxy group, a biphenyloxy group, etc.

The "C₇₋₁₀ aralkyl group" means an aralkyl group having 7 to 10 carbon atoms, and specific examples may be mentioned a benzyl group, a 1-phenylethyl group, a 2-phenylethyl group, a 1-phenylpropyl group, a naphthylmethyl group, a 1-naphthylethyl group, a 1-naphthylpropyl group, etc.

The "tri-C₁₋₆ alkylsilyl group" means a group in which the same or different three above-mentioned "C₁₋₆ alkyl groups" are bonded to a silyl group, and specific examples may be mentioned a trimethylsilyl group, a triethylsilyl group, a triisopropylsilyl group, a t-butyldimethylsilyl group, a di-t-butylisobutylsilyl group, etc.

The "tri-C₁₋₆ alkylsilyloxy group" means a group in which the same or different three above-mentioned "C₁₋₆ alkyl group" are bonded to a silyloxy group, and specific examples may be mentioned a trimethylsilyloxy group, a triethylsilyloxy group, a triisopropylsilyloxy group, a t-butyldimethylsilyloxy group, a di-t-butylisobutylsilyloxy group, etc.

The "mono-C₁₋₆ alkylamino group" means a group in which one of the above-mentioned "C₁₋₆ alkyl group" is bonded to an amino group, and specific examples may be mentioned a monomethylamino group, a monoethylamino group, a mono-n-propylamino group, a monoisopropylamino group, a mono-n-butylamino group, a monoisobutylamino group, a mono-t-butylamino group, a mono-n-pentylamino group, a mono-n-hexylamino group, etc.

The "di-C₁₋₆ alkylamino group" means a group in which the same or different above-mentioned two "C₁₋₆ alkyl groups" are bonded to an amino group, and specific examples may be mentioned a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, an N-ethyl-N-n-hexylamino group, etc.

The "5 to 10-membered heterocyclic group" means a monocyclic or fused cyclic heterocyclic group having a number of atoms constituting the ring of 5 to 10, and having 1 to 4 hetero atoms independently selected from the group consisting of a nitrogen atom, an oxygen atom and a sulfur atom in the atoms constituting the ring. The heterocyclic group may be either of saturated, partially unsaturated or unsaturated, and specific examples may be mentioned a pyrrolidinyl group, a tetrahydrofuryl group, a tetrahydrothienyl group, a piperidyl group, a tetrahydropyranyl group, a tetrahydrothiopyranyl group, a pyrrol group, a furyl group, a thienyl group, a pyridyl group, a pyrimidinyl group, a pyridazinyl group, an azepanyl group, an oxepanyl group, a thiepanyl group, an azepinyl group, an oxepinyl group, a thiepinyl group, an imidazolyl group, a pyrazolyl group, an oxazolyl group, a thiazolyl group, an imidazolinyl group, a pyrazinyl group, a morpholinyl group, a thiazinyl group, an indolyl group, an isoindolyl group, a benzimidazolyl group, a purinyl group, a quinolyl group, an isoquinolyl group, a quinoxalinyl group, a cinnolinyl group, a pteridinyl group, a chromenyl group, an isochromenyl group, etc.

The "aliphatic hydrocarbon group" is a linear, branched or cyclic, saturated or unsaturated aliphatic hydrocarbon group, and may be mentioned an alkyl group, a cycloalkyl group, an alkenyl group, an alkynyl group, an aralkyl group, etc., and specific examples may be mentioned a C₁₋₄₀ alkyl group, a C₃₋₆ cycloalkyl group, a C₂₋₆ alkenyl group, a C₂₋₆ alkynyl group, a C₇₋₁₀ aralkyl group, etc.

The "aromatic hydrocarbon group" means a hydrocarbon group constituted by a single ring or a plural number of rings and at least one ring shows an aromaticity, and specific examples may be mentioned a phenyl group, a naphthyl group, an anthracenyl group, an indenyl group, a phenacenyl group, an indanyl group, etc.

The terms "which may have a substituent(s)" mean the group is unsubstituted or substituted by an optional number of an optional substituent(s).

The above-mentioned "optional substituent(s)" is not particularly limited with regard to the kind as long as it is a substituent which does not impair any bad effect on the reaction with which the present invention is targeted.

The "substituent" in the "aliphatic hydrocarbon group which may have a substituent(s)" may be mentioned, for example, a C₆₋₁₄ aryl group, a C₆₋₁₄ aryloxy group, a 5 to 10-membered heterocyclic group, a hydroxy group, a C₁₋₄₀ alkoxy group, a C₃₋₆ cycloalkoxy group, an acetoxy group, a benzoyloxy group, an amino group, a mono-C₁₋₆ alkylamino group, an N-acetylamino group, a di-C₁₋₆ alkylamino group, a halogen atom, a C₁₋₆ alkoxycarbonyl group, a phenoxycarbonyl group, an N-methylcarbamoyl group, an N-phenylcarbamoyl group, a tri-C₁₋₆ alkylsilyl group, a tri-C₁₋₆ alkylsilyloxy group, a cyano group, a nitro group, a carboxy group, etc., preferably a C₆₋₁₄ aryl group, a C₁₋₄₀ alkoxy group, a di-C₁₋₆ alkylamino group, a tri-C₁₋₆ alkylsilyl group or a tri-C₁₋₆ alkylsilyloxy group, and more preferably a C₆₋₁₄ aryl group, a C₁₋₄₀ alkoxy group or a tri-C₁₋₆ alkylsilyl group.

The "substituent" in the "aromatic hydrocarbon group which may have a substituent(s)" may be mentioned, for example, a C₁₋₄₀ alkyl group, a C₃₋₆ cycloalkyl group, a C₆₋₁₄ aryl group, a C₆₋₁₄ aryloxy group, a 5 to 10-membered heterocyclic group, a hydroxy group, a C₁₋₄₀ alkoxy group, a C₃₋₆ cycloalkoxy group, an acetoxy group, a benzoyloxy group, an amino group, a mono-C₁₋₆ alkylamino group, an N-acetylamino group, a di-C₁₋₆ alkylamino group, a halogen atom, a C₁₋₆ alkoxycarbonyl group, a phenoxycarbonyl group, an N-methylcarbamoyl group, an N-phenylcarbamoyl group, a cyano group, a nitro group, a carboxy group, etc., preferably a C₁₋₄₀ alkyl group, a C₁₋₄₀ alkoxy group or a di-C₁₋₆ alkylamino group, and more preferably a C₁₋₄₀ alkyl group or a C₁₋₄₀ alkoxy group.

The expression "two of R¹, R² and R³ form a 5- to 7-membered ring together with the Si atom to which they are bonded" means that two of R¹, R² and R³ together form a C₄₋₆ alkylene chain whereby forming a 5- to 7-membered ring together with the Si atom to which they are bonded, and specific examples may be mentioned silolane, silinane, silepane, etc.

In the present specification, the "silyl protective group having a specific structure" means a protective group which binds to the C-terminus of an amino acid or a peptide represented by the following formula (III): 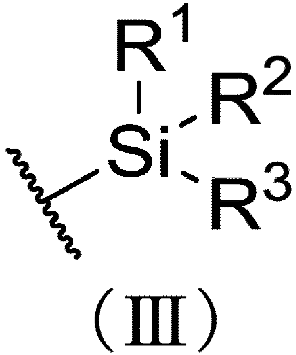 [wherein,
R¹, R² and R³ each independently represent an aliphatic hydrocarbon group which may have a substituent(s), an aromatic hydrocarbon group which may have a substituent(s) or -OR⁴ (R⁴ represents an aliphatic hydrocarbon group which may have a substituent(s), an aromatic hydrocarbon group which may have a substituent(s).), two of R¹, R² and R³ may form a 5- to 7-membered ring together with the Si atom to which they are bonded, and a total number of the carbon atoms in the R¹R²R³Si group is 8 or more.].

The terms "a total number of the carbon atoms in the R¹R²R³Si group" mean a total of the carbon atoms possessed by R¹, R² and R³, respectively, and when at least one among R¹, R² and R³ has a substituent(s), the carbon number in the substituent(s) is also contained.

In the formula (II), R¹, R² and R³ are preferably each independently an aliphatic hydrocarbon group which may have a substituent(s), more preferably two or three of R¹, R² and R³ each independently are a secondary or tertiary aliphatic hydrocarbon group, further preferably among R¹, R² and R³, two or three of them each independently are a secondary or tertiary C₃₋₆ alkyl group or a C₃₋₆ cycloalkyl group, further more preferably among R¹, R² and R³, two or three of them each independently are a t-butyl group, an s-butyl group, an i-propyl group, a cyclopentyl group or a cyclohexyl group.

In the formula (II), as the other embodiments of R¹, R² and R³, preferably R¹, R² and R³ are each independently an aliphatic hydrocarbon group which may have a substituent(s), more preferably among R¹, R² and R³, two of them each independently are a secondary aliphatic hydrocarbon group, and the remaining one is a secondary or tertiary aliphatic hydrocarbon group which may have a substituent(s), which is a group different from the above-mentioned two, further preferably among R¹, R² and R³, two of them each independently are a secondary or tertiary C₃₋₆ alkyl group or a C₃₋₆ cycloalkyl group, and the remaining one is a secondary C₃₋₆ alkyl group or a C₃₋₆ cycloalkyl group which may have a substituent(s), which is a group different from the above-mentioned two, and further more preferably among R¹, R² and R³, one of them is a t-butyl group or a cumyl group, and the remaining two are each independently a t-butyl group, an i-propyl group, an s-butyl group, a cyclopentyl group or a cyclohexyl group.

In the formula (II), as the other embodiments of R¹, R² and R³, preferably R¹, R² and R³ are each independently an aliphatic hydrocarbon group which may have a substituent(s), more preferably among R¹, R² and R³, two of them each independently are a tertiary aliphatic hydrocarbon group, further preferably among R¹, R² and R³, two of them each independently are a tertiary C₄₋₆ alkyl group, and further more preferably among R¹, R² and R³, two of them are t-butyl groups, and the remaining one is an i-butyl group, a benzyl group, an octadecyl group or a (trimethylsilyl)methyl group.

When the deprotection step of the protective group at the N-terminus is under basic or neutral conditions, in the formula (II), as the other embodiments of R¹, R² and R³, each independently represent -OR⁴. R⁴ is preferably an aliphatic hydrocarbon group which may have a substituent(s), more preferably an aliphatic hydrocarbon group, further preferably a C₁₋₆ alkyl group, further more preferably a tertiary C₄₋₆ alkyl group, and particularly preferably a t-butyl group.

The tertiary aliphatic hydrocarbon group is preferably a t-butyl group, an α,α-dimethylbenzyl group (a cumyl group), a thexyl group or a 1-adamantyl group, more preferably a t-butyl group or an α,α-dimethylbenzyl group, and particularly preferably a t-butyl group.

The secondary aliphatic hydrocarbon group is preferably an isopropyl group, a 2-butyl group, a 3-pentyl group, a cyclopentyl group or a cyclohexyl group, and more preferably an isopropyl group, a cyclopentyl group or a cyclohexyl group.

In the formula (III), a total number of the carbon atoms in the R¹R²R³Si group is preferably 10 to 100, more preferably 10 to 40, and further preferably 10 to 26.

As the characteristics of the silyl protective group (the protective group of the present invention) having a specific structure to be used in the present invention, there may be mentioned, for example, as follows.
(a) The protective group of the present invention is stable under acidic conditions (in the presence of a reagent such as 15% by mass hydrogen chloride/1,4-dioxane, etc.) at which a Boc group at the N-terminus is deprotected (see Synthetic Example 4, etc., mentioned later).
(b) The protective group of the present invention is stable under reduction conditions (in the presence of a reagent such as 10% by mass Pd-C, a hydrogen gas, etc.) at which a Cbz group at the N-terminus is deprotected (see Synthetic Example 2, etc., mentioned later).
(c) The protective group of the present invention is stable under basic conditions (in the presence of a reagent such as diethylamine, etc.) at which an Fmoc group at the N-terminus is deprotected (see Synthetic Example 3, etc., mentioned later).
(d) The protective group of the present invention is stable under the conditions of silica gel column chromatography at which a TMS group or a TBS group at the C-terminus is deprotected.

The "N-protected amino acid" and the "N-protected peptide" mean an amino acid or a peptide in which an amino group at the N-terminus is protected and a carboxy group at the C-terminus is not protected.

The "C-protected amino acid" and the "C-protected peptide" mean an amino acid or a peptide in which a carboxy group at the C-terminus is protected and an amino group at the N-terminus is not protected.

The amino acid to be used in the present invention is an organic compound having both functional groups of an amino group and a carboxy group, preferably an α-amino acid, a β-amino acid, a γ-amino acid or a δ-amino acid, more preferably an α-amino acid or a β-amino acid, and further preferably an α-amino acid. Also, when two or more amino groups are present (for example, arginine, lysine, etc.), when two or more carboxy groups are present (for example, glutamic acid, aspartic acid, etc.), or a reactive functional group is present (for example, cysteine, serine, etc.) in these amino acids, the amino acid to be used in the present invention includes an amino acid in which an amino group, a carboxy group and/or a reactive functional group which does not participate in formation of a peptide is/are protected and/or modified.

The amino group of the amino acid to be used in the present invention may be substituted. The substituent of the amino group is preferably an aliphatic hydrocarbon group which may have a substituent(s), more preferably a C₁₋₆ alkyl group or a C₇₋₁₀ aralkyl group, and further preferably a methyl group.

The amino acids constituting the peptide to be used in the present invention are the above-mentioned amino acids.

The steric structure of the α-amino acid is not particularly limited, and is preferably an L-isomer.

The "temporary protective group" means a protective group at the terminal side from which a peptide chain is to be elongated and a protective group deprotected before subjecting to the peptide elongation reaction (amidation reaction), and in elongation of the peptide chain from the N-terminal side, a protective group at the N-terminus is mentioned. Specific examples of the protective group at the N-terminus may be mentioned a carbamate protective group (a 9-fluorenylmethoxycarbonyl group, a t-butoxycarbonyl group, a benzyloxycarbonyl group, an allyloxycarbonyl group, a 2,2,2-trichloroethoxycarbonyl group, a 2-(p-biphenyl)isopropyloxycarbonyl group, etc.), an amide protective group (an acetyl group, a trifluoroacetyl group, etc.), an imide protective group (a phthaloyl group, etc.), a sulfonamide protective group (a p-toluenesulfonyl group, a 2-nitrobenzenesulfonyl group, etc.), a benzyl group, etc.

All technical terms and scientific terms used in the present specification have the same meanings as commonly understood by those skilled in the art to which the present invention belongs. Any optional methods and materials similar or equivalent to those described in the present specification can be used in the practice or test of the present invention and preferable methods and materials are mentioned below. All publications and patents referred to in the present specification are incorporated in the present specification by reference, for example, for the purpose of describing and disclosing the constructs and methodologies, in the publications that may be used in connection with the described invention.

### (Specific explanation of producing method of peptide of the present invention)

In the following, each step (i) to (viii) of the producing method of the peptide of the present invention is explained.

As one embodiment, production of the peptide of the present invention is constituted by the respective unit step described as the following steps (i) to (viii).

As one embodiment, production of the peptide of the present invention can be carried out by subjecting to all the unit step described as the following steps (i) to (viii), or optionally combining some of these.

Incidentally, this specific explanation is explained by the following.
(a) R² and R³ in the description of the steps (i) to (viii) are the same as the definitions as mentioned above.
(b) Specific conditions of the reaction are not particularly limited as long as production of the peptide of the present invention is accomplished. Preferable conditions in the respective reactions are appropriately mentioned in detail.
(c) The solvents described in the respective reactions may be used singly or in admixture of two or more kinds.

### Step (i): Protection step of C-terminus

The present step is a step of introducing a silyl protective group having a specific structure into a C-terminus of an N-protected amino acid or an N-protected peptide.

For introduction of the silyl protective group having a specific structure, a silyl protecting agent can be used. The silyl protecting agent is represented by the following formula (IV).

The silyl protecting agent represented by the formula (IV): 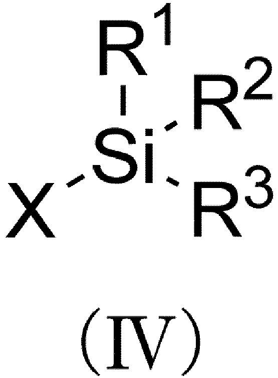 [wherein,
X represents a hydrogen atom or a leaving group such as a halogen atom, a cyano group or a trifluoromethanesulfonyloxy group, etc.,
R² and R³ each independently represent an aliphatic hydrocarbon group which may have a substituent(s), an aromatic hydrocarbon group which may have a substituent(s), -OR⁴ (R⁴ represents an aliphatic hydrocarbon group which may have a substituent(s) or an aromatic hydrocarbon group which may have a substituent(s).), two of R¹, R² and R³ may form a 5- to 7-membered ring together with the Si atom to which they are bonded, and a total number of the carbon atoms in the R¹R²R³Si group is 8 or more.].

R² and R³ in the formula (IV) have the same meanings as defined above.

In the present specification, the "N-protected amino acid" and the "N-protected peptide" are referred to as "P-AA-OH" (P is also referred to as a protective group at the N-terminus or a temporary protective group. OH represents hydroxy in the C-terminal carboxy group. AA represents a group derived from an amino acid or a peptide.).

The coupling reaction of the silyl protecting agent and the N-protected amino acid or the N-protected peptide can be carried out in the presence of a base or a metal catalyst. 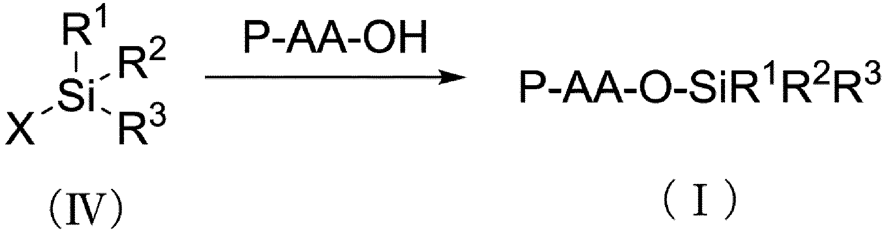 (wherein, each symbol is the same meaning as defined above.)

The base to be used in the present step is not particularly limited, and an example thereof may be mentioned an aliphatic amine (for example, dicyclohexylamine, piperidine, triethylamine, N,N-diisopropylethylamine and N-methylmorpholine), an aromatic amine (for example, pyridine, imidazole and N,N-dimethyl-4-aminopyridine), an alkali metal salt (for example, sodium hydrogen carbonate and potassium carbonate), etc. It is preferably an aliphatic amine or an aromatic amine, and more preferably N,N-diisopropylethylamine or imidazole.

The metal catalyst to be used in the present step is not particularly limited, and an example thereof may be mentioned a nickel catalyst (for example, nickel(II) chloride), a zinc catalyst (for example, zinc(II) chloride), a palladium catalyst (for example, palladium(II) acetate), a ruthenium catalyst (for example, triruthenium dodecacarbonyl), a copper catalyst (for example, triphenylphosphine copper hydride hexamer, copper(I) oxide), a manganese catalyst (for example, dimanganese(0) decacarbonyl), etc. It is preferably a palladium catalyst, a nickel catalyst, a zinc catalyst and a copper catalyst, and more preferably palladium(II) acetate, nickel(II) chloride, zinc(II) chloride and triphenylphosphine copper hydride hexamer.

An amount of the base or the metal catalyst to be used in the present step is preferably 0.01 equivalent to 50 equivalents based on the silyl protecting agent, more preferably 0.1 equivalent to 20 equivalents, and further preferably 0.2 equivalent to 5 equivalents.

The solvent to be used in the present step is not particularly limited as long as it does not inhibit the reaction, and an example thereof may be mentioned a halogen-containing hydrocarbon solvent (for example, dichloromethane, chloroform), an aromatic hydrocarbon solvent (for example, toluene, xylene), an ether solvent (for example, tetrahydrofuran, 1,4-dioxane, cyclopentyl methyl ether, methyl-t-butyl ether), an amide solvent (for example, N,N-dimethylformamide), a nitrile solvent (for example, acetonitrile), etc. It is preferably a halogen-containing hydrocarbon solvent or an ether solvent, more preferably dichloromethane, tetrahydrofuran or cyclopentyl methyl ether.

An amount of the solvent to be used in the present step is preferably 100-fold by mass or less based on the silyl protecting agent, more preferably 1-fold by mass to 50-fold by mass, and further preferably 5-fold by mass to 20-fold by mass.

A reaction temperature is not particularly limited, and preferably from -20°C to a reflux temperature of the reaction mixture, more preferably -20°C to 50°C, and further preferably -10°C to 30°C.

### Step (ii): Deprotection step of N-terminus

The present step is a step of removing the protective group at the N-terminus of the amino acid or the peptide obtained in the above-mentioned step (i).

As the protective group at the N-terminus, a temporary protective group of the amino group generally used in the technical field such as peptide chemistry, etc., can be used, preferably a protective group that is eliminated under the conditions different from those of elimination of the silyl protective group having a specific structure, more preferably a carbamate protective group (a 9-fluorenylmethoxycarbonyl group, a t-butoxycarbonyl group, a benzyloxycarbonyl group, a 2,2,2-trichloroethoxycarbonyl group, an allyloxycarbonyl group, etc.), and further preferably a 9-fluorenylmethoxy-carbonyl group, a t-butoxycarbonyl group or a benzyloxycarbonyl group.

Deprotecting conditions can be optionally selected depending on the kind of the protective group at the N-terminus, and deprotection is preferably carried out under the conditions different from those of elimination of the silyl protective group having a specific structure. For example, in the case of a 9-fluorenylmethoxycarbonyl group, it is carried out by treating with a base, in the case of a t-butoxycarbonyl group, it is carried out by treating with an acid, and in the case of a benzyloxycarbonyl group, it is carried out in a neutral state by subjecting to hydrogenation, for example, in the presence of a metal catalyst.

When the total number of the carbon atoms in the R¹R²R³Si group is 8 or 9, a pH of the deprotection condition is preferably a neutral state (6 to 8). Also, when the total number of the carbon atoms in the R¹R²R³Si group is 10 or more, a pH of the deprotection condition is not particularly limited.

The base to be used in the present step may be mentioned dimethylamine, diethylamine, piperidine, morpholine, dicyclohexylamine, N,N-dimethyl-4-aminopyridine, etc.

The acid to be used in the present step may be mentioned hydrochloric acid, sulfuric acid, trifluoroacetic acid, trifluoromethanesulfonic acid, etc.

The metal catalyst to be used in the present step may be mentioned a palladium catalyst (for example, 5% by mass palladium carbon powder STD type, 10% by mass palladium carbon powder PE type, 5% by mass palladium carbon powder NX type, 5% by mass palladium carbon powder K type, 5% by mass palladium carbon powder PE type, ASCA-2), a platinum catalyst (for example, 3% by mass platinum carbon powder STD type, 3% by mass platinum carbon powder SN101 type), a ruthenium catalyst (for example, 5% by mass ruthenium carbon powder A type, 5% by mass ruthenium carbon powder B type), and alumina powder.

The solvent to be used in the present step is not particularly limited as long as it does not inhibit the reaction, and an example thereof may be mentioned an alcohol solvent (for example, methanol, ethanol, 2-propanol, 2,2,2-trifluoroethanol), halogen-containing hydrocarbon solvents (for example, dichloromethane, chloroform), an aromatic hydrocarbon solvent (for example, toluene, xylene), ether solvent (for example, tetrahydrofuran, 1,4-dioxane, cyclopentyl methyl ether, methyl-t-butyl ether), amide solvents (for example, N,N-dimethylformamide), a nitrile solvent (for example, acetonitrile), etc. It is preferably an alcohol solvent, a halogen-containing hydrocarbon solvent or an ether solvent, and more preferably isopropanol, 2,2,2-trifluoroethanol, dichloromethane, tetrahydrofuran or methyl-t-butyl ether.

### Step (iii): Peptide chain elongation step

The present step is a step of condensing an N-protected amino acid or an N-protected peptide to the N-terminus of the C-protected amino acid or C-protected peptide obtained in the step (ii).

The present step is carried out using a condensation agent under condensing conditions generally used in the technical field of peptide chemistry, etc.

The condensation agent to be used in the present step is not particularly limited, and an example thereof may be mentioned a carbodiimide condensation agent (for example, N,N'-dicyclohexylcarbodiimide (DCC), N,N'-diisopropylcarbodiimide, 1-ethyl-3-dimethylaminopropylcarbodiimide hydrochloride (EDCI)), a chloroformate condensation agent (for example, ethyl chloroformate, isobutyl chloroformate), an imidazole condensation agent (for example, 1,1'-carbonyldiimidazole (CDI)), a phosphonium condensation agent (for example, (benzotriazol-1-yloxy)tri-pyrrolidinophosphonium hexafluorophosphate (PyBOP (Registered Trademark)), bromotripyrrolidinophosphonium hexafluorophosphate (PyBrop (Registered Trademark))), an uronium condensation agent (for example, O-(benzotriazol-l-yl)-N,N,N',N'-tetramethyluronium tetrafluoroborate (TBTU), 1-[bis(dimethylamino)-methylene]-5-chloro-1H-benzotriazolium 3-oxide hexafluorophosphate (HCTU), O-benzotriazol-N,N,N',N'-tetramethyluronium hexafluoroborate (HBTU) and (1-cyano-2-ethoxy-2-oxoethylidenaminooxy) dimethylamino-morpholino-carbenium hexafluorophosphate (COMU)).

An amount of the condensation agent to be used is preferably 0.1 equivalent to 20 equivalents based on the C-protected amino acid or the C-protected peptide, more preferably 1 equivalent to 10 equivalents, and further preferably 1 equivalent to 5 equivalents.

In the present step, an additive(s) and a base(s) can be optionally used as long as they do not inhibit the reaction.

The additive(s) to be used in the present step is not particularly limited, and an example thereof may be mentioned N,N-dimethyl-4-aminopyridine (DMAP), 1-hydroxybenzotriazole (HOBt), ethyl 1-hydroxy-IH-1,2,3-triazol-5-carboxylate (HOCt), 1-hydroxy-7-azabenzotriazol (HOAt), (hydroxyimino)cyanoethyl acetate (OxymaPure), etc.

An amount of the additive(s) to be used is preferably 0.01 equivalent to 20 equivalents based on the C-protected amino acid or the C-protected peptide, more preferably 0.2 equivalent to 10 equivalents, and further preferably 1 equivalent to 5 equivalents.

The base(s) to be used in the present step is not particularly limited, and an example thereof may be mentioned an aliphatic amine (for example, triethylamine, N,N-diisopropylethylamine, N-methylmorpholine) and an aromatic amine (for example, pyridine), etc. It is preferably an aliphatic amine, and more preferably N,N-diisopropylethylamine.

An amount of the base(s) to be used is preferably 1 equivalent to 50 equivalents based on the C-protected amino acid or the C-protected peptide, more preferably 1 equivalent to 10 equivalents, and further preferably 1 equivalent to 5 equivalents.

The solvent to be used in the present step is not particularly limited as long as it does not inhibit the reaction, and an example thereof may be mentioned a halogen-containing hydrocarbon solvent (for example, dichloromethane, chloroform), an aromatic hydrocarbon solvent (for example, toluene, xylene), an ether solvent (for example, tetrahydrofuran, 1,4-dioxane, cyclopentyl methyl ether, methyl-t-butyl ether), an amide solvent (for example, N,N-dimethylformamide, etc.), and a nitrile solvent (for example, acetonitrile), etc. It is preferably a halogen-containing hydrocarbon solvent or an ether solvent, and more preferably dichloromethane, tetrahydrofuran or cyclopentyl methyl ether.

An amount of the solvent to be used is preferably 100-fold by mass or less based on the C-protected amino acid or the C-protected peptide, more preferably 1-fold by mass to 50-fold by mass, and further preferably 5-fold by mass to 20-fold by mass.

A reaction temperature is not particularly limited, preferably from -40°C to a reflux temperature of the reaction mixture, more preferably -20°C to 50°C, and further preferably -10°C to 30°C.

A reaction time is not particularly limited, preferably from initiating the reaction to 72 hours, more preferably 0.1 hour to 48 hours, and further preferably 1 to 24 hours.

For confirmation of the progress of the reaction, the same method as in the general liquid phase organic synthesis reaction can be used. That is, the reaction can be traced using thin layer chromatography, high performance liquid chromatography, high performance liquid chromatography/mass analysis (LC/MS), etc.

### Step (iv): Purification step

The present step is a step of purifying the peptide obtained in the above-mentioned step (iii) by precipitation or liquid-separating operation.

In the precipitation operation, a good solvent for dissolving a peptide and/or a poor solvent insolubilizing the same can be used.

The good solvent to be used in the present step is optionally selected depending on the obtained peptide, and an example thereof may be mentioned a halogen-containing hydrocarbon solvent (for example, dichloromethane, chloroform), an aromatic hydrocarbon solvent (for example, toluene, xylene), an ether solvent (for example, tetrahydrofuran, 1,4-dioxane, cyclopentyl methyl ether, methyl-t-butyl ether) and an ester solvent (for example, ethyl acetate, isopropyl acetate), etc. It is preferably a halogen-containing hydrocarbon solvent, an ether solvent or an ester solvent, and more preferably dichloromethane, tetrahydrofuran, cyclopentyl methyl ether, methyl-t-butyl ether or isopropyl acetate.

The poor solvent to be used in the present step is optionally selected depending on the obtained peptide, and an example thereof may be mentioned an alcohol solvent (for example, methanol, ethanol, isopropanol), an amide solvent (for example, N,N-dimethylformamide), a nitrile solvent (for example, acetonitrile) and an ester solvent (for example, ethyl acetate, isopropyl acetate), etc. It is preferably an alcohol solvent, a nitrile solvent or an ester solvent, and more preferably methanol, acetonitrile or ethyl acetate.

In the liquid-separating operation, a good solvent in which a peptide is dissolved is washed with water, or an acidic and/or basic aqueous solution depending on impurities capable of containing in the objective peptide whereby the impurities can be removed.

The acidic aqueous solution to be used in the present step is not particularly limited, and an example thereof may be mentioned hydrochloric acid, sulfuric acid, an acetic acid aqueous solution, a phosphoric acid aqueous solution, a citric acid aqueous solution, an ammonium chloride aqueous solution, etc. It is preferably hydrochloric acid, a phosphoric acid aqueous solution, a citric acid aqueous solution or an ammonium chloride aqueous solution.

The basic aqueous solution to be used in the present step is not particularly limited, and an example thereof may be mentioned an aqueous sodium hydrogen carbonate solution, an aqueous potassium hydrogen carbonate solution, an aqueous sodium carbonate solution, an aqueous potassium carbonate solution, aqueous ammonia, etc. It is preferably a sodium hydrogen carbonate aqueous solution or aqueous ammonia.

Also, in the method for producing a peptide of the present invention, by repeating the following steps (v) to (vii) with a desired number of times with respect to the peptide obtained in the step (iv), the peptide chain can be further elongated.
(v) a step of removing the temporary protective group at the N-terminus of the peptide obtained in the purification step,
(vi) a step of condensing an N-protected amino acid or an N-protected peptide to the N-terminus of the C-protected peptide obtained in the above-mentioned step (v), and
(vii) a step of precipitating or liquid-separating the peptide obtained in the above-mentioned step (vi).

Either of the steps can be carried out by the same operation as the above-mentioned steps (ii) to (iv).

The present step may be carried out with respect to the amino acid or the peptide obtained by the deprotection step of the N-terminus in the above-mentioned step (ii) or (v).

In the method for producing a peptide of the present invention, it is optionally possible to omit the purification step of the step (iv) or step (vii) within the range which does not affect to the reaction of the next step.

### Step (viii): Deprotection step of C-terminus

The present step is a step of obtaining an N-protected peptide by removing the silyl protective group having a specific structure from the peptide isolated by the purification step of the above-mentioned step (iv) or (vii).

The deprotecting agent to be used in the present step is not particularly limited, and an example thereof may be mentioned a fluorinating agent (for example, potassium fluoride, calcium fluoride, hydrogen fluoride, hydrogen fluoride-pyridine, tetrabutylammonium fluoride).

An amount of the deprotecting agent to be used is preferably 1 equivalent to 50 equivalents based on the peptide to be used, more preferably 1 equivalent to 10 equivalents, and further preferably 1 equivalent to 5 equivalents.

The solvent to be used in the present step is not particularly limited as long as it does not inhibit the reaction, and an example thereof may be mentioned an alcohol solvent (for example, methanol, ethanol), a halogen-containing hydrocarbon solvent (for example, dichloromethane, chloroform), an aromatic hydrocarbon solvent (for example, toluene, xylene), an ether solvent (for example, tetrahydrofuran, 1,4-dioxane, cyclopentyl methyl ether), an amide solvent (for example, N,N-dimethylformamide, N-methylpyrrolidone, 1,3-dimethyl-2-imidazolidinone) and water, etc. It is preferably an alcohol solvent, a halogen-containing hydrocarbon solvent, an ether solvent, an amide solvent or water, more preferably an alcohol solvent, a halogen-containing hydrocarbon solvent or an ether solvent, and further preferably methanol, dichloromethane or tetrahydrofuran.

An amount of the solvent to be used is preferably 100-fold by mass or less based on the peptide to be used, more preferably 1-fold by mass to 50-fold by mass, and further preferably 5-fold by mass to 20-fold by mass.

A reaction temperature is not particularly limited, and is preferably from - 20°C to a reflux temperature of the reaction mixture, more preferably -20°C to 50°C, and further preferably -10°C to 30°C.

In the respective reactions, when the reactant has a hydroxy group, a mercapto group, an amino group, a carboxy group or a carbonyl group (in particular, when it has a functional group at the side chain of the amino acid or the peptide), a protective group which is generally used in the peptide chemistry, etc., may be introduced into these groups, and by removing the protective group after the reaction depending on necessity, the objective compound can be obtained.

Protection and deprotection can be carried out by subjecting to a protection and deprotection reaction using a generally known protective group (for example, see Protective Groups in Organic Synthesis, Fourth edition, written by T. W. Greene, John Wiley & Sons Inc. (2006), etc.).

The above-mentioned purification step (iv) may be carried out with respect to the peptide obtained by the present step.

### EXAMPLES

In the following, the present invention is further explained in detail by referring to Reference Synthetic Examples and Synthetic Examples, but the present invention is not limited to these Examples.

In the present specification, when an amino acid, etc., are indicated by abbreviations, each indication is based on an abbreviation by IUPAC-IUB Commission on Biochemical Nomenclature or an abbreviation commonly used in this field of the art.

The proton nuclear magnetic resonance (¹H-NMR) of Examples is measured by using JNM-ECP300 manufactured by JEOL Ltd., or JNM-ECX300 manufactured by JEOL Ltd., or Ascend™500 manufactured by Bruker in deuterated chloroform or deuterated dimethylsulfoxide solvent otherwise specifically mentioned, and the chemical shift is shown by a δ value (ppm) when tetramethylsilane is used as an internal standard (0.0 ppm).

In the description of the NMR spectrum, "s" means singlet, "d" means doublet, "t" means triplet, "q" means quartet, "dd" means doublet of doublet, "dt" means doublet of triplet, "m" means multiplet, "br" means broad, "J" means a coupling constant, "Hz" means hertz, "CDCl₃" means deuterated chloroform and "DMSO-d6" means deuterated dimethylsulfoxide.

The high-performance liquid chromatography/mass analysis is measured by using any of ACQUITY UPLC H-Class/QDa manufactured by Waters Corporation, ACQUITY UPLC H-Class/SQD2 manufactured by Waters Corporation, or LC-20AD/Triple Tof5600 manufactured by Shimadzu Corporation otherwise specifically mentioned.

In the description of the high-performance liquid chromatography/mass analysis, ESI+ is a positive mode of an electrospray ionization method, and M+H means a proton adduct and M+Na means a sodium adduct.

In the description of the high-performance liquid chromatography/mass analysis, ESI- means a negative mode of an electrospray ionization method, and M-H means a proton defected material.

Purification by silica gel column chromatography is carried out by using either of Hi-Flash column manufactured by Yamazen Corporation, SNAP Ultra Silica Cartridge manufactured by Biotage, silica gel 60 manufactured by Merck or PSQ60B manufactured by Fuji Silysia Chemical Ltd., otherwise specifically mentioned.

### Synthetic Example 1: Synthesis of H-MePhe-OBIBS

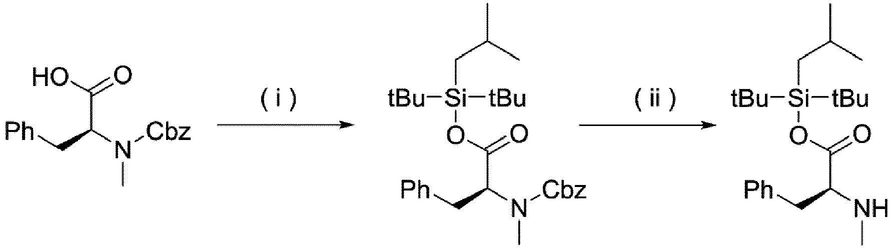

(i) Cbz-MePhe-OH (2.50 g, 7.98 mmol) and di-t-butylisobutylsilane triflate (3.08 g, 8.83 mmol) were mixed with methylene chloride (30.0 g), the mixture was cooled to 0°C, and after adding N,N-diisopropylethylamine (1.24 g, 9.58 mmol) dropwise, stirred at room temperature for 2 hours. The obtained reaction mixture was washed with an aqueous saturated ammonium chloride solution (20.3 g), the obtained organic layer was concentrated and purified by silica gel column chromatography to obtain Cbz-MePhe-OBIBS (3.45 g, Yield: 85%) as a colorless liquid.
   MASS (ESI+) m/z; 512.38 (M+H)+
(ii) Cbz-MePhe-OBIBS (0.60 g, 1.17 mmol) was mixed with 2,2,2-trifluoroethanol (6.0 g), and after adding 10% by mass Pd-C (60.2 mg, 0.057 mmol) thereto, the mixture was stirred under hydrogen gas atmosphere at room temperature for 2 hours. The reaction mixture was filtered and the obtained filtrate was concentrated. The concentrate was dissolved in methylene chloride (30.3 g), and water (20.0 g) was added thereto and the liquids were separated. The organic layer was concentrated to obtain H-MePhe-OBIBS (0.42 g, Yield: 97%).
   MASS (ESI+) m/z; 378.35 (M+H)+

### Synthetic Example 2: Synthesis of Fmoc-MePhe-MePhe-MePhe-OBIBS

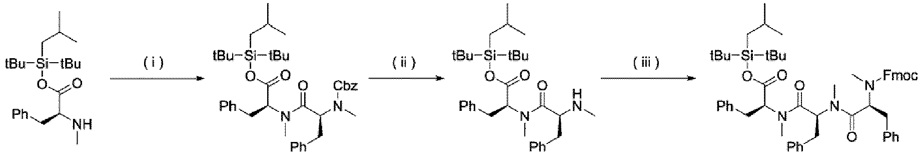

(i) H-MePhe-OBIBS (0.41 g, 1.08 mmol) was dissolved in methylene chloride (8.9 g), Cbz-MePhe-OH (0.56 g, 1.77 mmol) and 1-ethyl-3-(3-dimethylaminopropyl)carbodiimide hydrochloride (0.34 g, 1.76 mmol) were added thereto under ice-cooling and stirred for 3 hours. After returned to room temperature and stirring for 2 hours, chloroform (30.9 g) and water (20.2 g) were added thereto and the liquids were separated. The organic layer was washed with an aqueous saturated sodium hydrogen carbonate solution (20.0 g), 5% by mass aqueous citric acid solution (20.1 g) and an aqueous saturated ammonium chloride solution (20.0 g) in this order, the obtained organic layer was concentrated and purified by silica gel column chromatography to obtain Cbz-MePhe-MePhe-OBIBS (0.65 g, Yield: 89%) as a colorless liquid. MASS (ESI+) m/z; 673.51 (M+H)+
(ii) Cbz-MePhe-MePhe-OBIBS (0.41g, 0.61 mmol) was dissolved in 2,2,2-trifluoroethanol (4.0 g), and after adding 10% by mass Pd-C (41.2 mg, 0.039 mmol), the mixture was stirred under hydrogen gas atmosphere at room temperature for 22 hours. The reaction mixture was filtered and the obtained filtrate was concentrated. The concentrate was dissolved in chloroform (30.0 g), and water (20.0 g) was added thereto and the liquids were separated. The organic layer was concentrated to obtain H-MePhe-MePhe-OBIBS (0.32 g, Yield: 99%). MASS (ESI+) m/z; 539.46 (M+H)+
(iii) H-MePhe-MePhe-OBIBS (0.30 g, 0.56 mmol) and Fmoc-MePhe-OH (0.34 g, 0.84 mmol) were dissolved in methylene chloride (6.1 g), and under ice-cooling, 1-ethyl-3-(3-dimethylaminopropyl)carbodiimide hydrochloride (0.16 g, 0.84 mmol) was added thereto and the mixture was stirred for 6 hours. After diluting the obtained reaction mixture with chloroform (30.0 g), it was washed with an aqueous saturated ammonium chloride solution (20.2 g) and an aqueous saturated sodium hydrogen carbonate solution (20.1 g) in this order. The obtained organic layer was dried over magnesium sulfate, and then, the solution obtained by filtration was concentrated. The obtained residue was purified by silica gel column chromatography to obtain Fmoc-MePhe-MePhe-MePhe-OBIBS (0.49 g, Yield: 95%) as a white solid.
   MASS (ESI+) m/z; 922.51 (M+H)+

### Synthetic Example 3: Synthesis of Boc-MePhe-MePhe-MePhe-MePhe-OBIBS

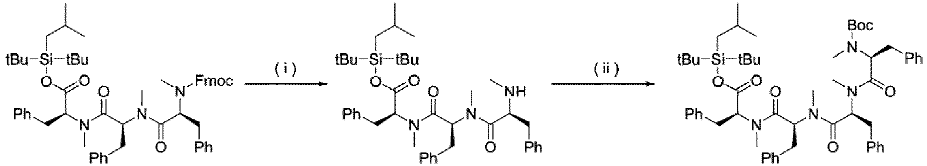

(i) Fmoc-MePhe-MePhe-MePhe-OBIBS (0.30 g, 0.32 mmol) was mixed with methylene chloride (6.0 g) and cooled to 0°C, and after adding diethylamine (0.60 g, 8.2 mmol), the mixture was stirred for 1 hour. After returning to room temperature and stirring for 22 hours, the mixture was diluted with chloroform (30.1 g), washed with an aqueous saturated ammonium chloride solution (20.2 g) twice, and the organic layer was concentrated, and then, purified by silica gel column chromatography to obtain H-MePhe-MePhe-MePhe-OBIBS (0.21 g, Yield: 93%) as a yellow liquid.
   MASS (ESI+) m/z; 700.44 (M+H)+
(ii) H-MePhe-MePhe-MePhe-OBIBS (0.20 g, 0.28 mmol) and Boc-MePhe-OH (0.12 g, 0.43 mmol) were mixed with methylene chloride (4.0 g), the mixture was cooled to 0°C, 1-ethyl-3-(3-dimethylaminopropyl)carbodiimide hydrochloride (0.083 g, 0.43 mmol) was added thereto and the mixture was stirred for 3 hours. After returning to room temperature and stirring for 3 hours, the obtained reaction mixture was diluted with chloroform (30.0 g), and washed with an aqueous saturated sodium hydrogen carbonate solution (20.2 g) and an aqueous saturated ammonium chloride solution (20.0 g) in this order. The obtained organic layer was concentrated and purified by silica gel column chromatography to obtain Boc-MePhe-MePhe-MePhe-MePhe-OBIBS (0.25 g, Yield: 90%) as a white solid.
   MASS (ESI+) m/z; 961.58 (M+H)+

### Synthetic Example 4: Synthesis of Cbz-MePhe-MePhe-MePhe-MePhe-MePhe-OBIBS

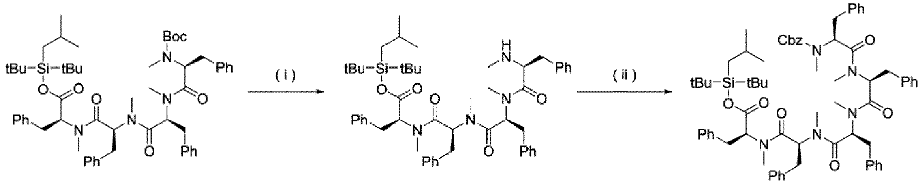

(i) Boc-MePhe-MePhe-MePhe-MePhe-OBIBS (0.18 g, 0.19 mmol) was mixed with methylene chloride (4.0 g), the mixture was cooled to 0°C, 15% by mass hydrogen chloride-1,4-dioxane (2.0 g, 8.2 mmol) was added thereto, and then, the mixture was stirred for 3 hours. After returning to room temperature and stirring for 4 hours, the obtained reaction mixture was diluted with chloroform (60.0 g), and then, an aqueous saturated sodium hydrogen carbonate solution (40.3 g) was added thereto and the liquids were separated, and the organic layer was washed with water (40.1 g). The obtained organic layer was concentrated to obtain H-MePhe-MePhe-MePhe-MePhe-OBIBS (0.16 g, Yield: 99%) as a white solid.
   MASS (ESI+) m/z; 861.53 (M+H)+
(ii) H-MePhe-MePhe-MePhe-MePhe-OBIBS (0.16 g, 0.18 mmol) and Cbz-MePhe-OH (0.038 g, 0.29 mmol) were mixed with methylene chloride (3.2 g), the mixture was cooled to 0°C, 1-ethyl-3-(3-dimethylaminopropyl)carbodiimide hydrochloride (0.054 g, 0.28 mmol) was added thereto and the mixture was stirred for 7 hours. The obtained reaction mixture was diluted with chloroform (40.2 g), and then, washed with an aqueous saturated sodium hydrogen carbonate solution (30.0 g) and an aqueous saturated ammonium chloride solution (30.0 g) in this order. The obtained organic layer was concentrated and purified by silica gel column chromatography to obtain Cbz-MePhe-MePhe-MePhe-MePhe-MePhe-OBIBS (0.19 g, Yield: 93%) as a white solid.
   MASS (ESI+) m/z; 1156.65 (M+H)+

### Synthetic Example 5: Synthesis of Cbz-MePhe-MePhe-MePhe-MePhe-MePhe-OH

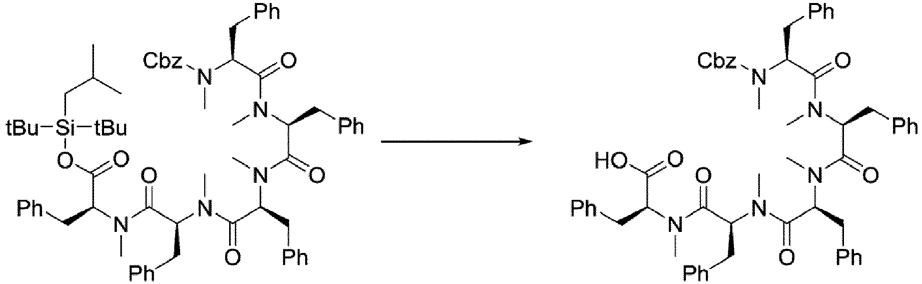

Cbz-MePhe-MePhe-MePhe-MePhe-MePhe-OBIBS (0.15 g, 0.13 mmol) was mixed with methylene chloride (3.0 g), the mixture was cooled to 0°C, 1M fluorinated tetrabutylammonium-tetrahydrofuran solution (0.14 mL, 0.14 mmol) was added thereto and the mixture was stirred for 2 hours. To the obtained reaction mixture were added 1M hydrochloric acid (20 g) and chloroform (40.0 g) and the liquids were separated, and the organic layer was washed with 1% by mass aqueous sodium chloride solution. The obtained organic layer was concentrated and purified by silica gel column chromatography to obtain Cbz-MePhe-MePhe-MePhe-MePhe-MePhe-OH (0.12 g, Yield: 95%) as a white solid.
MASS (ESI+) m/z; 958.47 (M+H)+

### Synthetic Example 6: Synthesis of H-MePhe-OBIBS

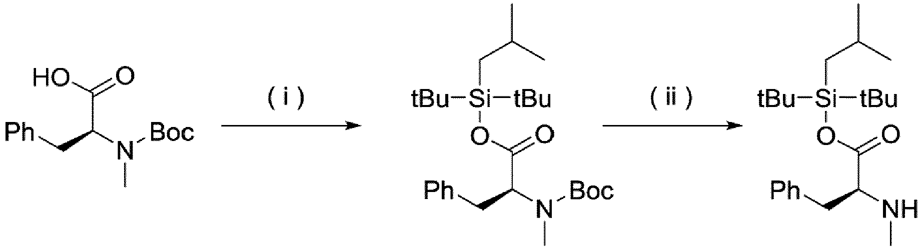

(i) Boc-MePhe-OH (0.88 g, 3.17 mmol) and N,N-diisopropylethylamine (0.46 g, 3.59 mmol) were mixed with methylene chloride (13.3 g), the mixture was cooled to 0°C, di-t-butylisobutylsilane triflate (1.00 g, 2.87 mmol) was added dropwise thereto and the mixture was stirred for 3 hours. The obtained reaction mixture was washed with 5% by mass aqueous sodium hydrogen carbonate solution (10.0 g) and an aqueous saturated ammonium chloride solution (10.0 g) in this order, and the obtained organic layer was concentrated and purified by silica gel column chromatography to obtain Boc-MePhe-OBIBS (1.32 g, Yield: 95.9%) as a colorless liquid.
   MASS (ESI+) m/z; 478.46 (M+H)+
(ii) Boc-MePhe-OBIBS (0.10 g, 0.21 mmol) was mixed with methylene chloride (2.7 g), the mixture was cooled to 0°C, 15% by mass hydrogen chloride-1,4-dioxane (0.82 g, 3.2 mmol) was added thereto, and the mixture was stirred at 5°C for 25 hours. Water was added to the obtained reaction mixture (6.0 g) and the liquids were separated, and the organic layer was washed with 8% by mass aqueous sodium hydrogen carbonate solution (2.0 g). The obtained organic layer was concentrated to obtain H-MePhe-OBIBS (0.078 g, Yield: 99%) as a white solid.

### Synthetic Example 7: Synthesis of Boc-Ala-MePhe-MePhe-OBIBS

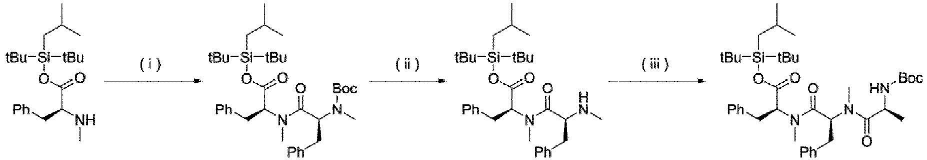

(i) Boc-MePhe-OH (0.50 g, 1.79 mmol) and 1-ethyl-3-(3-dimethylaminopropyl)-carbodiimide hydrochloride (0.35 g, 1.83 mmol) were mixed with methylene chloride (8.9 g), the mixture was cooled to 0°C, a methylene chloride solution (3.1 g) of H-MePhe-OBIBS (0.42 g, 1.12 mmol) was added thereto and after the mixture was stirred at 0°C for 3.5 hours, Boc-MePhe-OH (0.17 g, 0.60 mmol) and 1-ethyl-3-(3-dimethylaminopropyl)carbodiimide hydrochloride (0.12 g, 0.61 mmol) were added thereto and the mixture was stirred for 18 hours. To the obtained reaction mixture was added 2M hydrochloric acid (2.3 mL, 4.6 mmol) and the liquids were separated. The obtained organic layer was concentrated and purified by silica gel column chromatography to obtain Boc-MePhe-MePhe-OBIBS (0.73 g, Yield: 102%) as a colorless liquid.
   MASS (ESI+) m/z; 639.56 (M+H)+
(ii) Boc-MePhe-MePhe-OBIBS (0.32 g, 0.50 mmol) was mixed with methylene chloride, the mixture was cooled to 0°C, 15% by mass hydrogen chloride-1,4-dioxane (3.3 g, 12.8 mmol) was added thereto and the mixture was stirred at 5°C for 20 hours. Water was added to the obtained reaction mixture (6.0 g) and the liquids were separated, and the organic layer was washed with 8% by mass aqueous sodium hydrogen carbonate solution (4.0 g) and water (10.0 g) in this order. The obtained organic layer was concentrated to obtain H-MePhe-MePhe-OBIBS (0.25 g, Yield: 92%) as a colorless liquid.
   MASS (ESI+) m/z; 539.45 (M+H)+
(iii) Boc-Ala-OH (0.16 g, 0.87 mmol) and 1-ethyl-3-(3-dimethylaminopropyl)-carbodiimide hydrochloride (0.17 g, 0.88 mmol) were mixed with methylene chloride (3.7 g), the mixture was cooled to 0°C, a methylene chloride solution (3.2 g) of H-MePhe-MePhe-OBIBS (0.21 g, 0.40 mmol) was added thereto, and then, the mixture was stirred at 0°C for 3 hours. To the obtained reaction mixture was added 1M hydrochloric acid (2.3 mL, 2.3 mmol) and the liquids were separated, and the organic layer was washed with 8% by mass aqueous sodium hydrogen carbonate solution (2.3 g). The obtained organic layer was concentrated and purified by silica gel column chromatography to obtain Boc-Ala-MePhe-MePhe-OBIBS (0.24 g, Yield: 85%) as a white solid.
   MASS (ESI+) m/z; 710.57 (M+H)+

### Synthetic Example 8: Synthesis of Boc-Ala-MePhe-MePhe-OH

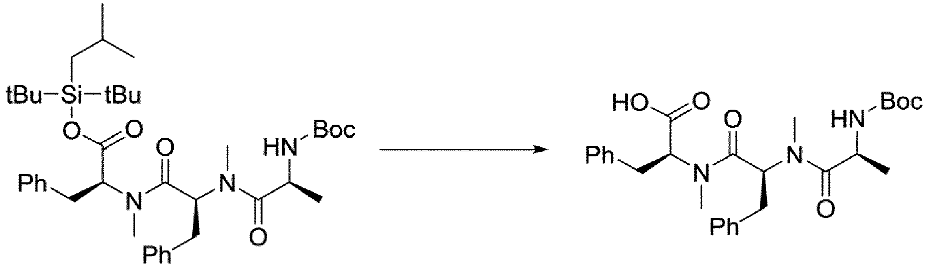

Boc-Ala-MePhe-MePhe-OBIBS (0.093 g, 0.13 mmol) was mixed with methanol (0.40 g) and tetrahydrofuran (1.3 g), the mixture was cooled to 0°C, potassium fluoride (0.015 g, 0.26 mmol) was added thereto, and then, the mixture was stirred for 18 hours. To the obtained reaction mixture were added an aqueous saturated sodium chloride solution (1.0 g), water (4.0 g) and methylene chloride (5.4 g), the liquids were separated, and methylene chloride (5.4 g) was added to the aqueous layer to carry out extraction. The organic layers were combined and washed with 0.25M hydrochloric acid (8.0 mL). The obtained organic layer was concentrated and purified by silica gel column chromatography to obtain Boc-Ala-MePhe-MePhe-OH (0.064 g, Yield: 96%) as a colorless liquid.
MASS (ESI+) m/z; 534.31 (M+Na)+

### Synthetic Example 9: Synthesis of H-Arg(Ts)-OBIBS

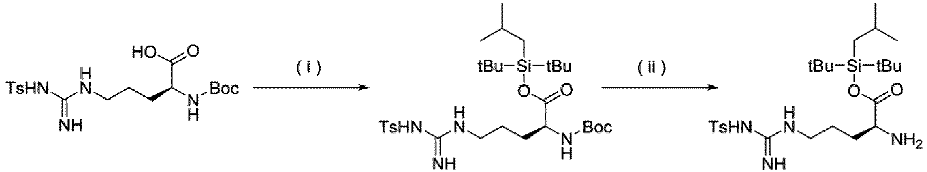

(i) Boc-Arg(Ts)-OH (0.80 g, 1.87 mmol) and N,N-diisopropylethylamine (0.29 g, 2.24 mmol) were mixed with methylene chloride (16.0 g), the mixture was cooled to 0°C, di-t-butylisobutylsilane triflate (0.72 g, 2.06 mmol) was added dropwise thereto, then, the mixture was stirred at room temperature for 2 hours, and N,N-diisopropylethylamine (0.12 g, 0.94 mmol) and di-t-butylisobutylsilane triflate (0.33 g, 0.94 mmol) were each added thereto and the mixture was stirred for 1 hour. To the obtained reaction mixture were added water (10.0 g), 10% by mass aqueous citric acid solution (6.0 g), water (6.0 g) in this order, and the obtained organic layer was concentrated and purified by silica gel column chromatography to obtain Boc-Arg(Ts)-OBIBS (0.98 g, Yield: 83%) as a colorless liquid.
   ¹H-NMR (CDCl₃)
   δ ppm: 0.86 (2H, d, J=6.7Hz), 0.95 (6H, d, J=6.4Hz), 1.04 (9H, s), 1.04 (9H, s), 1.41 (9H, s), 1.55-1.72 (3H, m), 1.80-2.09 (2H, m), 2.39 (3H, s), 3.05-3.50 (2H, br), 4.15-4.30 (1H, m), 5.32 (1H, d, J=8.3Hz), 6.15-6.95 (2H, br), 7.21 (2H, d, J=8.0Hz), 7.74 (2H, d, J=8.0Hz)
   MASS (ESI+) m/z; 627.5 (M+H)+
(ii) Boc-Arg(Ts)-OBIBS (0.63 g, 1.00 mmol) was mixed with methylene chloride (12.6 g), the mixture was cooled to 0°C, 15% by mass hydrogen chloride-1,4-dioxane (1.22 g, 5.00 mmol) was added thereto, then, the mixture was stirred at 0°C for 12.5 hours, and 15% by mass hydrogen chloride-1,4-dioxane (1.22 g, 5.00 mmol) was added thereto, and the mixture was stirred for 4 hours. To the obtained reaction mixture was added 5% by mass aqueous sodium hydrogen carbonate solution (10.1 g) and the liquids were separated, and the organic layer was further washed with water (8.0 g) twice. The obtained organic layer was concentrated and purified by silica gel column chromatography to obtain H-Arg(Ts)-OBIBS (0.50 g, Yield: 95%) as a white solid.
   ¹H-NMR (CDCl₃)
   δ ppm: 0.87 (2H, d, J=6.9Hz), 0.94 (3H, d, J=6.6Hz), 0.95 (3H, d, J=6.6Hz), 1.04 (9H, s), 1.04 (9H, s), 1.41 (9H, s), 1.55-2.09 (5H, m), 2.38 (3H, s), 3.12-3.25 (2H, br), 3.43 (1H, dd, J=8.71, 4.51Hz), 6.28-6.75 (2H, br), 7.22 (2H, d, J=8.1Hz), 7.76 (2H, d, J=8.1Hz)
   MASS (ESI+) m/z; 527.4 (M+H)+

### Synthetic Example 10: Synthesis of Boc-Arg(Ts)-Ala-Arg(Ts)-OBIBS

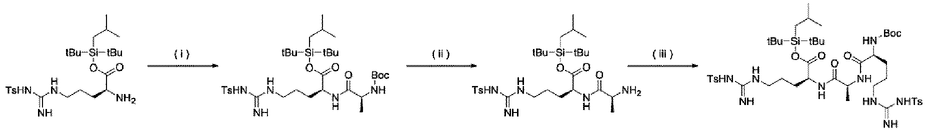

(i) H-Arg(Ts)-OBIBS (0.49 g, 0.93 mmol) and Boc-Ala-OH (0.35 g, 1.86 mmol) were mixed with methylene chloride (19.6 g), the mixture was cooled to 0°C, 1-ethyl-3-(3-dimethylaminopropyl)carbodiimide hydrochloride (0.36 g, 1.86 mmol) was added thereto, and then, the mixture was stirred at 0°C for 1.5 hours. Water was added to the obtained reaction mixture (10.0 g) and the liquids were separated, and a minute amount of N,N-dimethyl-4-aminopyridine and 5% by mass aqueous sodium hydrogen carbonate solution (5.0 g) were added thereto and the liquids were separated. The obtained organic layer was further washed with water (10.0 g), the organic layer was concentrated and purified by silica gel column chromatography to obtain Boc-Ala-Arg(Ts)-OBIBS (0.64 g, Yield: 99%) as a white solid.
   ¹H-NMR (CDCl₃)
   δ ppm: 0.86 (2H, d, J=6.6Hz), 0.94 (3H, d, J=6.6Hz), 0.94 (3H, d, J=6.6Hz), 1.04 (9H, s), 1.04 (9H, s), 1.34 (3H, d, J=7.2Hz), 1.41 (9H, s), 1.55-1.72 (3H, m), 1.90-2.09 (2H, m), 2.39 (3H, s), 3.05-3.50 (2H, br), 4.05-4.20 (1H, m), 4.45-4.55 (1H, m), 5.12 (1H, br), 6.20-6.55 (3H, br), 6.91 (1H, d, J=8.4Hz), 7.21 (2H, d, J=8.4Hz), 7.75 (2H, d, J=8.4Hz)
   MASS (ESI+) m/z; 698.6 (M+H)+
(ii) Boc-Ala-Arg(Ts)-OBIBS (0.63 g, 0.90 mmol) was mixed with methylene chloride (12.6 g), the mixture was cooled to 0°C, 15% by mass hydrogen chloride-1,4-dioxane solution (1.10 g, 4.50 mmol) was added thereto, then, the mixture was stirred at 5°C for 16.5 hours, 5% by mass aqueous sodium hydrogen carbonate solution (9.1 g) was added thereto and the liquids were separated, and the organic layer was further washed with 10% by mass brine solution twice. The obtained organic layer was dried over magnesium sulfate and filtered, and the obtained solution was concentrated to obtain H-Ala-Arg(Ts)-OBIBS (0.50 g, Yield: 93%) as a white solid.
   ¹H-NMR (CDCl₃)
   δ ppm: 0.88 (2H, d, J=6.9Hz), 0.94 (3H, d, J=6.6Hz), 0.95 (3H, d, J=6.6Hz), 1.04 (9H, s), 1.05 (9H, s), 1.34 (3H, d, J=7.2Hz), 1.50-1.80 (3H, m), 1.90-2.09 (2H, m), 2.38 (3H, s), 3.00-3.60 (2H, br), 3.54 (1H, dd, J=6.6, 13.8Hz), 4.46-4.52 (1H, m), 5.12 (1H, br), 6.35-6.90 (3H, br), 7.21 (2H, d, J=8.4Hz), 7.75 (2H, d, J=8.4Hz), 8.11 (1H, br)
   MASS (ESI+) m/z; 598.4 (M+H)+
(iii) H-Ala-Arg(Ts)-OBIBS (0.47 g, 0.79 mmol) and Boc-Arg(Ts)-OH (0.51 g, 1.19 mmol) were mixed with methylene chloride (18.8 g), the mixture was cooled to 0°C, 1-ethyl-3-(3-dimethylaminopropyl)carbodiimide hydrochloride (0.23 g, 1.19 mmol) was added thereto, and then, the mixture was stirred at 0°C for 2 hours. Water was added to the obtained reaction mixture (10.0 g) and the liquids were separated, a minute amount of N,N-dimethyl-4-aminopyridine, 5% by mass aqueous sodium hydrogen carbonate solution, 10% by mass brine solution and 5% by mass aqueous citric acid solution were added thereto and the liquids were separated. The obtained organic layer was further washed with water and brine solution, and after concentrating the organic layer, the concentrate was purified by silica gel column chromatography to obtain Boc-Arg(Ts)-Ala-Arg(Ts)-OBIBS (0.72 g, Yield: 91%) as a white solid.
   ¹H-NMR (CDCl₃)
   δ ppm: 0.85 (2H, d, J=6.6Hz), 0.93 (6H, d, J=6.6Hz), 1.02 (18H, s), 1.42 (12H, s), 1.55-1.75 (7H, m), 1.85-2.09 (2H, m), 2.39 (6H, s), 2.80-3.90 (4H, br), 4.20-4.55 (3H, br), 5.85-7.00 (6H, br), 7.21 (2H, d, J=8.1Hz), 7.45-7.80 (5H, br)
   MASS (ESI+) m/z; 1008.6 (M+H)+

### Synthetic Example 11: Synthesis of Boc-Arg(Ts)-Arg(Ts)-Ala-Arg(Ts)-OBIBS

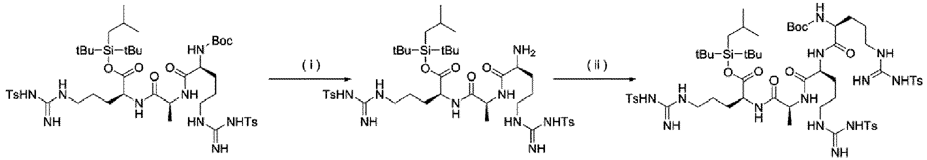

(i) Boc-Arg(Ts)-Arg(Ts)-Ala-Arg(Ts)-OBIBS (0.69 g, 0.68 mmol) was mixed with methylene chloride (13.7 g), the mixture was cooled to 5°C, 15% by mass hydrogen chloride-1,4-dioxane solution (0.83 g, 3.4 mmol) was added thereto, and then, the mixture was stirred at 5°C for 19 hours, 5% by mass aqueous sodium hydrogen carbonate solution was added thereto and the liquids were separated, to the obtained organic layer was further added 10% by mass brine solution, 5% by mass aqueous sodium hydrogen carbonate solution and an aqueous ammonia and the liquids were separated, and the organic layer was washed with 10% by mass brine solution. After the obtained organic layer was dried over magnesium sulfate, and the solution obtained by filtration was concentrated to obtain H-Arg(Ts)-Ala-Arg(Ts)-OBIBS (0.58 g, Yield: 94%) as a white solid.
   ¹H-NMR (CDCl₃)
   δ ppm: 0.84 (2H, d, J=6.6Hz), 0.92 (6H, d, J=6.6Hz), 1.02 (18H, s), 1.39 (12H, s), 1.45-1.68 (16H, m), 2.36 (9H, s), 3.0-3.60 (7H, br), 4.00-4.90 (3H, br), 6.00-7.10 (9H, br), 7.18 (7H, d, J=7.8Hz), 7.68 (8H, m)
   MASS (ESI+) m/z; 908.6 (M+H)+
(ii) H-Arg(Ts)-Ala-Arg(Ts)-OBIBS (0.57 g, 0.63 mmol) and Boc-Arg(Ts)-OH (0.40 g, 0.95 mmol) were mixed with methylene chloride (22.8 g), the mixture was cooled to 1°C, 1-ethyl-3-(3-dimethylaminopropyl)carbodiimide hydrochloride (0.18 g, 0.95 mmol) was added thereto and the mixture was stirred at 0°C for 1.5 hours. Water was added to the obtained reaction mixture and the liquids were separated, and the obtained organic layer was concentrated and purified by silica gel column chromatography to obtain Boc-Arg(Ts)-Arg(Ts)-Ala-Arg(Ts)-OBIBS (0.64 g, Yield: 78%) as a white solid.
   ¹H-NMR (CDCl₃)
   δ ppm: 0.85 (2H, d, J=6.6Hz), 0.93 (6H, d, J=6.6Hz), 1.02 (18H, s), 1.42 (12H, s), 1.55-1.75 (7H, m), 1.85-2.09 (2H, m), 2.39 (6H, s), 2.80-3.90 (4H, br), 4.20-4.55 (3H, br), 5.85-7.00 (6H, br), 7.21 (2H, d, J=8.1Hz), 7.45-7.80 (5H, br)
   MASS (ESI+) m/z; 1318.6 (M+H)+

### Synthetic Example 12: Synthesis of Boc-Arg(Ts)-Arg(Ts)-Arg(Ts)-Ala-Arg(Ts)-OBIBS

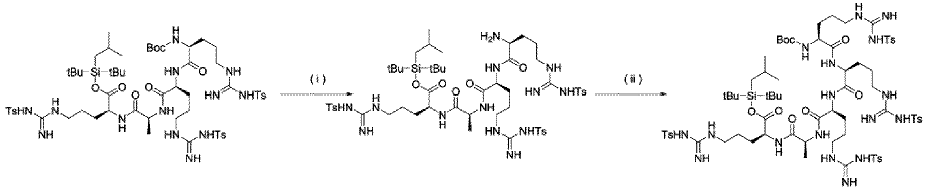

(i) Boc-Arg(Ts)-Arg(Ts)-Ala-Arg(Ts)-OBIBS (0.63 g, 0.48 mmol) was mixed with methylene chloride (12.6 g), the mixture was cooled to 0°C, 15% by mass hydrogen chloride-1,4-dioxane solution (0.58 g, 2.4 mmol) was added thereto, and then, the mixture was stirred at 5°C for 19.5 hours, and after adding 15% by mass hydrogen chloride-1,4-dioxane solution (0.31 g, 1.3 mmol) thereto, the mixture was further stirred for 3 hours. 5% by mass aqueous sodium hydrogen carbonate solution was added to the mixture, the liquids were separated and the obtained solution was concentrated to obtain H-Arg(Ts)-Arg(Ts)-Ala-Arg(Ts)-OBIBS (0.60 g, Yield: 104%) as a white solid.
   ¹H-NMR (CDCl₃)
   δ ppm: 0.84 (2H, d, J=6.6Hz), 0.91 (6H, d, J=6.6Hz), 1.01 (18H, s), 1.31 (3H, d, J=6.6Hz), 1.75-2.10 (17H, m), 2.36 (9H, s), 3.00-3.60 (7H, br), 4.00-4.85 (3H, br), 6.00-7.10 (9H, br), 7.19 (7H, d, J=7.2Hz), 7.4-8.4 (8H, br)
   MASS (ESI+) m/z; 1218.6 (M+H)+
(ii) H-Arg(Ts)-Arg(Ts)-Ala-Arg(Ts)-OBIBS (0.58 g, 0.48 mmol), Boc-Arg(Ts)-OH (0.31 g, 0.72 mmol) was mixed with methylene chloride (23.3 g), the mixture was cooled to 0°C, 1-ethyl-3-(3-dimethylaminopropyl)carbodiimide hydrochloride (0.14 g, 0.72 mmol) was added thereto and the mixture was stirred at 0°C for 1 hour. Water was added to the obtained reaction mixture, the liquids were separated, and the obtained organic layer was concentrated and purified by silica gel column chromatography to obtain Boc-Arg(Ts)-Arg(Ts)-Arg(Ts)-Ala-Arg(Ts)-OBIBS (0.67 g, Yield: 86%) as a white solid.
   ¹H-NMR (CDCl₃)
   δ ppm: 0.84 (2H, d, J=6.0Hz), 0.91 (6H, d, J=5.8Hz), 1.00 (18H, s), 1.38 (12H, br), 1.45-2.09 (16H, br), 2.36 (12H, s), 2.80-3.40 (8H, br), 3.90-4.85 (4H, br), 5.65-7.10 (12H, br), 7.19 (8H, d, J=7.2Hz), 7.4-8.1 (11H, br)
   MASS (ESI+) m/z; 1628.7 (M+H)+

### Synthetic Example 13: Synthesis of Boc-Ala-Arg(Ts)-Arg(Ts)-Arg(Ts)-Ala-Arg(Ts)-OBIBS

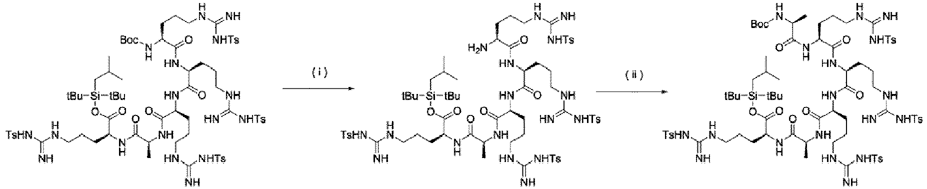

(i) Boc-Arg(Ts)-Arg(Ts)-Arg(Ts)-Ala-Arg(Ts)-OBIBS (0.66g,0.41 mmol) was mixed with methylene chloride (13.2 g), the mixture was cooled to 5°C, 15% by mass hydrogen chloride-1,4-dioxane solution (0.79 g, 3.3 mmol) was added thereto and the mixture was stirred at 5°C for 17.5 hours. 5% by mass aqueous sodium hydrogen carbonate solution, the liquids were separated and the obtained solution was concentrated to obtain H-Arg(Ts)-Arg(Ts)-Arg(Ts)-Ala-Arg(Ts)-OBIBS (0.59 g, Yield: 95%) as a white solid.
   ¹H-NMR (CDCl₃)
   δ ppm: 0.84 (2H, d, J=6.6Hz), 0.90 (6H, d, J=6.6Hz), 0.99 (18H, s), 1.29 (3H, br), 1.7-2.1 (18H, m), 2.33 (9H, s), 2.7-3.6 (9H, br), 3.9-4.9 (4H, br), 5.9-6.9 (11H, br), 6.9-7.2 (10H, br), 7.4-8.5 (12H, br)
   MASS (ESI+) m/z; 1528.7 (M+1)+
(ii) H-Arg(Ts)-Arg(Ts)-Arg(Ts)-Ala-Arg(Ts)-OBIBS (0.57 g, 0.37 mmol) and Boc-Arg(Ts)-OH (0.11 g, 0.56 mmol) were mixed with methylene chloride (22.8 g), the mixture was cooled to 0°C, 1-ethyl-3-(3-dimethylaminopropyl)carbodiimide hydrochloride (0.11 g, 0.56 mmol) was added thereto and the mixture was stirred at 0°C for 1 hour. Water was added to the obtained reaction mixture and the liquids were separated, the organic layer was washed with a saturated brine solution, then, concentrated and purified by silica gel column chromatography to obtain Boc-Ala-Arg(Ts)-Arg(Ts)-Arg(Ts)-Ala-Arg(Ts)-OBIBS (0.645 g, Yield: 102%) as a white solid.
   ¹H-NMR (CDCl₃)
   δ ppm: 0.83 (2H, d, J=6.3Hz), 0.91 (6H, d, J=6.0Hz), 1.00 (18H, s), 1.3-1.5 (15H, br), 1.6-2.1 (17H, br), 2.36 (12H, s), 2.7-3.90 (8H, br), 4.2-4.8 (6H, br), 5.8-7.1 (11H, br), 7.18 (9H, d, J=6.9Hz), 7.4-8.1 (12H, br)
   MASS (ESI+) m/z; 1699.7 (M+H)+

### Synthetic Example 14: Synthesis of Fmoc-Cys(Trt)-Ala-Arg(Ts)-Arg(Ts)-Arg(Ts)-Ala-Arg(Ts)-OBIBS

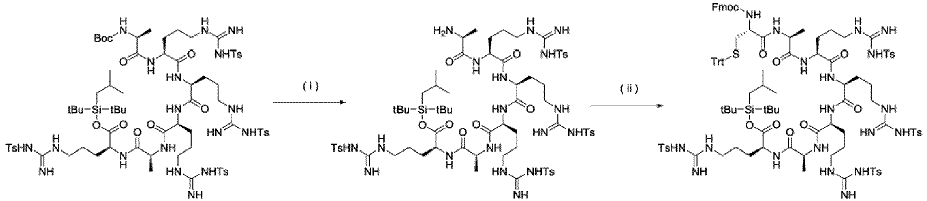

(i) Boc-Ala-Arg(Ts)-Arg(Ts)-Arg(Ts)-Ala-Arg(Ts)-OBIBS (0.63 g, 0.37 mmol) was mixed with methylene chloride (12.6 g), the mixture was cooled to 6°C, 15% by mass hydrogen chloride-1,4-dioxane solution (0.72 g, 3.0 mmol) was added thereto and the mixture was stirred at 6°C for 15 hours. 5% by mass aqueous sodium hydrogen carbonate solution was added to the mixture and the liquids were separated, and the organic layer was washed with a saturated brine solution and the obtained solution was concentrated to obtain H-Ala-Arg(Ts)-Arg(Ts)-Arg(Ts)-Ala-Arg(Ts)-OBIBS (0.58 g, Yield: 98%) as a white solid.
   ¹H-NMR (CDCl₃)
   δ ppm: 0.83 (2H, d, J=6.6Hz), 0.90 (6H, d, J=6.3Hz), 0.99 (18H, s), 1.27 (6H, br), 1.7-2.1 (20H, m), 2.34 (12H, s), 2.7-3.6 (10H, br), 3.9-4.9 (4H, br), 5.9-6.9 (12H, br), 6.9-7.2 (10H, br), 7.4-8.5 (13H, br)
   MASS (ESI+) m/z; 1599.7 (M+H)+
(ii) H-Ala-Arg(Ts)-Arg(Ts)-Arg(Ts)-Ala-Arg(Ts)-OBIBS (0.57 g, 0.36 mmol) and Fmoc-Cys(Trt)-OH (0.31 g, 0.54 mmol) were mixed with methylene chloride (22.8 g), the mixture was cooled to 0°C, 1-ethyl-3-(3-dimethylaminopropyl)carbodiimide hydrochloride (0.10g,0.54 mmol) was added thereto and the mixture was stirred at 0°C for 2 hours. Water was added to the obtained reaction mixture and the liquids were separated, the organic layer was concentrated, solvent substitution to ethyl acetate was carried out twice and precipitated solid was collected by filtration and dried to obtain Fmoc-Cys(Trt)-Ala-Arg(Ts)-Arg(Ts)-Arg(Ts)-Ala-Arg(Ts)-OBIBS (0.71 g, Yield: 92%) as a white solid.
   ¹H-NMR (DMSO-d6)
   δ ppm: 0.81 (2H, d, J=6.9Hz), 0.90 (6H, d, J=6.6Hz), 0.99 (18H, s), 1.16 (6H, m), 1.3-1.8 (16H, br), 1.95 (1H, m), 2.31 (12H, br), 2.41 (2H, m), 2.9-3.2 (8H, br), 4.1-4.4 (10H, m), 6.4-7.1 (9H, br), 7.27 (30H, m), 7.6-7.8 (12H, m), 7.8-8.1 (6H, br), 8.24 (1H, br)
   MASS (ESI+) m/z; 2166.9 (M+H)+

### Synthetic Example 15: Synthesis of Ac-Cys(Trt)-Ala-Arg(Ts)-Arg(Ts)-Arg(Ts)-Ala-Arg(Ts)-OBIBS

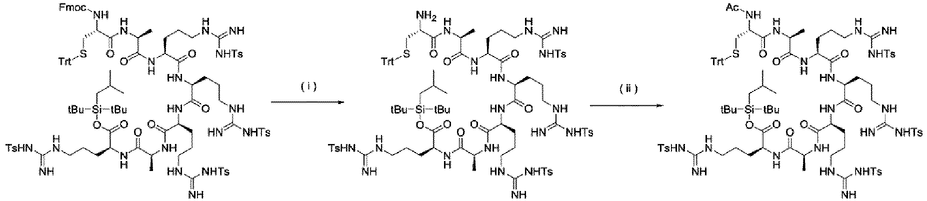

(i) Fmoc-Cys(Trt)-Ala-Arg(Ts)-Arg(Ts)-Arg(Ts)-Ala-Arg(Ts)-OBIBS (0.59g,0.27 mmol) was mixed with methylene chloride (11.7 g) at room temperature, and after adding diethylamine (0.99 g, 13.5 mmol) thereto, the mixture was stirred for 16.5 hours. 20% by mass aqueous ammonium chloride solution was added to the mixture and the liquids were separated twice, the organic layer was washed with 5% by mass aqueous sodium hydrogen carbonate solution and a saturated brine solution, and the organic layer was dried over magnesium sulfate, solvent substitution to ethyl acetate was carried out twice and the obtained solid was collected by filtration and dried to obtain H-Cys(Trt)-Ala-Arg(Ts)-Arg(Ts)-Arg(Ts)-Ala-Arg(Ts)-OBIBS (0.43 g, Yield: 81%) as a white solid.
   ¹H-NMR (DMSO-d6)
   δ ppm: 0.81 (2H, d, J=6.3Hz), 0.90 (6H, d, J=6.6Hz), 0.99 (18H, s), 1.16 (6H, m), 1.3-1.8 (17H, br), 1.93 (1H, m), 2.31 (14H, br), 2.9-3.2 (9H, br), 4.1-4.4 (6H, m), 6.4-7.1 (9H, br), 7.27 (27H, m), 7.61 (9H, m), 7.8-8.1 (5H, br), 8.21 (1H, br),
   MASS (ESI+) m/z; 1944.8 (M+H)+
(ii) H-Cys(Trt)-Ala-Arg(Ts)-Arg(Ts)-Arg(Ts)-Ala-Arg(Ts)-OBIBS (0.37 g, 0.19 mmol) and N,N-dimethyl-4-aminopyridine (0.14 g, 1.14 mmol) were mixed with methylene chloride (14.8 g), the mixture was cooled to 0°C, acetic anhydride (0.14 g, 0.54 mmol) was added thereto and the mixture was stirred at 0°C for 2.5 hours. Water was added to the obtained reaction mixture, the liquids were separated, and the organic layer was washed with a saturated brine solution and concentrated, solvent substitution to ethyl acetate was carried out twice and the precipitated solid was collected by filtration and dried to obtain Ac-Cys(Trt)-Ala-Arg(Ts)-Arg(Ts)-Arg(Ts)-Ala-Arg(Ts)-OBIBS (0.38 g, Yield: 100%) as a white solid.
   ¹H-NMR (DMSO-d6)
   δ ppm: 0.81 (2H, d, J=6.6Hz), 0.90 (6H, d, J=6.3Hz), 0.99 (18H, s), 1.16 (6H, m), 1.3-1.8 (17H, br), 1.84 (3H, s), 1.95 (1H, m), 2.32 (14H, m), 2.9-3.2 (8H, br), 4.1-4.4 (7H, m), 6.4-7.1 (9H, br), 7.27 (28H, m), 7.62 (8H, m), 7.8-8.1 (5H, br), 8.1-8.3 (1H, br)
   MASS (ESI+) m/z; 1986.8 (M+H)+

### Synthetic Example 16: Synthesis of Ac-Cys(Trt)-Ala-Arg(Ts)-Arg(Ts)-Arg(Ts)-Ala-Arg(Ts)-OH

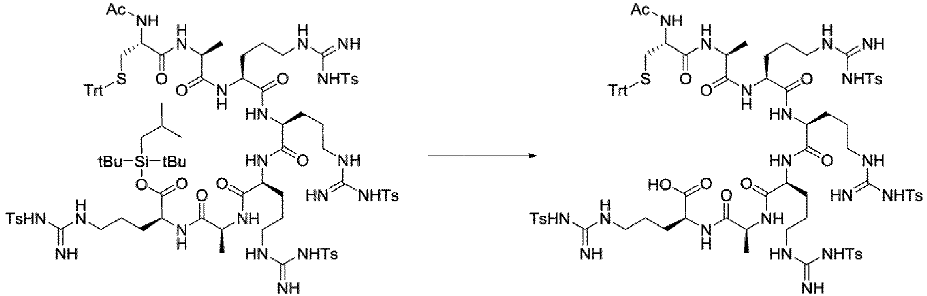

Ac-Cys(Trt)-Ala-Arg(Ts)-Arg(Ts)-Arg(Ts)-Ala-Arg(Ts)-OBIBS (0.36 g, 0.18 mmol) was mixed with methanol (7.2 g) at room temperature, cesium fluoride (0.14 g, 0.90 mmol) was added thereto and the mixture was stirred for 23 hours. Phosphoric acid was added thereto and the mixture was stirred for 1 hour, then, the solid was collected by filtration and dried, and the obtained solid was suspended and washed with water and then collected by filtration and dried to obtain Ac-Cys(Trt)-Ala-Arg(Ts)-Arg(Ts)-Arg(Ts)-Ala-Arg(Ts)-OH (0.27 g, Yield: 85%) as a white solid.
¹H-NMR (DMSO-d6)
δ ppm: 1.15 (3H, d, J=7.2Hz), 1.21 (3H, d, J=7.5Hz), 1.3-1.8 (16H, br), 1.84 (3H, s), 2.32 (14H, m), 2.9-3.2 (8H, br), 3.69 (1H, m), 4.1-4.4 (6H, br), 6.4-7.1 (8H, br), 7.27 (26H, m), 7.62 (9H, m), 7.8-8.2 (6H, br), 8.2-8.3 (1H, br)
MASS (ESI+) m/z; 1788.7 (M+H)+

### Synthetic Example 17: Synthesis of H-Phe-Phe-O(t-Bu)Silolane

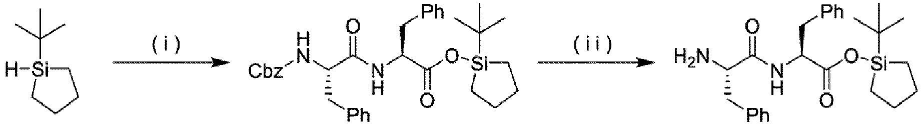

(i) 1-(t-Butyl)silolane (0.75 g, 5.29 mmol) was dissolved in methylene chloride (20.9 g), trifluoromethanesulfonic acid (0.69 g, 4.6 mmol) was added thereto at 0°C, and the mixture was stirred at room temperature for 2 hours. The obtained reaction mixture was added to a methylene chloride (10.6 g) solution of Cbz-Phe-Phe-OH (1.57 g, 3.51 mmol) and imidazole (0.36 g, 5.27 mmol) at 0°C, and after returning to room temperature, the mixture was stirring for 1.5 hours. To the obtained reaction mixture were added an aqueous saturated ammonium chloride solution (8.0 g) and water (8.0 g), and the liquids were separated. The obtained organic layer was concentrated and purified by silica gel column chromatography to obtain Cbz-Phe-Phe-O(t-Bu)Silolane (1.59 g, Yield: 77%) as a white solid.
   MASS (ESI+) m/z; 587.36 (M+H)+
(ii) Cbz-Phe-Phe-O(t-Bu)Silolane (0.75 g, 1.71 mmol) was dissolved in 2,2,2-trifluoroethanol (10.4 g), 10% by mass Pd-C (80 mg, 0.08 mmol) was added thereto and the mixture was stirred under a hydrogen gas atmosphere for 1 hour. The reaction mixture was filtered and the obtained filtrate was concentrated to obtain H-Phe-Phe-O(t-Bu)Silolane(0.63 g, Yield: 100%) as a brown oil.
   MASS (ESI+) m/z; 453.38 (M+H)+

### Synthetic Example 18: Synthesis of Fmoc-Ser(Bn)-Ala-Phe-Phe-O(t-Bu)Silolane

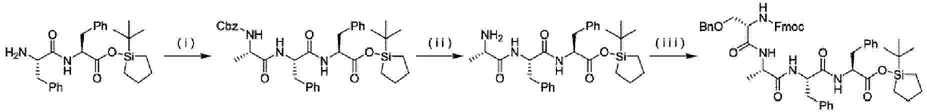

(i) H-Phe-Phe-O(t-Bu)Silolane (0.58 g, 1.28 mmol) and Cbz-Ala-OH (0.44 g, 1.96 mmol) were dissolved in methylene chloride (7.7 g), 1-ethyl-3-(3-dimethylaminopropyl)carbodiimide hydrochloride (0.35 g, 1.84 mmol) was added thereto under ice-cooling and the mixture was stirred for 1.5 hours. The obtained reaction mixture was washed with 4% by mass hydrochloric acid (5.8 g) and an aqueous saturated sodium hydrogen carbonate solution (5.8 g) in this order, and the obtained organic layer was concentrated and purified by silica gel column chromatography to obtain Cbz-Ala-Phe-Phe-O(t-Bu)Silolane (0.535 g, Yield: 65%) as a white solid.
   MASS (ESI+) m/z; 658.41 (M+H)+
(ii) Cbz-Ala-Phe-Phe-O(t-Bu)Silolane (0.50 g, 0.77 mmol) was dissolved in 2,2,2-trifluoroethanol (8.3 g), 10% by mass Pd-C (55 mg, 0.052 mmol) was added thereto and the mixture was stirred under a hydrogen gas atmosphere for 2.5 hours. After filtering the reaction mixture, the obtained filtrate was concentrated to obtain H-Ala-Phe-Phe-O(t-Bu)Silolane (0.440 g, Yield: 110%) as a brown solid.
   MASS (ESI+) m/z; 524.42 (M+H)+
(iii) H-Ala-Phe-Phe-O(t-Bu)Silolane (0.401 g, 0.766 mmol) and Fmoc-Ser(Bn)-OH (0.484 g, 1.16 mmol) were dissolved in methylene chloride (5.3 g), and 1-ethyl-3-(3-dimethylaminopropyl)carbodiimide hydrochloride (0.224 g, 1.17 mmol) was added thereto under ice-cooling and the mixture was stirred for 1 hour. The obtained reaction mixture was washed with 4% by mass hydrochloric acid (4.0 g) and an aqueous saturated sodium hydrogen carbonate solution (4.0 g) in this order, and the obtained organic layer was concentrated and purified by silica gel column chromatography to obtain Fmoc-Ser(Bn)-Ala-Phe-Phe-O(t-Bu)Silolane (0.213 g, Yield: 30%) as a colorless solid.
   MASS (ESI+) m/z; 923.57 (M+H)+

### Synthetic Example 19: Synthesis of H-Phe-Phe-OTIPS

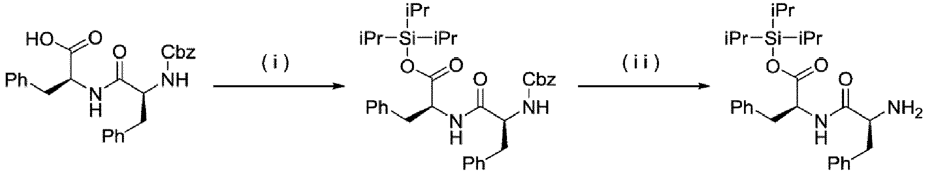

(i) Cbz-Phe-Phe-OH (1.01 g, 2.26 mmol) and TIPS-Cl (0.55 g, 2.85 mmol) were mixed with methylene chloride (20.2 g), the mixture was cooled to 0°C, and imidazole (0.18 g, 2.64 mmol) was added thereto and the mixture was stirred for 2 hours. After returned to room temperature and stirring for 2 hours, chloroform and water were added to the obtained reaction mixture and the liquids were separated. The obtained organic layer was concentrated and purified by silica gel column chromatography to obtain Cbz-Phe-Phe-OTIPS (1.20 g, Yield: 88%) as a white solid.
   MASS (ESI+) m/z; 603.32 (M+H)+
(ii) Cbz-Phe-Phe-OTIPS (0.50 g, 0.83 mmol) was mixed with 2,2,2-trifluoroethanol (5.0 g) and t-butyl methyl ether (10.0 g), 10% by mass Pd-C (0.30 g) was added thereto and the mixture was stirred under a hydrogen gas atmosphere at room temperature for 5 hours. After filtering the reaction mixture, the obtained filtrate was concentrated to obtain H-Phe-Phe-OTIPS (0.40 g, Yield: 100%) as a colorless oil.
   MASS (ESI+) m/z; 616.35 (M+H)+

### Synthetic Example 20: Synthesis of Fmoc-Phe-Phe-Phe-Phe-OTIPS

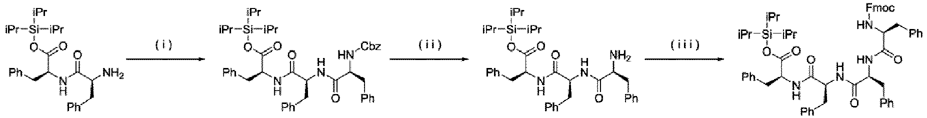

(i) Cbz-Phe-OH (0.23 g, 0.77 mmol) was dissolved in methylene chloride (3.0 g), 1-ethyl-3-(3-dimethylaminopropyl)carbodiimide hydrochloride (0.15 g, 0.78 mmol) was added thereto under ice-cooling and a methylene chloride (3.0 g) solution of H-Phe-Phe-OTIPS (0.27 g, 0.58 mmol) was added dropwise thereto and the mixture was stirred for 2 hours. The obtained reaction mixture was diluted with chloroform, washed with an aqueous saturated ammonium chloride solution and an aqueous saturated sodium hydrogen carbonate solution in this order. The obtained organic layer was concentrated and purified by silica gel column chromatography to obtain Cbz-Phe-Phe-Phe-OTIPS (0.30 g, Yield: 68%) as a white solid.
   MASS (ESI+) m/z; 749.39 (M+H)+
(ii) Cbz-Phe-Phe-Phe-OTIPS (0.25g, 0.33 mmol) was mixed with 2,2,2-trifluoroethanol (5.0 g) and t-butyl methyl ether (2.5 g), 10% by mass Pd-C (0.10 g) was added thereto and the mixture was stirred under a hydrogen gas atmosphere at room temperature for 3 hours. After filtering the reaction mixture, the obtained filtrate was concentrated to obtain H-Phe-Phe-Phe-OTIPS (0.21 g, Yield: 100%) as a colorless oil.
   MASS (ESI+) m/z; 469.29 (M+H)+
(iii) Fmoc-Phe-OH (0.15 g, 0.39 mmol) was dissolved in methylene chloride (2.0 g), 1-ethyl-3-(3-dimethylaminopropyl)carbodiimide hydrochloride (0.08 g, 0.42 mmol) was added thereto under ice-cooling and a methylene chloride (2.5 g) solution of H-Phe-Phe-Phe-OTIPS (0.20 g, 0.32 mmol) was added dropwise thereto and the mixture was stirred for 3 hours. The obtained reaction mixture was diluted with chloroform, and washed with an aqueous saturated ammonium chloride solution and an aqueous saturated sodium hydrogen carbonate solution in this order. The obtained organic layer was concentrated and purified by silica gel column chromatography to obtain Fmoc-Phe-Phe-Phe-Phe-OTIPS (0.25 g, Yield: 77%) as a white solid.
   MASS (ESI+) m/z; 985.49 (M+H)+

### Synthetic Example 21: Synthesis of Cbz-Phe-Phe-OSi(tBu)₂(Me)

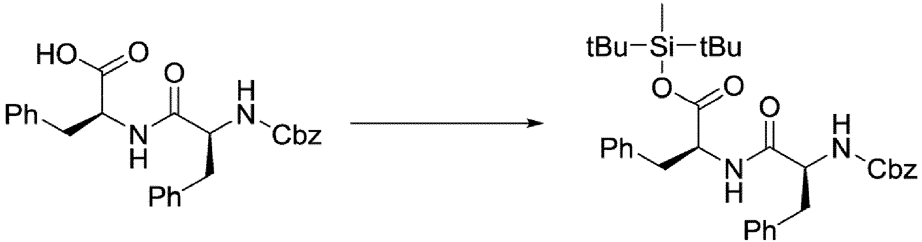

Di-t-butylmethylsilane (0.50 g, 3.16 mmol) was dissolved in methylene chloride (2.5 g), trifluorosulfonic acid (0.47 g, 3.16 mmol) was added thereto at 0°C and the mixture was stirred at room temperature for 1 hour. The obtained reaction mixture was added to a methylene chloride (5.0 g) solution of Cbz-Phe-Phe-OH (1.55 g, 3.48 mmol) and N, N-diisopropylethylamine (0.49 g, 3.79 mmol) at 0°C, and then, the mixture was stirred for 1 hour. The obtained reaction mixture was diluted with t-butyl methyl ether, then, washed with 5% by mass potassium hydrogen carbonate twice and further washed with water. The obtained organic layer was washed with 5% by mass citric acid and further washed with water twice. The obtained organic layer was concentrated to obtain Cbz-Phe-Phe-OSi(tBu)₂(Me) (1.90 g, Yield: 100%) as a colorless oil.
MASS (ESI+) m/z; 603.4 (M+H)+

### Synthetic Example 22: Synthesis of H-Phe-Phe-Phe-OSi(tBu)₂(Me)

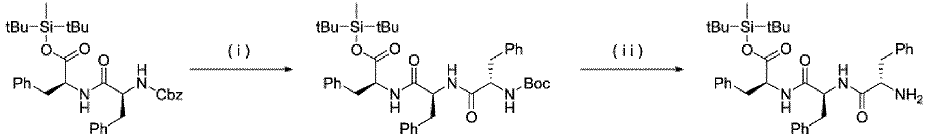

(i) Cbz-Phe-Phe-OSi(tBu)₂(Me) (1.90 g, 3.16 mmol) was dissolved in methylene chloride (9.5 g) and 2,2,2-trifluoroethanol (19.0 g) at 40°C, 10% by mass Pd-C (170 mg) was added thereto and the mixture was stirred under a hydrogen gas atmosphere for 2 hours. After filtering the reaction mixture, the obtained filtrate was concentrated. The obtained concentrate was dissolved in methylene chloride (15.0 g), 1-ethyl-3-(3-dimethylaminopropyl)carbodiimide hydrochloride (0.91 g, 4.74 mmol) and Boc-Phe-OH (1.09 g, 4.11 mmol) were added thereto under ice-cooling and the mixture was stirred for 16 hours. The obtained reaction mixture was diluted with methyl cyclopentyl ether, then, washed with water, 10% by mass aqueous potassium hydrogen carbonate solution (5.0 g) and N,N-dimethyl-4-aminopyridine (0.02 g) were added thereto, and the liquids were separated. The obtained organic layer was washed with water twice, then, washed with 10% by mass aqueous citric acid solution, and further washed with water twice. The obtained organic layer was concentrated and purified by silica gel column chromatography to obtain Boc-Phe-Phe-Phe-OSi(tBu)₂(Me) (2.08 g, Yield: 92%) as a colorless oil.
   MASS (ESI+) m/z; 716.49 (M+H)+
(ii) Boc-Phe-Phe-Phe-OSi(tBu)₂(Me) (1.70 g, 2.37 mmol) was dissolved in methylene chloride (34.0 g), 15% by mass hydrogen chloride-1,4-dioxane (4.62 g, 19.0 mmol) was added thereto and the mixture was stirred for 17 hours. The obtained reaction mixture was washed with 10% by mass aqueous potassium hydrogen carbonate solution and further washed with water twice. The obtained organic layer was concentrated to obtain H-Phe-Phe-Phe-OSi(tBu)₂(Me) (1.24 g, Yield: 85%) as a colorless oil.
   MASS (ESI+) m/z; 616.39 (M+H)+

### Reference Synthetic Example 1: Synthesis of HSi(sBu)₂(tBu)

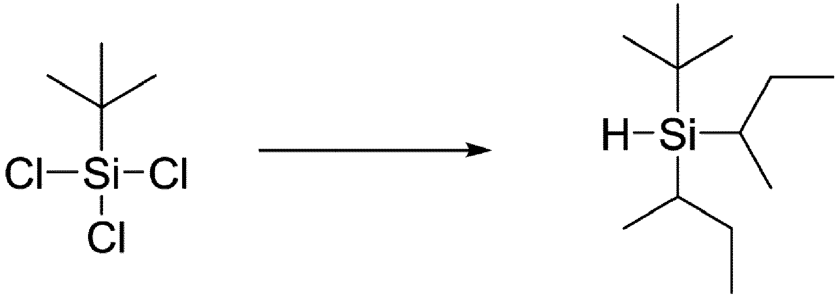

Heptane (6.8 g) and 1.0M s-butyl lithium-hexane solution (17.5mL, 18 mmol) were mixed at 0°C, then, a heptane (6.8 g) solution of t-butyltrichlorosilane (1.00 g, 5.22 mmol) was added thereto and the mixture was stirred at 90°C for 4 hours. The obtained reaction mixture was cooled to 0°C, 2-propanol (0.78 g), heptane (14 g) and water (20 g) were added thereto and the liquids were separated. The obtained organic layer was concentrated and purified by silica gel column chromatography to obtain HSi(sBu)₂(tBu) (0.46 g, Yield: 44%) as a colorless liquid.
¹H-NMR (CDCl₃)
δ ppm: 0.86-0.98 (12H, m), 1.01 (9H, s), 1.07-1.38 (6H, m)

### Synthetic Example 23: Synthesis of H-Phe-OSi(sBu)₂(tBu)

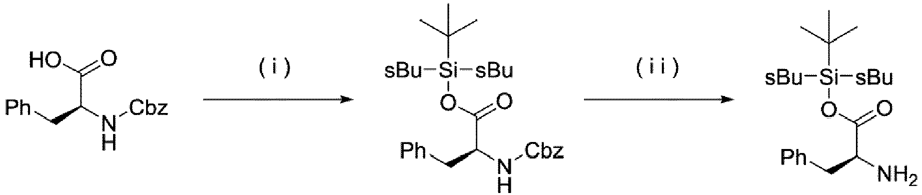

(i) HSi(sBu)₂(tBu) (0.41 g, 2.06 mmol) was dissolved in methylene chloride (6.8 g), trifluoromethanesulfonic acid (0.31 g, 2.1 mmol) was added thereto at 0°C and the mixture was stirred at room temperature for 2 hours. The obtained reaction mixture was added to a methylene chloride (3.3 g) solution of Cbz-Phe-OH (0.51 g, 1.72 mmol) and imidazole (0.18 g, 2.64 mmol) at 0°C, and then, the mixture was stirred at room temperature for 2 hours. To the obtained reaction mixture were added an aqueous saturated ammonium chloride solution (4.0 g) and water (1.0 g), and the liquids were separated. The obtained organic layer was concentrated and purified by silica gel column chromatography to obtain Cbz-Phe-OSi(sBu)₂(tBu) (0.822 g, Yield: 97%) as a colorless liquid.
   MASS (ESI+) m/z; 498.35 (M+H)+
(ii) Cbz-Phe-OSi(sBu)₂(tBu) (0.60 g, 1.20 mmol) was dissolved in 2,2,2-trifluoroethanol (8.3 g), 10% by mass Pd-C (59.3 mg, 0.06 mmol) was added thereto and the mixture was stirred under a hydrogen gas atmosphere for 5 hours. After filtering the reaction mixture, the obtained filtrate was concentrated. The obtained concentrate was purified by silica gel column chromatography to obtain H-Phe-OSi(sBu)₂(tBu) (0.382 g, Yield: 88%) as a colorless liquid.
   MASS (ESI+) m/z; 364.45 (M+H)+

### Synthetic Example 24: Synthesis of Fmoc-Ser(Bn)-Phe-Phe-OSi(sBu)₂(tBu)

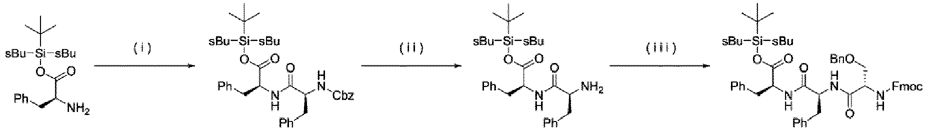

(i) Cbz-Phe-OH (0.47 g, 1.56 mmol) and 1-ethyl-3-(3-dimethylaminopropyl)carbodiimide hydrochloride (0.29 g, 1.53 mmol) were dissolved in methylene chloride (2.7 g), H-Phe-OSi(sBu)₂(tBu) (0.33 g, 0.92 mmol) was added thereto under ice-cooling and the mixture was stirred for 40 minutes. The obtained reaction mixture was washed with 4% by mass hydrochloric acid (3.0 g) and an aqueous saturated sodium hydrogen carbonate solution (3.0 g) in this order. The obtained organic layer was concentrated and purified by silica gel column chromatography to obtain Cbz-Phe-Phe-Si(sBu)₂(tBu) (0.589 g, Yield: 100%) as a colorless oil.
   MASS (ESI+) m/z; 645.47 (M+H)+
(ii) Cbz-Phe-Phe-OSi(sBu)₂(tBu) (0.58 g, 0.89 mmol) was dissolved in 2,2,2-trifluoroethanol (8.1 g), 10% by mass Pd-C (54.5 mg, 0.05 mmol) was added thereto and the mixture was stirred under a hydrogen gas atmosphere for 3 hours. The reaction mixture was filtered and the obtained filtrate was concentrated to obtain H-Phe-Phe-OSi(sBu)₂(tBu) (0.48 g, Yield: 100%) as a colorless oil.
   MASS (ESI+) m/z; 511.43 (M+H)+
(iii) H-Phe-Phe-OSi(sBu)₂(tBu) (0.46 g, 0.89 mmol) and Fmoc-Ser(Bn)-OH (0.56 g, 1.34 mmol) were dissolved in methylene chloride (6.0 g), 1-ethyl-3-(3-dimethylaminopropyl)carbodiimide hydrochloride (0.26 g, 1.37 mmol) was added thereto under ice-cooling and the mixture was stirred for 1 hour. The obtained reaction mixture was washed with 4% by mass hydrochloric acid (5.0 g) and an aqueous saturated sodium hydrogen carbonate solution (5.0 g) in this order. The obtained organic layer was concentrated and purified by silica gel column chromatography to obtain Fmoc-Ser(Bn)-Phe-Phe-OSi(sBu)₂(tBu) (0.76 g, Yield: 93%) as a pale yellow solid.
   MASS (ESI+) m/z; 910.59 (M+H)+

### Synthetic Example 25: Synthesis of Boc-Ala-Ser(Bn)-Phe-Phe-OSi(sBu)₂(tBu)

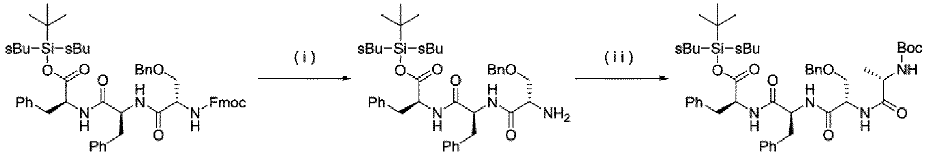

(i) Fmoc-Ser(Bn)-Phe-Phe-OSi(sBu)₂(tBu) (0.567 g, 0.623 mmol) was mixed with methylene chloride (7.6 g), diethylamine (0.92 g, 12.5 mmol) was added thereto at 0°C, and the mixture was stirred at 5°C for 18 hours. To the obtained reaction mixture was added 4% by mass hydrochloric acid (6.0 g) and the liquids were separated. After concentrating the organic layer, the concentrate was purified by silica gel chromatography to obtain H-Ser(Bn)-Phe-Phe-OSi(sBu)₂(tBu) (0.348 g, Yield: 81%) as a colorless oil.
   MASS (ESI+) m/z; 688.52 (M+H)+
(ii) H-Ser(Bn)-Phe-Phe-OSi(sBu)₂(tBu) (0.320 g, 0.465 mmol) and Boc-Ala-OH (0.132 g, 0.697 mmol) were dissolved in methylene chloride (4.3 g), 1-ethyl-3-(3-dimethylaminopropyl)carbodiimide hydrochloride (0.141 g, 0.738 mmol) was added thereto under ice-cooling and the mixture was stirred for 1 hour. To the mixed solution were added Boc-Ala-OH (0.019 g, 0.10 mmol) and 1-ethyl-3-(3-dimethylaminopropyl)carbodiimide hydrochloride (0.017 g, 0.088 mmol) and the mixture was stirred for 40 minutes. The obtained reaction mixture was washed with 4% by mass hydrochloric acid (3.0 g) and an aqueous saturated sodium hydrogen carbonate solution (3.0 g) in this order. The obtained organic layer was concentrated and purified by silica gel column chromatography to obtain Boc-Ala-Ser(Bn)-Phe-Phe-OSi(sBu)₂(tBu) (0.356 g, Yield: 89%) as a white solid.
   MASS (ESI+) m/z; 859.54 (M+H)+

### Synthetic Example 26: Synthesis of Fmoc-Pro-Ala-Ser(Bn)-Phe-Phe-OH

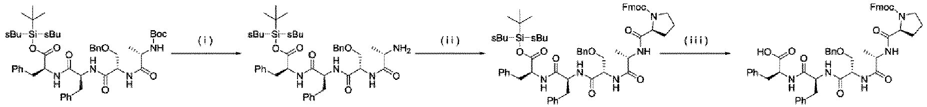

(i) Boc-Ala-Ser(Bn)-Phe-Phe-OSi(sBu)₂(tBu) (0.322 g, 0.375 mmol) was dissolved in methylene chloride (4.3 g), the mixture was cooled to 0°C, 15% by mass hydrogen chloride-1,4-dioxane (2.7 g, 10.4 mmol) was added thereto and the mixture was stirred at the same temperature for 20 hours. The obtained reaction mixture was washed with 5% by mass aqueous sodium chloride solution (4.0 g), 8% by mass aqueous sodium chloride solution (5.0 g), an aqueous saturated sodium hydrogen carbonate solution (3.0 g) and water (5.0 g) in this order. The obtained organic layer was concentrated to obtain H-Ala-Ser(Bn)-Phe-Phe-OSi(sBu)₂(tBu) (0.225 g, Yield: 79%) as a white solid.
   MASS (ESI+) m/z; 759.58 (M+H)+
(ii) H-Ala-Ser(Bn)-Phe-Phe-OSi(sBu)₂(tBu) (0.22 g, 0.29 mmol) and Fmoc-Pro-OH (0.20 g, 0.59 mmol) were dissolved in methylene chloride (2.9 g), 1-ethyl-3-(3-dimethylaminopropyl)carbodiimide hydrochloride (0.12 g, 0.60 mmol) was added thereto under ice-cooling and the mixture was stirred for 1 hour. The obtained reaction mixture was washed with 4% by mass hydrochloric acid (2.0 g) and an aqueous saturated sodium hydrogen carbonate solution (2.0 g) in this order. The obtained organic layer was concentrated and purified by silica gel column chromatography to obtain Fmoc-Pro-Ala-Ser(Bn)-Phe-Phe-OSi(sBu)₂(tBu) (0.32 g, Yield: 100%) as a white solid.
   MASS (ESI+) m/z; 1078.59 (M+H)+
(iii) Fmoc-Pro-Ala-Ser(Bn)-Phe-Phe-OSi(sBu)₂(tBu) (0.13 g, 0.12 mmol) was mixed with methanol (0.55 g) and tetrahydrofuran (1.8 g), potassium fluoride (0.01 g, 0.25 mmol) was added thereto at 0°C and the mixture was stirred for 3 hours. To the obtained reaction mixture were added 7% by mass aqueous sodium chloride solution (3.0 g), and methylene chloride (2.7 g) was added thereto and the liquids were separated. Methylene chloride (3.0 g) was added to the obtained aqueous layer to carry out extraction twice, and the organic layers were mixed. The obtained organic layer was concentrated and purified by silica gel column chromatography to obtain Fmoc-Pro-Ala-Ser(Bn)-Phe-Phe-OH (0.08 g, Yield: 72%) as a white solid.
   MASS (ESI+) m/z; 880.49 (M+H)+

### Reference Synthetic Example 2: Synthesis of Cbz-Phe-OSi(tBu)₂(Bn)

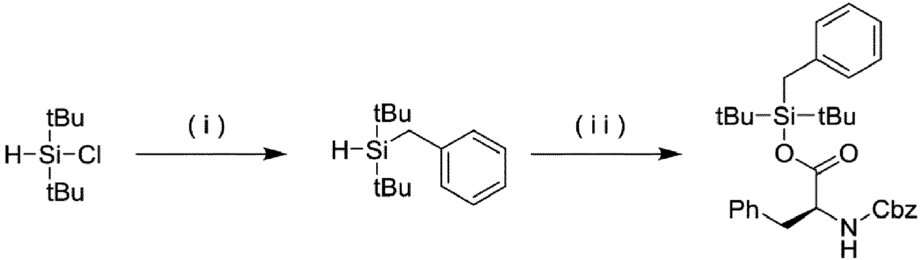

(i) Di-t-butylchlorosilane (0.10 g, 0.58 mmol) was mixed with tetrahydrofuran (0.9 g), the mixture was cooled to 0°C, 2.0M benzyl magnesium chloride-tetrahydrofuran solution (0.34mL, 0.68 mmol) was added thereto, and the mixture was stirred at room temperature for 3 hours. This solution was cooled to 0°C, benzyl magnesium chloride-tetrahydrofuran solution (0.17mL, 0.34 mmol) was added thereto and the mixture was stirred at room temperature for 30 minutes. To the obtained reaction mixture were added 10% by mass aqueous ammonium chloride solution (3.0 g) and toluene (4.0 g) and the liquids were separated. The obtained organic layer was concentrated and purified by silica gel column chromatography to obtain di-t-butylbenzylsilane (0.11 g, Yield: 83%) as a colorless liquid.
   ¹H-NMR (CDCl₃)
   δ ppm: 0.99 (18H, s), 2.22 (2H, d, J=3.5Hz), 3.56 (1H, t, J=3.5Hz), 7.03-7.28 (5H, m)
(ii) Di-t-butylbenzylsilane (0.07 g, 0.31 mmol) was dissolved in methylene chloride (1.1 g), trifluoromethanesulfonic acid (0.05 g, 0.32 mmol) was added thereto at 0°C and the mixture was stirred at room temperature for 1 hour. The obtained reaction mixture was added to a methylene chloride (0.5 g) solution of Cbz-Phe-OH (0.08 g, 0.27 mmol) and imidazole (0.03 g, 0.41 mmol) at 0°C, and after returning to room temperature, the mixture was stirred for 5 hours. The liquids of the obtained reaction mixture were separated using an aqueous saturated ammonium chloride solution (1.0 g) and water (1.0 g). The obtained organic layer was concentrated and purified by silica gel column chromatography to obtain Cbz-Phe-OSi(tBu)₂(Bn) (0.09 g, Yield: 66%) as a colorless liquid.
   MASS (ESI+) m/z; 532.11 (M+H)+

### Synthetic Example 27: Synthesis of Cbz-Phe-Phe-OSi(tBu)₂(Bn)

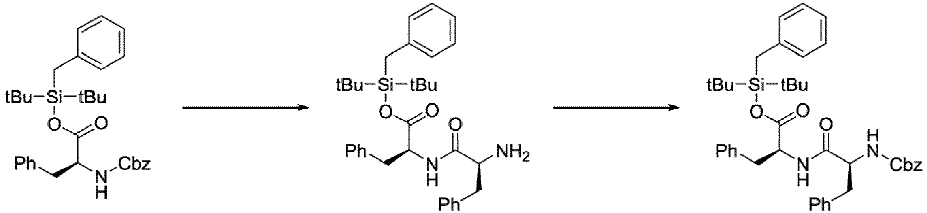

Cbz-Phe-OSi(tBu)₂(Bn) (0.15 g, 0.28 mmol) was dissolved in 2,2,2-trifluoroethanol (3.0 g) at 40°C, 10% by mass Pd-C (15 mg) was added thereto and the mixture was stirred under a hydrogen gas atmosphere for 2 hours. 10% by mass Pd-C (20 mg) was further added to the mixture, then, the mixture was stirred under a hydrogen gas atmosphere for 4 hours. The reaction mixture was filtered and the obtained filtrate was concentrated. The obtained concentrate was dissolved in methylene chloride (1.1 g), then, 1-ethyl-3-(3-dimethylaminopropyl)carbodiimide hydrochloride (0.11 g, 0.56 mmol) and Cbz-Phe-OH (0.17 g, 0.56 mmol) were added thereto under ice-cooling and the mixture was stirred for about 2 hours. The obtained reaction mixture was diluted with methylene chloride, and then, washed with 5% by mass hydrochloric acid, and further washed with water three times. The obtained organic layer was concentrated and purified by silica gel column chromatography to obtain Cbz-Phe-Phe-OSi(tBu)₂(Bn) (0.18 g, Yield: 96%) as a colorless oil.
MASS (ESI+) m/z; 679.48 (M+H)+

### Synthetic Example 28: Synthesis of Boc-Ala-Phe-Phe-OSi(tBu)₂(Bn)

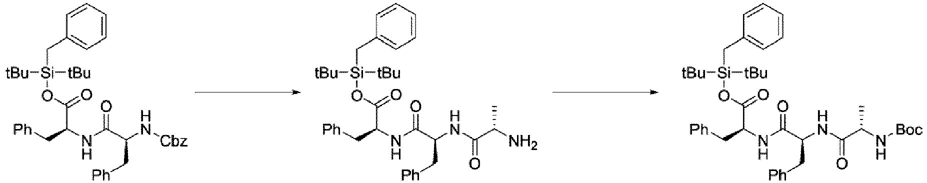

Cbz-Phe-Phe-OSi(tBu)₂(Bn) (0.17 g, 0.25 mmol) was dissolved in methylene chloride (3.4 g) and 2,2,2-trifluoroethanol (0.34 g) at 40°C, 10% by mass Pd-C (17 mg) was added thereto and the mixture was stirred under a hydrogen gas atmosphere for 2 hours. The reaction mixture was filtered and the obtained filtrate was concentrated. The obtained concentrate was dissolved in methylene chloride (1.4 g), 1-ethyl-3-(3-dimethylaminopropyl)carbodiimide hydrochloride (0.10 g, 0.50 mmol) and Cbz-Phe-OH (0.10 g, 0.50 mmol) were added thereto under ice-cooling and the mixture was stirred for 4 hours. The obtained reaction mixture was diluted with methylene chloride and then washed with water three times. The obtained organic layer was concentrated and the residue was purified by silica gel column chromatography to obtain Boc-Ala-Phe-Phe-OSi(tBu)₂(Bn) (0.18 g, Yield: 100%) as a colorless oil.
MASS (ESI+) m/z; 716.14 (M+H)+

### Synthetic Example 29: Synthesis of Fmoc-Cys(Trt)-Ala-Phe-Phe-OSi(tBu)₂(Bn)

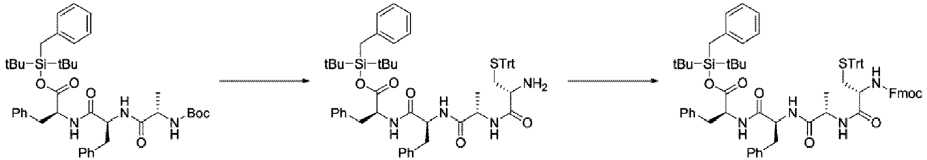

Boc-Ala-Phe-Phe-OSi(tBu)₂(Bn) (0.18 g, 0.25 mmol) was dissolved in methylene chloride (5.4 g) at room temperature, 15% by mass hydrogen chloride-1,4-dioxane (0.49 g, 2.0 mmol) was added thereto and the mixture was stirred for 25 hours. The obtained reaction mixture was diluted with methylene chloride, then, washed with 10% by mass aqueous potassium hydrogen carbonate solution, and further washed with water twice. The obtained organic layer was concentrated and dissolved in methylene chloride (1.54 g), under ice-cooling, 1-ethyl-3-(3-dimethylaminopropyl)carbodiimide hydrochloride (0.096 g, 0.50 mmol) and Fmoc-Cys(Trt)-OH (0.29 g, 0.50 mmol)were added thereto under ice-cooling and stirred for 3 hours. The obtained reaction mixture was concentrated and purified by silica gel column chromatography to obtain Fmoc-Cys(Trt)-Ala-Phe-Phe-OSi(tBu)₂(Bn) (0.27 g, Yield: 90%) as a white solid.
MASS (ESI+) m/z; 1183.54 (M+H)+

### Synthetic Example 30: Synthesis of Cbz-MeAla-Cys(Trt)-Ala-Phe-Phe-OSi(tBu)₂(Bn)

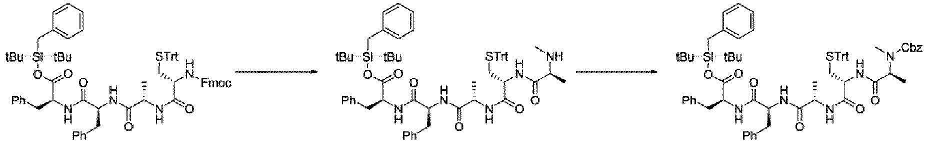

Fmoc-Cys(Trt)-Ala-Phe-Phe-OSi(tBu)₂(Bn) (0.15 g, 0.13 mmol) was dissolved in methylene chloride (4.5 g) at room temperature, diethylamine (0.46 g, 6.5 mmol) was added thereto and the mixture was stirred for 7 hours. The obtained reaction mixture was diluted with methylene chloride, and washed with 5% by mass aqueous sodium carbonate solution and further washed with water twice. The obtained organic layer was concentrated, and then, purified by silica gel column chromatography to obtain a white solid. The obtained solid was dissolved in methylene chloride (1.22 g), 1-ethyl-3-(3-dimethylaminopropyl)carbodiimide hydrochloride (0.05 g, 0.26 mmol) and Fmoc-Cys(Trt)-OH (0.06 g, 0.26 mmol) were added thereto under ice-cooling, and the mixture was stirred for 2 hours. The obtained reaction mixture was concentrated, and then, purified by silica gel column chromatography to obtain Cbz-MeAla-Cys(Trt)-Ala-Phe-Phe-OSi(tBu)₂(Bn) (0.11 g, Yield: 75%) as a white solid.
MASS (ESI+) m/z; 1180.94 (M+H)+

### Synthetic Example 31: Synthesis of Cbz-MeAla-Cys(Trt)-Ala-Phe-Phe-OH

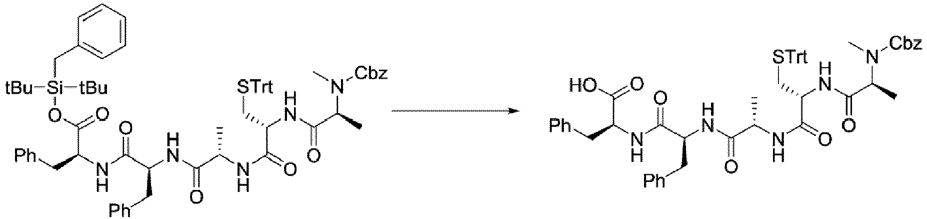

Cbz-MeAla-Cys(Trt)-Ala-Phe-Phe-OSi(tBu)₂(Bn) (0.10 g, 0.08 mmol) was dissolved in methanol (4.0 g) at room temperature, potassium fluoride (0.05 g, 0.8 mmol) was added thereto and the mixture was stirred for 2 hours. The obtained reaction mixture was diluted with ethyl acetate, silica gel was added and the mixture was stirred and filtered. The obtained silica gel was washed with methanol, and then, the washed solution was concentrated to obtain Cbz-MeAla-Cys(Trt)-Ala-Phe-Phe-OH (0.08 g, Yield: 100%) as a white solid.
MASS (ESI-) m/z; 946.49(M-H)-

### Reference Synthetic Example 3: Synthesis of di-t-butyloctadecylsilane

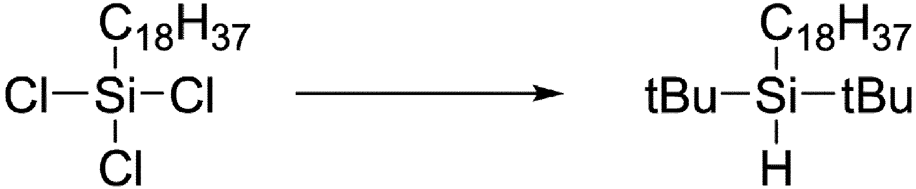

Octadecyltrichlorosilane (22.5 g, 58.0 mmol) was mixed with n-heptane (237mL), and 1.6M t-butyl lithium pentane solution (136mL, 21.7 mmol) was added dropwise thereto at room temperature. The obtained reaction mixture was refluxed at 100°C, and after the distilling mixed solution (123 g) of pentane and heptane was taken out, and the mixture was stirred at 100°C for 22 hours. The obtained reaction mixture was cooled to 0°C, isopropyl alcohol (1.9 g) was added thereto, diluted with hexane (50mL), and then, washed with water (50mL) and a saturated brine solution (25mL) in this order, the obtained organic layer was concentrated and purified by silica gel column chromatography to obtain di-t-butyloctadecylsilane (21.7 g, Yield: 91%) as a colorless liquid.
¹H-NMR (CDCl₃)
δ ppm: 0.57-0.64 (2H, m), 0.88 (3H, t, J=6.7Hz), 1.00 (18H, s), 1.23-1.29 (30H, m), 1.39-1.49 (2H, m), 3.29 (1H, t, J=2.6Hz)

### Synthetic Example 32: Synthesis of H-Phe-OSi(tBu)₂(C₁₈H₃₇)

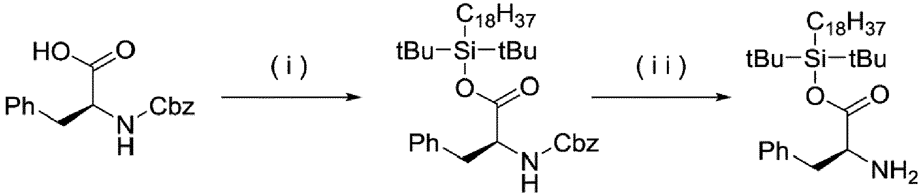

(i) di-t-butyloctadecylsilane (0.32 g, 8.02 mmol) was mixed with methylene chloride (2.0 g), the mixture was cooled to 0°C, trifluoromethanesulfonic acid (0.12 g, 8.02 mmol) was added thereto, and then, the mixture was stirred at room temperature for 1 hour. The obtained reaction mixture was cooled to 0°C, a methylene chloride (2.0 g) solution Cbz-Phe-OH (0.20 g, 6.68 mmol) and imidazole (0.07 g, 10.2 mmol) was added dropwise thereto and the mixture was stirred at room temperature for 20 hours. The obtained reaction mixture was diluted with chloroform, and then, washed with an aqueous saturated ammonium chloride solution (20.0 g) and water in this order. The obtained organic layer was concentrated and purified by silica gel column chromatography to obtain Cbz-Phe-OSi(tBu)₂(C₁₈H₃₇) (0.42 g, Yield: 89%) as a colorless liquid.
   MASS (ESI+) m/z; 694.52 (M+H)+
(ii) Cbz-Phe-OSi(tBu)₂(C₁₈H₃₇) (0.11 g, 1.58 mmol) was mixed with 2,2,2-trifluoroethanol (2.0 g) and methylene chloride (2.0 g), 10% by mass Pd-C (11.9 mg) was added thereto and the mixture was stirred under a hydrogen gas atmosphere at room temperature for 20 hours. After filtering the reaction mixture, the obtained filtrate was concentrated to obtain H-Phe-OSi(tBu)₂(C₁₈H₃₇) (0.08 g, Yield: 97%) as a colorless oil.
   MASS (ESI+) m/z; 560.48 (M+H)+

### Synthetic Example 33: Synthesis of Boc-Phe-Phe-Phe-OSi(tBu)₂(C₁₈H₃₇)

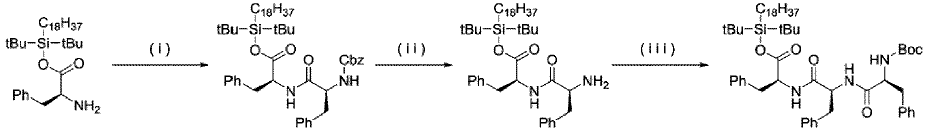

(i) H-Phe-OSi(tBu)₂(C₁₈H₃₇) (1.00 g, 1.79 mmol) was dissolved in methylene chloride (20.2 g), Cbz-Phe-OH (0.64 g, 2.15 mmol) and 1-ethyl-3-(3-dimethylaminopropyl)-carbodiimide hydrochloride (0.42 g, 2.15 mmol) were added thereto under ice-cooling and the mixture was stirred for 1 hour. The obtained reaction mixture was diluted with chloroform, and then, washed with an aqueous saturated ammonium chloride solution and an aqueous saturated sodium hydrogen carbonate solution in this order. The obtained organic layer was concentrated and purified by silica gel column chromatography to obtain Cbz-Phe-Phe-OSi(tBu)₂(C₁₈H₃₇) (1.47 g, Yield: 98%) as a white solid.
   MASS (ESI+) m/z; 841.59 (M+H)+
(ii) Cbz-Phe-Phe-OSi(tBu)₂(C₁₈H₃₇) (1.05 g, 1.25 mmol) was mixed with 2,2,2-trifluoroethanol (5.1 g) and methylene chloride (5.0 g), 10% by mass Pd-C (0.20 g) was added thereto and the mixture was stirred under a hydrogen gas atmosphere at room temperature for 27 hours. After filtering the reaction mixture, the obtained filtrate was concentrated. The concentrate was dissolved in chloroform, water was added thereto and the liquids were separated. The organic layer was concentrated to obtain H-Phe-Phe-OSi(tBu)₂(C₁₈H₃₇) (0.82 g, Yield: 94%) as a colorless oil.
   MASS (ESI+) m/z; 707.55 (M+H)+
(iii) H-Phe-Phe-OSi(tBu)₂(C₁₈H₃₇) (0.77 g, 1.09 mmol) and Boc-Phe-OH (0.58 g, 2.18 mmol) were dissolved in methylene chloride (16.0 g), 1-ethyl-3-(3-dimethylaminopropyl)carbodiimide hydrochloride (0.42 g, 2.18 mmol) was added thereto under ice-cooling and the mixture was stirred at room temperature for 15 hours. The obtained reaction mixture was diluted with chloroform, and then, washed with an aqueous saturated sodium hydrogen carbonate solution and an aqueous saturated ammonium chloride solution in this order. The obtained organic layer was dried over magnesium sulfate, and then, the solution obtained by filtration was concentrated. The obtained residue was purified by silica gel column chromatography to obtain Boc-Phe-Phe-Phe-OSi(tBu)₂(C₁₈H₃₇) (0.99 g, Yield: 91%) as a white solid.
   MASS (ESI+) m/z; 954.67 (M+H)+

### Synthetic Example 34: Synthesis of Fmoc-Phe-Phe-Phe-Phe-OSi(tBu)₂(C₁₈H₃₇)

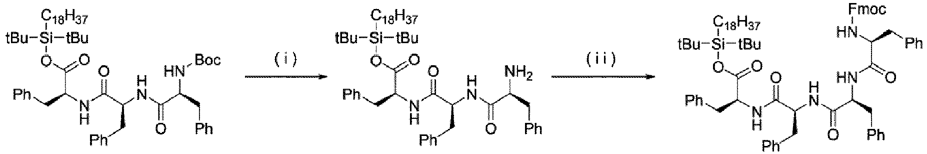

(i) Boc-Phe-Phe-Phe-OSi(tBu)₂(C₁₈H₃₇) (0.59 g, 0.62 mmol) was mixed with methylene chloride (12.0 g), the mixture was cooled to 0°C, 15% by mass hydrogen chloride-1,4-dioxane (3.1 g, 10.5 mmol) was added thereto and the mixture was stirred for 1 hour, and then, the mixture was returned to room temperature and stirred for 2 hours. The obtained reaction mixture was diluted with chloroform, and washed with an aqueous saturated sodium hydrogen carbonate solution and an aqueous saturated sodium chloride solution in this order. The obtained organic layer was concentrated to obtain H-Phe-Phe-Phe-Phe-OSi(tBu)₂(C₁₈H₃₇) (0.15 g, Yield: 96%) as a white solid.
   MASS (ESI+) m/z; 854.62 (M+H)+
(ii) H-Phe-Phe-Phe-OSi(tBu)₂(C₁₈H₃₇) (0.50 g, 0.59 mmol) and Fmoc-Phe-OH (0.27 g, 0.70 mmol) were mixed with methylene chloride (10.0 g), the mixture was cooled to 0°C, 1-ethyl-3-(3-dimethylaminopropyl)carbodiimide hydrochloride (0.14 g, 0.73 mmol) was added thereto and the mixture was stirred for 1 hour. After returning to room temperature and stirring for 1 hour, the obtained reaction mixture was diluted with chloroform, and then, washed with an aqueous saturated sodium hydrogen carbonate solution and an aqueous saturated ammonium chloride solution in this order. The obtained organic layer was concentrated and purified by silica gel column chromatography to obtain Fmoc-Phe-Phe-Phe-Phe-OSi(tBu)₂(C₁₈H₃₇) (0.67 g, Yield:
   94%) as a white solid.
   MASS (ESI+) m/z; 1223.76 (M+H)+

### Synthetic Example 35: Synthesis of Cbz-Phe-Phe-Phe-Phe-Phe-OSi(tBu)₂(C₁₈H₃₇)

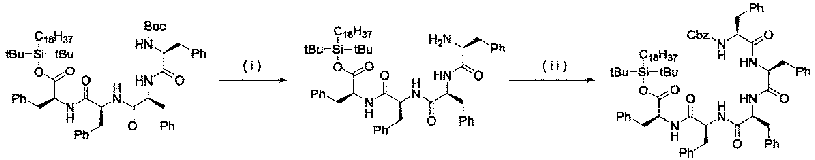

(i) Fmoc-Phe-Phe-Phe-Phe-OSi(tBu)₂(C₁₈H₃₇) (0.40 g, 0.33 mmol) was mixed with methylene chloride (8.0 g), the mixture was cooled to 0°C, and diethylamine (0.72 g, 9.8 mmol) was added thereto. After returning to room temperature and stirring for 5 hours, the obtained reaction mixture was diluted with chloroform and washed with an aqueous saturated ammonium chloride solution, and the liquids were separated. The obtained organic layer was concentrated to obtain H-Phe-Phe-Phe-Phe-OSi(tBu)₂(C₁₈H₃₇) (0.32 g, Yield: 96%) as a white solid.
   MASS (ESI+) m/z; 1001.69 (M+H)+
(ii) H-Phe-Phe-Phe-Phe-OSi(tBu)₂(C₁₈H₃₇) (0.25 g, 0.25 mmol) and Cbz-Phe-OH (0.09 g, 0.30 mmol) were mixed with methylene chloride (15.0 g), the mixture was cooled to 0°C, 1-ethyl-3-(3-dimethylaminopropyl)carbodiimide hydrochloride (0.06 g, 0.31 mmol) was added thereto and the mixture was stirred for 1 hour. After returning to room temperature and stirring for 1 hour, methanol (45.0 g) was added to the obtained reaction mixture to cause precipitation followed by filtration to obtain Cbz-Phe-Phe-Phe-Phe-Phe-OSi(tBu)₂(C₁₈H₃₇) (0.31 g, Yield: 95%) as a white solid.
   MASS (ESI+) m/z; 1282.79 (M+H)+

### Reference Synthetic Example 4: Synthesis of H-Si(tBu₂(OCH₂CH₂Ph)

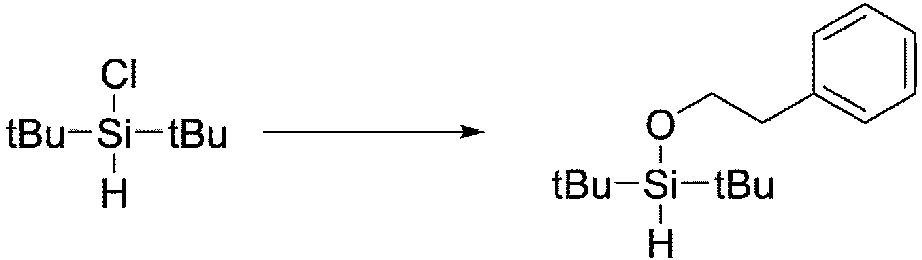

2-Phenylethyl alcohol (4.10 g, 33.6 mmol) was mixed with methylene chloride (10 g), triethylamine(5.66 g, 55.9 mmol), N,N-dimethyl-4-aminopyridine (0.69 g, 5.67 mmol) and di-tert-butylchlorosilane (5.01 g, 28.0 mmol) were added to the mixture at 0°C and the mixture was stirred at room temperature for 15 hours. To the obtained reaction mixture were added an aqueous saturated sodium hydrogen carbonate solution (20 g) at 0°C and the liquids were separated. The obtained organic layer was concentrated and purified by silica gel column chromatography to obtain dit-butyl(2-phenylethyloxy)silane (6.62 g, Yield: 89%) as a colorless liquid.
¹H-NMR (CDCl₃)
δ ppm: 0.97 (18H, s), 2.87 (2H, d, J=7.4Hz), 3.94 (2H, d, J=7.4Hz), 3.97 (1H, s), 7.16-7.31 (5H, m)

### Synthetic Example 36: Synthesis of H-Phe-OSi(tBu)₂(OCH₂CH₂Ph)

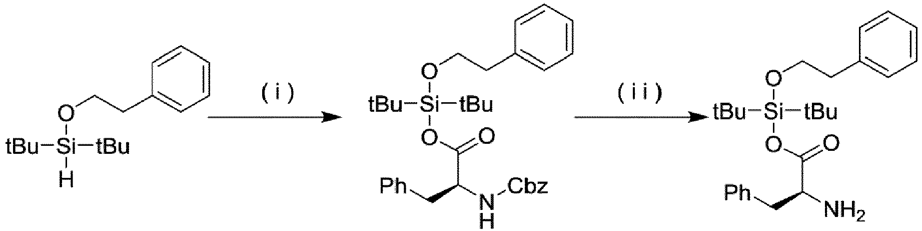

(i) Cbz-Phe-OH (4.25 g, 14.2 mmol) and di-t-butyl(2-phenylethyloxy)silane (2.52 g, 9.51 mmol) were mixed with N,N-dimethylformamide (25 g), zinc chloride (0.27 g, 2.0 mmol) was added thereto and the mixture was stirred at 60°C for 30 minutes. Zinc chloride (0.27 g, 2.0 mmol) was added to the reaction mixture and the mixture was stirred at 120°C for 20 hours. To the obtained reaction mixture were added 5% by mass aqueous sodium chloride solution (20 g) and ethyl acetate(32 g) at room temperature and the liquids were separated. The obtained organic layer was concentrated and purified by silica gel column chromatography to obtain Cbz-Phe-OSi(tBu)₂(OCH₂CH₂Ph) (1.53 g, Yield: 29%) as a colorless liquid.
   MASS (ESI+) m/z; 562.38 (M+H)+
(ii) Cbz-Phe-OSi(tBu)₂(OCH₂CH₂Ph) (0.81 g, 1.4 mmol) was dissolved in 2,2,2-trifluoroethanol (11 g), 10% by mass Pd-C (77 mg, 0.07 mmol) was added thereto and the mixture was stirred under a hydrogen gas atmosphere for 3 hours. After filtering the reaction mixture, the obtained filtrate was concentrated. The obtained concentrate was purified by silica gel column chromatography to obtain H-Phe-OSi(tBu)₂(OCH₂CH₂Ph) (0.437 g, Yield: 71%) as a colorless liquid.
   MASS (ESI+) m/z; 428.39 (M+H)+

### Synthetic Example 37: Synthesis of Boc-Asp(OBn)-Phe-Phe-OSi(tBu)₂(OCH₂CH₂Ph)

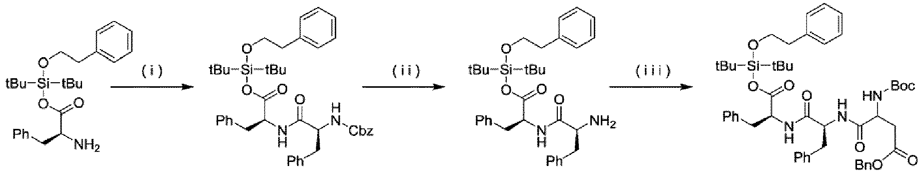

(i) Cbz-Phe-OH (0.58 g, 1.92 mmol) and 1-ethyl-3-(3-dimethylaminopropyl)carbodiimide hydrochloride (0.36 g, 1.90 mmol) were dissolved in methylene chloride (8.0 g), H-Phe-OSi(tBu)₂(OCH₂CH₂Ph) (0.41 g, 0.95 mmol) was added thereto under ice-cooling and the mixture was stirred for 2 hours. The obtained reaction mixture was washed with 4% by mass hydrochloric acid (4.0 g) and an aqueous saturated sodium hydrogen carbonate solution (4.0 g) in this order. The obtained organic layer was concentrated and purified by silica gel column chromatography to obtain Cbz-Phe-Phe-OSi(tBu)₂(OCH₂CH₂PH) (0.678 g, Yield: 94%) as a white solid.
   MASS (ESI+) m/z; 709.45 (M+H)+
(ii) Cbz-Phe-Phe-OSi(tBu)₂(OCH₂CH₂Ph) (0.62 g, 0.87 mmol) was dissolved in 2,2,2-trifluoroethanol (12.5 g), 10% by mass Pd-C (60 mg, 0.05 mmol) was added thereto and the mixture was stirred under a hydrogen gas atmosphere for 1.5 hours. The reaction mixture was filtered and the obtained filtrate was concentrated to obtain H-Phe-Phe-OSi(tBu)₂(OCH₂CH₂Ph) (0.50 g, Yield: 100%) as a brown oil.
   MASS (ESI+) m/z; 575.39 (M+H)+
(iii) H-Phe-Phe-OSi(tBu)₂(OCH₂CH₂Ph) (0.49 g, 0.85 mmol) and Boc-Asp(OBn)-OH (0.41 g, 1.28 mmol) were dissolved in methylene chloride (9.3 g), 1-ethyl-3-(3-dimethylaminopropyl)carbodiimide hydrochloride (0.25 g, 1.28 mmol) was added thereto under ice-cooling and the mixture was stirred for 1 hour. The obtained reaction mixture was washed with 4% by mass hydrochloric acid (5.0 g) and an aqueous saturated sodium hydrogen carbonate solution (5.0 g) in this order, and the obtained organic layer was concentrated and purified by silica gel column chromatography to obtain Boc-Asp(OBn)-Phe-Phe-OSi(tBu)₂(OCH₂CH₂Ph) (0.63 g, Yield: 84%) as a white solid.
   MASS (ESI+) m/z; 880.53 (M+H)+

### Synthetic Example 38: Synthesis of Fmoc-Gly-Asp(OBn)-Phe-Phe-OSi(tBu)₂(OCH₂CH₂Ph)

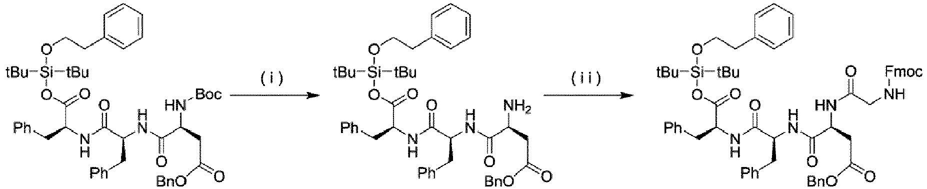

(i) Boc-Asp(OBn)-Phe-Phe-OSi(tBu)₂(OCH₂CH₂Ph) (0.30 g, 0.34 mmol) was dissolved in methylene chloride (8.0 g), the mixture was cooled to 0°C, 15% by mass hydrogen chloride-1,4-dioxane (2.5 g, 9.6 mmol) was added thereto and the mixture was stirred for 24 hours. The obtained reaction mixture was diluted with water (3.0 g), and then, washed with an aqueous saturated sodium hydrogen carbonate solution (3.0 g) and an aqueous saturated sodium chloride solution (3.0 g) in this order. The obtained organic layer was concentrated to obtain H-Asp(OBn)-Phe-Phe-OSi(tBu)₂(OCH₂CH₂Ph) (0.26 g, Yield: 99%) as a pale yellow liquid.
   MASS (ESI+) m/z; 780.54 (M+H)+
(ii) H-Asp(OBn)-Phe-Phe-OSi(tBu)₂(OCH₂CH₂Ph) (0.25 g, 0.32 mmol) and Fmoc-Gly-OH (0.14 g, 0.48 mmol) were dissolved in methylene chloride (4.7 g), 1-ethyl-3-(3-dimethylaminopropyl)carbodiimide hydrochloride (0.09 g, 0.49 mmol) was added thereto under ice-cooling and the mixture was stirred for 1 hour. The obtained reaction mixture was washed with 4% by mass hydrochloric acid (2.5 g) and an aqueous saturated sodium hydrogen carbonate solution (2.5 g) in this order, and the obtained organic layer was concentrated and purified by silica gel column chromatography to obtain Fmoc-Gly-Asp(OBn)-Phe-Phe-OSi(tBu)₂(OCH₂CH₂Ph) (0.30 g, Yield: 90%) as a white solid.
   MASS (ESI+) m/z; 1059.70 (M+H)+

### Synthetic Example 39: Synthesis of Fmoc-Lys(Cbz)-Gly-Asp(OBn)-Phe-Phe-OH

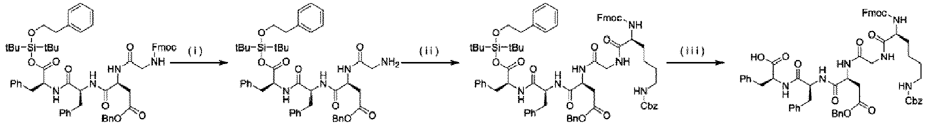

(i) Fmoc-Gly-Asp(OBn)-Phe-Phe-OSi(tBu)₂(OCH₂CH₂Ph) (0.14 g, 0.14 mmol) was mixed with methylene chloride (2.8 g), diethylamine (0.21 g, 2.83 mmol) was added thereto at 0°C and the mixture was stirred at 0°C for 3 hours, and further, the mixture was returned to room temperature and stirred for 5 hours. To the obtained reaction mixture was added a saturated aqueous ammonium chloride solution (1.5 g), and the liquids were separated. The obtained organic layer was concentrated and purified by silica gel column chromatography to obtain H-Gly-Asp(OBn)-Phe-Phe-OSi(tBu)₂(OCH₂CH₂Ph) (0.06 g, Yield: 49%) as a white solid.
   MASS (ESI+) m/z; 837.46 (M+H)+
(ii) H-Gly-Asp(OBn)-Phe-Phe-OSi(tBu)₂(OCH₂CH₂Ph) (0.05 g, 0.06 mmol), Fmoc-Lys(Cbz)-OH (0.05 g, 0.09 mmol) were dissolved in methylene chloride (1.3 g), 1-ethyl-3-(3-dimethylaminopropyl)carbodiimide hydrochloride (0.02 g, 0.11 mmol) was added thereto under ice-cooling and the mixture was stirred for 1.5 hours. The obtained reaction mixture was washed with 4% by mass hydrochloric acid (1.0 g) and an aqueous saturated sodium hydrogen carbonate solution (1.0 g) in this order, and the obtained organic layer was concentrated and purified by silica gel column chromatography to obtain Fmoc-Lys(Cbz)-Gly-Asp(OBn)-Phe-Phe-OSi(tBu)₂(OCH₂CH₂Ph) (0.041 g, Yield: 49%) as a white solid.
(iii) Fmoc-Lys(Cbz)-Gly-Asp(OBn)-Phe-Phe-OSi(tBu)₂(OCH₂CH₂Ph) (0.04 g, 0.03 mmol) was mixed with methanol (0.20 g) and tetrahydrofuran (0.67 g), potassium fluoride (9.4 mg, 0.16 mmol) was added thereto at 0°C and the mixture was stirred for 3 hours. To the obtained reaction mixture were added water (2.0 g) and methylene chloride (2.0 g) and the liquids were separated. Methylene chloride (3.0 g) was added to the obtained aqueous layer to carry out extraction three times, and the organic layers were mixed. The obtained organic layer was concentrated and purified by silica gel column chromatography to obtain Fmoc-Lys(Cbz)-Gly-Asp(OBn)-Phe-Phe-OH (0.024 g, Yield: 69%) as a white solid.
   MASS (ESI+) m/z; 1059.55 (M+H)+

### Synthetic Example 40: Synthesis of H-Phe-OSi(OtBu)₃

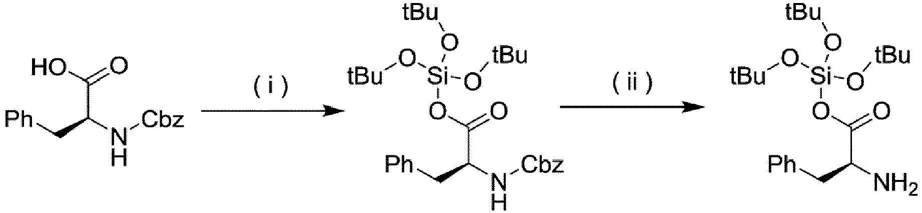

(i) Cbz-Phe-OH (0.30 g, 1.00 mmol), tert-butanol (0.48 g) and pyridine (0.39 g, 4.9 mmol) were mixed, tetrachlorosilane (0.17 g, 1.0 mmol) was added thereto at 0°C and the mixture was stirred for 2.5 hours. Pyridine (0.20 g, 2.5 mmol) and tetrachlorosilane (0.081 g, 0.48 mmol) were added thereto and the mixture was stirred for 15 hours. The obtained reaction mixture was diluted with chloroform, washed with 10% by mass aqueous ammonium chloride solution, water and 5% by mass aqueous sodium hydrogen carbonate solution in this order, and the organic layer was concentrated. The obtained residue was purified by silica gel column chromatography to obtain Cbz-Phe-OSi(OtBu)₃ (0.27 g, Yield: 49%) as a colorless oil.
   MASS (ESI+) m/z; 546.2 (M+H)+
(ii) Cbz-Phe-OSi(OtBu)₃ (0.22 g, 0.40 mmol) was dissolved in 2,2,2-trifluoroethanol (2.2 g), 10% by mass Pd-C (66 mg) was added thereto and the mixture was stirred under a hydrogen gas atmosphere at room temperature for 2 hours, and at 35°C for 3 hours. The reaction mixture was filtered and the obtained filtrate was concentrated to obtain H-Phe-OSi(OtBu)₃ (0.15 g, Yield: 90%) as a white liquid.
   MASS (ESI+) m/z; 412.4 (M+H)+

### Synthetic Example 41: Synthesis of Fmoc-Phe-Phe-Phe-OSi(OtBu)₃

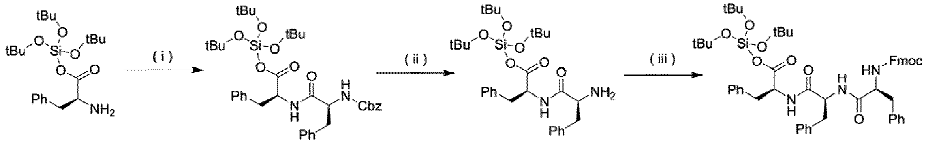

(i) H-Phe-OSi(OtBu)₃ (70 mg, 0.17 mmol) and Cbz-Phe-OH (76 mg, 0.25 mmol) were dissolved in methylene chloride (1.4 g), N,N'-diisopropylcarbodiimide (32 mg, 0.25 mmol) was added thereto under ice-cooling and the mixture was stirred for 2 hours. The obtained reaction mixture was diluted with chloroform (3 mL), washed with 10% by mass aqueous ammonium chloride solution and 5% by mass aqueous sodium hydrogen carbonate solution, the liquids were separated, and the organic layer was concentrated. The obtained residue was purified by silica gel column chromatography to obtain Cbz-Phe-Phe-OSi(OtBu)₃ (91 mg, Yield: 77%) as a white solid.
   MASS (ESI+) m/z; 693.5 (M+H)+
(ii) Cbz-Phe-Phe-OSi(OtBu)₃ (0.090 g, 0.13 mmol) was dissolved in 2,2,2-trifluoroethanol (1.8 g), 10% by mass Pd-C (27 mg) was added thereto and the mixture was stirred under a hydrogen gas atmosphere at room temperature for 4.5 hours. Further, 10% by mass Pd-C (10 mg) was added thereto and the mixture was stirred for 2 hours. The reaction mixture was filtered and the obtained filtrate was concentrated to obtain H-Phe-Phe-OSi(OtBu)₃ (73 mg, Yield: 100%) as a colorless oil.
   MASS (ESI+) m/z; 559.4 (M+H)+
(iii) H-Phe-Phe-OSi(OtBu)₃ (73 mg, 0.13 mmol) and Fmoc-Phe-OH (76 mg, 0.20 mmol) were dissolved in methylene chloride (1.5 g), 1-ethyl-3-(3-dimethylaminopropyl)carbodiimide (30 mg, 0.19 mmol) was added thereto under ice-cooling, and the mixture was stirred for 30 minutes. The obtained reaction mixture was diluted with chloroform (3 mL), washed with 10% by mass aqueous ammonium chloride solution (1 mL), water (1 mL) and 5% by mass aqueous sodium hydrogen carbonate solution (1 mL) in this order, and the organic layer was concentrated. The obtained residue was purified by silica gel column chromatography to obtain Fmoc-Phe-Phe-Phe-OSi(OtBu)₃ (75 mg, Yield: 81%) as a colorless oil.
   MASS (ESI+) m/z; 928.5 (M+H)+

### Synthetic Example 42: Synthesis of Cbz-Lys(Boc)-Phe-Phe-Phe-OH

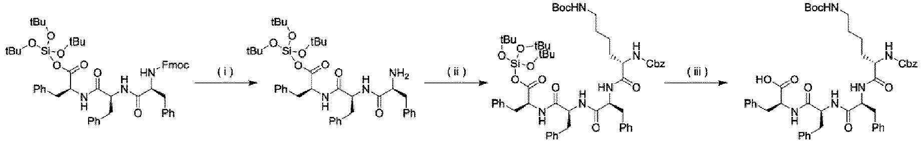

(i) To Fmoc-Phe-Phe-Phe-OSi(OtBu)₃ (63 mg, 0.068 mmol) were added methylene chloride (1.4 g) and diethylamine (0.11 g, 1.50 mmol), and the mixture was stirred at room temperature for 7 hours. The obtained reaction mixture was diluted with chloroform and washed with 10% by mass aqueous ammonium chloride solution, and the liquids were separated. The organic layer was concentrated and the obtained residue was purified by silica gel column chromatography to obtain H-Phe-Phe-Phe-OSi(OtBu)₃ (43 mg, Yield: 90%) as a pale yellow product.
   MASS (ESI+) m/z; 706.6 (M+H)+
(ii) H-Phe-Phe-Phe-OSi(OtBu)₃ (40 mg, 0.057 mmol) and Cbz-Lys(Boc)-OH (32 mg, 0.084 mmol) were dissolved in methylene chloride (1.2 g), 1-ethyl-3-(3-dimethylaminopropyl)carbodiimide (13 mg, 0.085 mmol) was added thereto under ice-cooling and the mixture was stirred for 1.5 hours. The obtained reaction mixture was diluted with chloroform and diluted with 10% by mass aqueous ammonium chloride solution and the liquids were separated. The organic layer was concentrated and the obtained residue was purified by silica gel column chromatography to obtain Cbz-Lys(Boc)-Phe-Phe-Phe-OSi(OtBu)₃ (55 mg, Yield: 90%) as a colorless oil.
   MASS (ESI+) m/z; 1068.7 (M+H)+
(iii) Cbz-Lys(Boc)-Phe-Phe-Phe-OSi(OtBu)₃ (50 mg, 0.047 mmol) was mixed with methanol (0.35 g) and tetrahydrofuran (1.1 g), potassium fluoride (4.1 mg, 0.071 mmol) was added thereto under ice-cooling and the mixture was stirred for 3 hours. After raising the temperature to room temperature followed by stirring for 3.5 hours, the obtained reaction mixture was concentrated. The mixture was diluted with chloroform (3 mL), 10% by mass aqueous ammonium chloride solution (1 mL) was added thereto and the liquids were separated, and chloroform was added to the aqueous layer to carry out extraction. The organic layers were combined, concentrated and purified by silica gel column chromatography to obtain Cbz-Lys(Boc)-Phe-Phe-Phe-OH (29 mg, Yield: 76%) as a white solid.
   MASS (ESI+) m/z; 822.4 (M+H)+

### Reference Synthetic Example 5: Synthesis of di-t-butylphenylsilane

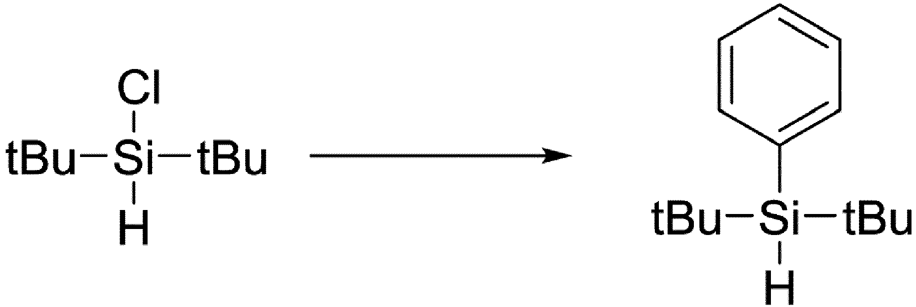

Di-tert-butylchlorosilane (1.0 g, 5.6 mmol) was mixed with tetrahydrofuran (5.0 g), 1.6M phenyl lithium butyl ether solution (4.3 mL, 6.9 mmol) was added dropwise thereto at 0°C, and the mixture was stirred at 40°C for 4 hours. To the obtained reaction mixture were added water (7.0 g) and hexane, and the liquids were separated and the aqueous layer was again extracted with hexane. The obtained organic layer was concentrated and purified by silica gel column chromatography to obtain di-t-butylphenylsilane (1.15 g, Yield: 94%) as a colorless liquid.
¹H-NMR (CDCl₃)
δ ppm: 1.04-1.06 (18H, br), 3.86 (1H, s), 7.29-7.39 (3H, m), 7.55-7.59 (2H, m)

### Synthetic Example 43: Synthesis of Cbz-Phe-OSi(tBu)₂(Ph)

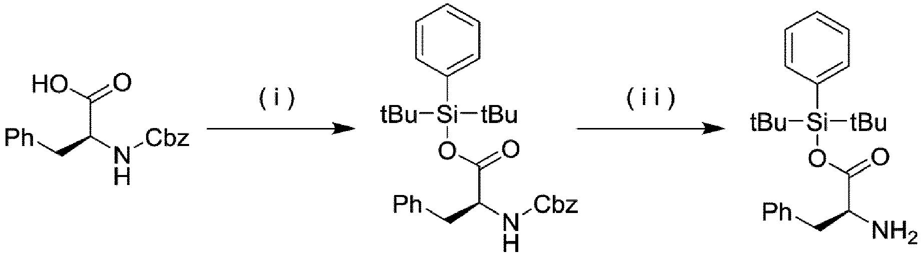

(i) Cbz-Phe-OH (0.54 g, 1.81 mmol) and di-t-butylphenylsilane (0.48 g, 2.18 mmol) were mixed with tetrahydrofuran (5.43 g), palladium acetate (122 mg, 0.54 mmol) was added thereto at room temperature and the mixture was stirred for 65 hours. The obtained reaction mixture was concentrated and purified by silica gel column chromatography to obtain Cbz-Phe-OSi(tBu)₂(Ph) (0.17 g, Yield: 18%) as a colorless liquid.
   MASS (ESI+) m/z; 518.3 (M+H)+
(ii) Cbz-Phe-OSi(tBu)₂(Ph) (98 mg, 0.19 mmol) was dissolved in 2,2,2-trifluoroethanol (1.2 g), 10% by mass Pd-C (8.2 mg) was added thereto and the mixture was stirred under a hydrogen gas atmosphere at 30°C for 2 hours. The reaction mixture was filtered and the obtained filtrate was concentrated. The residue was purified by silica gel column chromatography to obtain H-Phe-OSi(tBu)₂(Ph) (72 mg, Yield: 100%) as a colorless liquid.
   MASS (ESI+) m/z; 384.3 (M+H)+

### Synthetic Example 44: Synthesis of Boc-Phe-Phe-Phe-OSi(tBu)₂(Ph)

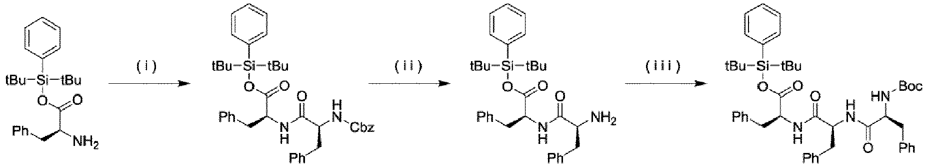

(i) H-Phe-OSi(tBu)₂(Ph) (70 mg, 0.18 mmol) and Cbz-Phe-OH (82 mg, 0.27 mmol) were dissolved in methylene chloride (1.5 g), 1-ethyl-3-(3-dimethylaminopropyl)-carbodiimide hydrochloride (53 mg, 0.84 mmol) was added thereto under ice-cooling and the mixture was stirred for 1 hour. The obtained reaction mixture was diluted with chloroform (2 mL), water (2 mL) and 5% by mass aqueous sodium hydrogen carbonate solution (1 mL) were added thereto and the liquids were separated. The aqueous layer was again extracted with chloroform (1 mL), and the organic layer was concentrated. The obtained residue was purified by silica gel column chromatography to obtain Cbz-Phe-Phe-OSi(tBu)₂(Ph) (0.11 g, Yield: 93%) as a colorless oil.
   MASS (ESI+) m/z; 665.5 (M+H)+
(ii) Cbz-Phe-Phe-OSi(tBu)₂(Ph) (0.11 g, 0.17 mmol) was dissolved in 2,2,2-trifluoroethanol (1.1 g), 10% by mass Pd-C (11 mg) was added thereto and the mixture was stirred under a hydrogen gas atmosphere at 35°C for 3 hours. 10% by mass Pd-C (22 mg) was added thereto and the mixture was stirred at 38°C for 2 hours. 10% by mass Pd-C (22 mg) was added thereto and stirred at room temperature for 18 hours, then, the reaction mixture was filtered and the obtained filtrate was concentrated. The residue was purified by silica gel column chromatography to obtain H-Phe-Phe-OSi(tBu)₂(Ph) (88 mg, Yield: 100%) as a colorless oil.
   MASS (ESI+) m/z; 531.4 (M+H)+
(iii) H-Phe-Phe-OSi(tBu)₂(Ph) (88 mg, 0.17 mmol) and Boc-Phe-OH (66 mg, 0.25 mmol) were dissolved in methylene chloride (1.8 g), 1-ethyl-3-(3-dimethylaminopropyl)carbodiimide hydrochloride (48 mg, 0.25 mmol) was added thereto under ice-cooling and the mixture was stirred at room temperature for 2 hours. Boc-Phe-OH (22 mg, 0.083 mmol) and 1-ethyl-3-(3-dimethylaminopropyl)carbodiimide hydrochloride (16 mg, 0.08 mmol) were added thereto, and the mixture was stirred at room temperature for 1.5 hours. The obtained reaction mixture was diluted with chloroform (2 mL), washed with 5% by mass aqueous sodium hydrogen carbonate solution (1 mL) and water (1 mL) in this order, and the organic layer was concentrated. The obtained residue was purified by silica gel column chromatography to obtain Boc-Phe-Phe-Phe-OSi(tBu)₂(Ph) (0.13 g, Yield: 100%) as a white solid.
   MASS (ESI+) m/z; 778.4 (M+H)+

### Synthetic Example 45: Synthesis of Fmoc-Gly-Phe-Phe-Phe-OSi(tBu)₂(Ph)

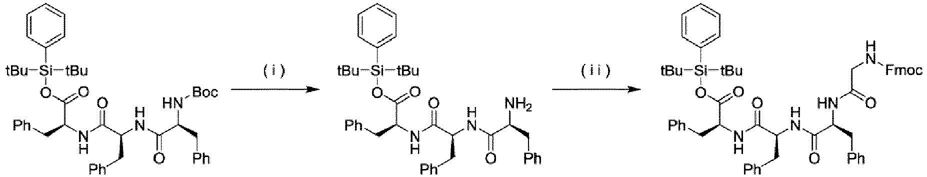

(i) Boc-Phe-Phe-Phe-OSi(tBu)₂(Ph) (0.13 g, 0.17 mmol) was mixed with methylene chloride (2.6 g), the mixture was cooled to 0°C, 15% by mass hydrogen chloride-1,4-dioxane (1.29 g) was added thereto and the mixture was stirred at room temperature for 2 hours. To the obtained reaction mixture was added 5% by mass aqueous sodium hydrogen carbonate solution, the mixture was neutralized and the liquids were separated, and the organic layer was further washed with water (1.0 g). The obtained organic layer was concentrated to obtain H-Phe-Phe-Phe-OSi(tBu)₂(Ph) (0.11 g, Yield: 99%) as a white solid.
   MASS (ESI+) m/z; 678.5 (M+H)+
(ii) H-Phe-Phe-Phe-OSi(tBu)₂(Ph) (0.11 g, 0.17 mmol) and Fmoc-Gly-OH (74 mg, 0.25 mmol) were dissolved in methylene chloride (2.2 g), 1-ethyl-3-(3-dimethylaminopropyl)carbodiimide hydrochloride (48 mg, 0.25 mmol) was added thereto under ice-cooling and the mixture was stirred for 0.5 hour. The obtained reaction mixture was diluted with chloroform (2 mL) and washed with 5% by mass aqueous sodium hydrogen carbonate solution (2 mL) and water (1 mL) in this order, and the organic layer was concentrated. The obtained residue was purified by silica gel column chromatography to obtain Fmoc-Gly-Phe-Phe-Phe-OSi(tBu)₂(Ph) (0.11 g, Yield: 71%) as a white solid.
   MASS (ESI+) m/z; 957.6 (M+H)+

### Reference Synthetic Example 6: Synthesis of IPBS-OTf

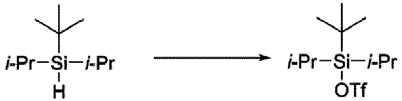

Di-i-propyl-t-butylsilane (2.59 g, 15.0 mmol) was dissolved in methylene chloride (10.0 g), trifluoromethanesulfonic acid (2.26 g, 15.0 mmol) was added dropwise thereto under ice-cooling and the mixture was stirred for 30 minutes. The formed di-i-propyl-t-butylsilyltriflate (4.82 g, 15.0 mmol) was used in the next reaction as a methylene chloride solution without isolation.

### Synthetic Example 46: Synthesis of Cbz-Phe-OIPBS

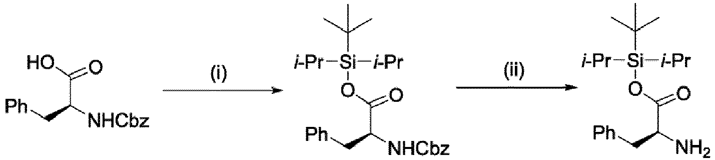

(i) Cbz-Phe-OH (3.00 g, 10.0 mmol) and di-i-propyl-t-butylsilyltriflate (4.82 g, 15.0 mmol) were mixed with methylene chloride (49.9 g), the mixture was cooled to 0°C, N,N-diisopropylethylamine (2.59 g, 20.1 mmol) was added dropwise thereto, and the mixture was stirred at room temperature for 2 hours. The obtained reaction mixture was washed with an aqueous saturated ammonium chloride solution (35.9 g), then, washed with an aqueous saturated sodium hydrogen carbonate solution (40.0 g), and the obtained organic layer was concentrated to obtain Cbz-Phe-OIPBS (5.43 g) as a crude product.
   MASS (ESI+) m/z; 471.2 (M+H)+
(ii) Cbz-Phe-OIPBS (5.43 g) was mixed with 2,2,2-trifluoroethanol (34.8 g), 10% by mass Pd-C (0.53 g, 0.50 mmol) and triethylsilane (4.66 g, 40.1 mmol) were added thereto and the mixture was stirred at room temperature for 2 hours. The reaction mixture was filtered, the obtained filtrate was concentrated, and then, the concentrate was purified by silica gel column chromatography to obtain H-Phe-OIPBS (3.26 g, 2-Step Yield: 97%) as a colorless liquid.
   MASS (ESI+) m/z; 336.4 (M+H)+

### Synthetic Example 47: Synthesis of Fmoc-Phe-Phe-Phe-OIPBS

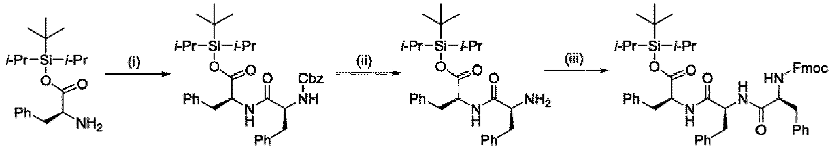

(i) H-Phe-OIPBS (0.50 g, 1.49 mmol) was dissolved in methylene chloride (9.9 g), Cbz-Phe-OH (0.67 g, 2.24 mmol) and 1-ethyl-3-(3-dimethylaminopropyl) carbodiimide hydrochloride (0.43 g, 2.24 mmol) were added thereto under ice-cooling and the mixture was stirred for 20 minutes. After returning to room temperature and stirring for further 1 hour, methylene chloride (20.1 g) and water (20.0 g) were added thereto and the liquids were separated. The organic layer was washed with an aqueous saturated sodium hydrogen carbonate solution (24.5 g) and an aqueous saturated sodium chloride solution (14.9 g) in this order, and the obtained organic layer was concentrated and purified by silica gel column chromatography to obtain Cbz-Phe-Phe-OIPBS (0.88 g, Yield: 95%) as a colorless liquid.
   MASS (ESI+) m/z; 617.6 (M+H)+
(ii) Cbz-Phe-Phe-OIPBS (0.87 g, 1.41 mmol) was mixed with 2,2,2-trifluoroethanol (9.8 g), 10% by mass Pd-C (0.075 g, 0.071 mmol) and triethylsilane (0.66 g, 5.64 mmol) were added thereto and the mixture was stirred at room temperature for 3 hours. The reaction mixture was filtered, and the obtained filtrate was concentrated to obtain H-Phe-Phe-OIPBS (1.14 g) as a crude product.
   MASS (ESI+) m/z; 483.5 (M+H)+
(iii) H-Phe-Phe-OIPBS (1.14 g) was dissolved in methylene chloride (9.4 g), Fmoc-Phe-OH (0.71 g, 1.83 mmol) and 1-ethyl-3-(3-dimethylaminopropyl)carbodiimide hydrochloride (0.35 g, 1.83 mmol) were added thereto under ice-cooling and the mixture was stirred for 20 minutes. After returning to room temperature and stirring for further 1 hour, methylene chloride (27.3 g) and water (25.1 g) were added thereto and the liquids were separated. The organic layer was washed with an aqueous saturated sodium hydrogen carbonate solution (29.0 g) and an aqueous saturated sodium chloride solution (20.0 g) in this order, and the obtained organic layer was concentrated and purified by silica gel column chromatography to obtain Fmoc-Phe-Phe-Phe-OIPBS (1.05 g, 2-Step Yield: 87%) as a white solid.
   MASS (ESI+) m/z; 852.7 (M+H)+

### Synthetic Example 48: Synthesis of Boc-Phe-Phe-Phe-Phe-OIPBS

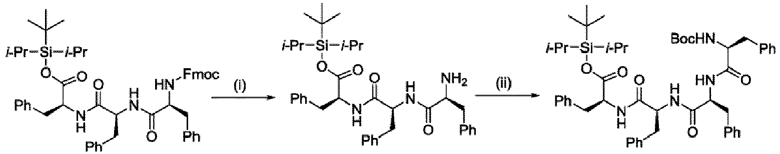

(i) Fmoc-Phe-Phe-Phe-OIPBS (0.74 g, 0.87 mmol) was mixed with methylene chloride (7.7 g) at room temperature, diethylamine (0.64 g, 8.70 mmol) was added thereto and the mixture was stirred for 2 hours. After adding 8% by mass aqueous hydrogen chloride solution (3.0 g) to the reaction mixture, it was diluted with methylene chloride (27.4 g) and the liquids were separated. The organic layer was washed with an aqueous saturated sodium chloride solution (13.5 g), and the obtained organic layer was concentrated and purified by silica gel column chromatography to obtain H-Phe-Phe-Phe-OIPBS (0.51 g, Yield: 94%) as a white solid.
   MASS (ESI+) m/z; 630.5 (M+H)+
(ii) H-Phe-Phe-Phe-OIPBS (0.51 g, 0.82 mmol) was dissolved in methylene chloride (5.4 g), Boc-Phe-OH (0.24 g, 0.90 mmol) and 1-ethyl-3-(3-dimethylaminopropyl)-carbodiimide hydrochloride (0.17 g, 0.90 mmol) were added thereto under ice-cooling and the mixture was stirred for 20 minutes. After returning to room temperature and stirring for further 1 hour, methylene chloride (26.6 g) and water (11.2 g) were added thereto and the liquids were separated. The organic layer was washed with an aqueous saturated sodium hydrogen carbonate solution (19.6 g), an aqueous saturated sodium chloride solution (12.2 g) in this order, the obtained organic layer was concentrated and purified by silica gel column chromatography to obtain Boc-Phe-Phe-Phe-Phe-OIPBS (0.64 g, Yield: 89%) as a white solid.
   MASS (ESI+) m/z; 877.8 (M+H)+

### Synthetic Example 49: Synthesis of Cbz-Phe-Phe-Phe-Phe-Phe-OIPBS

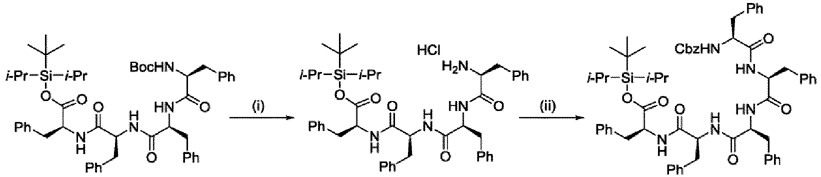

(i) Boc-Phe-Phe-Phe-Phe-OIPBS (0.64 g, 0.73 mmol) was dissolved in methylene chloride (4.8 g), 15% by mass hydrogen chloride-1,4-dioxane (5.46 g, 21.8 mmol) was added thereto under ice-cooling and the mixture was stirred for 20 minutes. After returning to room temperature and stirring for further 1 hour, the obtained reaction mixture was concentrated and subjected to azeotropic distillation with toluene (9.8 g) twice to obtain H-Phe-Phe-Phe-Phe-OIPBS hydrochloride (0.60 g) as a crude product.
   MASS (ESI+) m/z; 778.6 (M+H-HCl)+
(ii) H-Phe-Phe-Phe-Phe-OIPBS hydrochloride (0.10 g) was dissolved in methylene chloride (1.6 g), Cbz-Phe-OH (0.041 g, 0.14 mmol), O-(7-azabenzotriazol-1-yl)-N,N,N',N'-tetramethyluronium hexafluorophosphate (0.051 g, 0.14 mmol) and N,N-diisopropylethylamine (0.024 g, 0.18 mmol) were added thereto under ice-cooling and the mixture was stirred for 30 minutes. After returning to room temperature and stirring for further 1 hour, methylene chloride (13.3 g) and 10% by mass aqueous citric acid solution (5.4 g) were added thereto and the liquids were separated. The organic layer was washed with an aqueous saturated sodium hydrogen carbonate solution (11.6 g) and an aqueous saturated sodium chloride solution (7.3 g) in this order, and the obtained organic layer was concentrated and purified by silica gel column chromatography to obtain Cbz-Phe-Phe-Phe-Phe-Phe-OIPBS (0.11 g, 2-Step Yield: 86%) as a white solid.
   MASS (ESI+) m/z; 1060.0 (M+H)+

### Reference Synthetic Example 7: Synthesis of IPCS-OTf

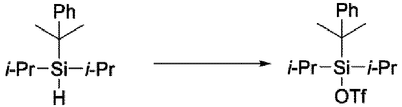

Di-i-propylcumylsilane (3.52 g, 15.0 mmol) was dissolved in methylene chloride (10.0 g), trifluoromethanesulfonic acid (2.26 g, 15.0 mmol) was added dropwise thereto under ice-cooling and the mixture was stirred for 30 minutes. The formed di-i-propylcumylsilyltriflate (5.75 g, 15.0 mmol) was used in the next reaction as a methylene chloride solution without isolation.

### Synthetic Example 50: Synthesis of Cbz-Phe-OIPCS

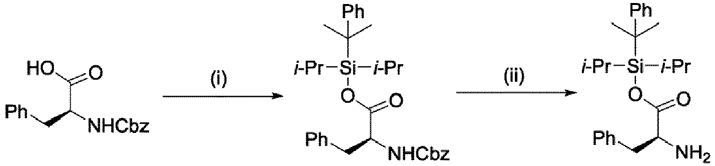

(i) Cbz-Phe-OH (3.00 g, 10.0 mmol) and di-i-propylcumylsilyltriflate (5.75 g, 15.0 mmol) were mixed with methylene chloride (49.9 g), the mixture was cooled to 0°C, N,N-diisopropylethylamine (2.59 g, 20.1 mmol) was added dropwise thereto, and the mixture was stirred at room temperature for 2 hours. The obtained reaction mixture was washed with an aqueous saturated ammonium chloride solution (40.0 g), and then, washed with an aqueous saturated sodium hydrogen carbonate solution (40.0 g) and the obtained organic layer was concentrated to obtain Cbz-Phe-OIPCS (5.90 g) as a crude product.
   MASS (ESI+) m/z; 533.1 (M+H)+
(ii) Cbz-Phe-OIPCS (5.90 g) was mixed with 2,2,2-trifluoroethanol (34.8 g), 10% by mass Pd-C (0.53 g, 0.50 mmol) and triethylsilane (4.66 g, 40.1 mmol) were added thereto and the mixture was stirred at room temperature for 3 hours. The reaction mixture was filtered, the obtained filtrate was concentrated and the concentrate was purified by silica gel column chromatography to obtain H-Phe-OIPCS (3.37 g, 2-Step Yield: 85%) as a colorless liquid.
   MASS (ESI+) m/z; 399.4 (M+H)+

### Synthetic Example 51: Synthesis of Fmoc-Phe-Phe-Phe-OIPCS

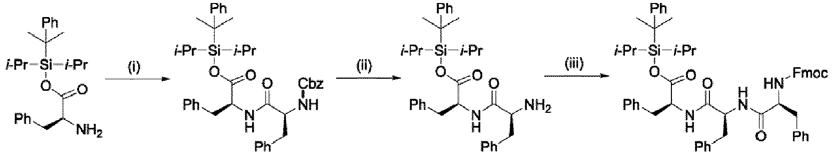

(i) H-Phe-OIPCS (0.50 g, 1.26 mmol) was dissolved in methylene chloride (8.4 g), Cbz-Phe-OH (0.57 g, 1.89 mmol) and 1-ethyl-3-(3-dimethylaminopropyl)carbodiimide hydrochloride (0.36 g, 1.89 mmol) were added thereto under ice-cooling and the mixture was stirred for 20 minutes. After returning to room temperature and stirring for further 1 hour, methylene chloride (19.7 g) and water (20.5 g) were added thereto and the liquids were separated. The organic layer was washed with an aqueous saturated sodium hydrogen carbonate solution (25.0 g) and an aqueous saturated sodium chloride solution (14.1 g) in this order, and the obtained organic layer was concentrated and purified by silica gel column chromatography to obtain Cbz-Phe-Phe-OIPCS (0.85 g, Yield: 99%) as a colorless liquid.
   MASS (ESI+) m/z; 679.6 (M+H)+
(ii) Cbz-Phe-Phe-OIPCS (0.85 g, 1.25 mmol) was mixed with 2,2,2-trifluoroethanol (8.7 g), 10% by mass Pd-C (0.066 g, 0.062 mmol) and triethylsilane (0.58 g, 4.99 mmol) were added thereto and the mixture was stirred at room temperature for 3 hours. The reaction mixture was filtered, and the obtained filtrate was concentrated to obtain H-Phe-Phe-OIPCS (0.98 g) as a crude product.
   MASS (ESI+) m/z; 545.5 (M+H)+
(iii) H-Phe-Phe-OIPCS (0.98 g) was dissolved in methylene chloride (8.3 g), Fmoc-Phe-OH (0.63 g, 1.62 mmol) and 1-ethyl-3-(3-dimethylaminopropyl)carbodiimide hydrochloride (0.31 g, 1.62 mmol) were added thereto under ice-cooling and the mixture was stirred for 20 minutes. After returning to room temperature and stirring for further 1 hour, methylene chloride (26.6 g) and water (28.8 g) were added thereto and the liquids were separated. The organic layer was washed with an aqueous saturated sodium hydrogen carbonate solution (30.3 g) and an aqueous saturated sodium chloride solution (20.0 g) in this order, and the obtained organic layer was concentrated and purified by silica gel column chromatography to obtain Fmoc-Phe-Phe-Phe-OIPCS (1.07 g, 2-Step Yield: 94%) as a white solid.
   MASS (ESI+) m/z; 915.0 (M+H)+

### Synthetic Example 52: Synthesis of Boc-Phe-Phe-Phe-Phe-OIPCS

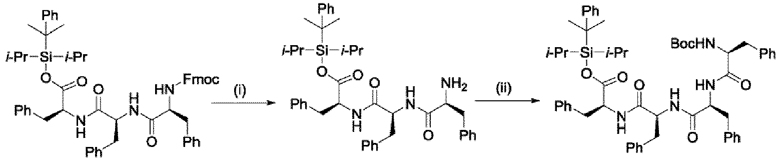

(i) Fmoc-Phe-Phe-Phe-OIPCS (0.83 g, 0.91 mmol) was mixed with methylene chloride (8.0 g) at room temperature, diethylamine (0.66 g, 9.06 mmol) was added thereto and the mixture was stirred for 2 hours. To the reaction mixture was added 8% by mass aqueous hydrogen chloride solution (3.0 g), and then, the mixture was diluted with methylene chloride (29.3 g) and the liquids were separated. The organic layer was washed with an aqueous saturated sodium chloride solution (16.0 g), and the obtained organic layer was concentrated and purified by silica gel column chromatography to obtain H-Phe-Phe-Phe-OIPCS (0.54 g, Yield: 86%) as a white solid.
   MASS (ESI+) m/z; 692.6 (M+H)+
ii) H-Phe-Phe-Phe-OIPCS (0.54 g, 0.78 mmol) was dissolved in methylene chloride (5.2 g), Boc-Phe-OH (0.23 g, 0.86 mmol) and 1-ethyl-3-(3-dimethylaminopropyl)carbodiimide hydrochloride (0.16 g, 0.86 mmol) were added thereto under ice-cooling and the mixture was stirred for 20 minutes. After returning to room temperature and stirring for further 1 hour, methylene chloride (18.7 g) and water (15.0 g) were added thereto and the liquids were separated. The organic layer was washed with an aqueous saturated sodium hydrogen carbonate solution (16.3 g), an aqueous saturated sodium chloride solution (13.8 g) in this order, the obtained organic layer was concentrated and purified by silica gel column chromatography to obtain Boc-Phe-Phe-Phe-Phe-OIPCS (0.59 g, Yield: 81%) as a white solid.
   MASS (ESI+) m/z; 939.9 (M+H)+

### Synthetic Example 53: Synthesis of Cbz-Phe-Phe-Phe-Phe-Phe-OIPCS

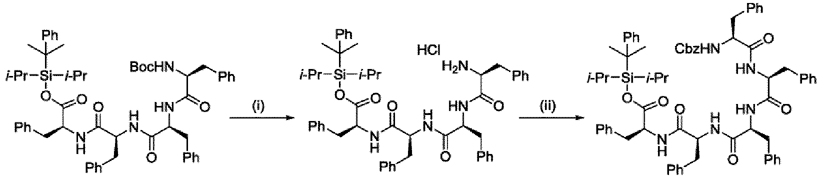

(i) Boc-Phe-Phe-Phe-Phe-OIPCS (0.59 g, 0.63 mmol) was dissolved in methylene chloride (4.1 g), 15% by mass hydrogen chloride-1,4-dioxane (4.74 g, 19.0 mmol) was added thereto under ice-cooling and the mixture was stirred for 20 minutes. After returning to room temperature and stirring for further 1 hour, the obtained reaction mixture was concentrated and subjected to azeotropic distillation with toluene (8.7 g) twice to obtain H-Phe-Phe-Phe-Phe-OIPCS hydrochloride (0.56 g) as a crude product.
   MASS (ESI+) m/z; 840.6 (M+H-HCl)+
(ii) H-Phe-Phe-Phe-Phe-OIPCS hydrochloride (0.10 g) was dissolved in methylene chloride (1.5 g), Cbz-Phe-OH (0.038 g, 0.13 mmol), O-(7-azabenzotriazol-1-yl)-N,N,N',N'-tetramethyluronium hexafluorophosphate (0.048 g, 0.13 mmol) and N,N-diisopropylethylamine (0.022 g, 0.17 mmol) were added thereto under ice-cooling and the mixture was stirred for 30 minutes. After returning to room temperature and stirring for further 1 hour, methylene chloride (15.4 g) and 10% by mass aqueous citric acid solution (6.8 g) were added thereto and the liquids were separated. The organic layer was washed with an aqueous saturated sodium hydrogen carbonate solution (12.0 g) and an aqueous saturated sodium chloride solution (8.0 g) in this order, and the obtained organic layer was concentrated and purified by silica gel column chromatography to obtain Cbz-Phe-Phe-Phe-Phe-Phe-OIPCS (0.12 g, 2-Step Yield: 93%) as a white solid.
   MASS (ESI+) m/z; 1120.9 (M+H)+

### Reference Synthetic Example 8: Synthesis of CPCS-OTf

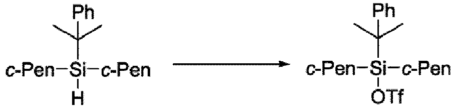

Di-cyclopentylcumylsilane (2.00 g, 6.98 mmol) was dissolved in methylene chloride (4.6 g), trifluoromethanesulfonic acid (1.05 g, 6.98 mmol) was added dropwise thereto under ice-cooling and the mixture was stirred for 30 minutes. The formed di-cyclopentylcumylsilyltriflate (3.03 g, 6.98 mmol) was used in the next reaction as a methylene chloride solution without isolation.

### Synthetic Example 54: Synthesis of Cbz-Phe-OCPCS

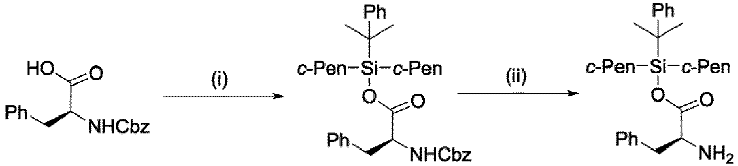

(i) Cbz-Phe-OH (1.39 g, 4.65 mmol) and di-cyclopentylcumylsilyltriflate (3.03 g, 6.98 mmol) were mixed with methylene chloride (23.3 g), the mixture was cooled to 0°C, N,N-diisopropylethylamine (1.20 g, 9.31 mmol) was added dropwise thereto and the mixture was stirred at room temperature for 2 hours. The obtained reaction mixture was washed with an aqueous saturated ammonium chloride solution (21.1 g), and then, washed with an aqueous saturated sodium hydrogen carbonate solution (25.8 g), and the obtained organic layer was concentrated to obtain Cbz-Phe-OCPCS (3.05 g) as a crude product.
   MASS (ESI+) m/z; 584.4 (M+H)+
(ii) Cbz-Phe-OCPCS (3.05 g) was mixed with 2,2,2-trifluoroethanol (12.9 g) and methylene chloride (3.1 g), 10% by mass Pd-C (0.50 g, 0.47 mmol) and triethylsilane (2.16 g, 18.6 mmol) were added thereto and the mixture was stirred at room temperature for 1 hour. The reaction mixture was filtered, the obtained filtrate was concentrated and the concentrate was purified by silica gel column chromatography to obtain H-Phe-OCPCS (1.91 g, 2-Step Yield: 91%) as a colorless liquid.
   MASS (ESI+) m/z; 450.4 (M+H)+

### Synthetic Example 55: Synthesis of Fmoc-Phe-Phe-Phe-OCPCS

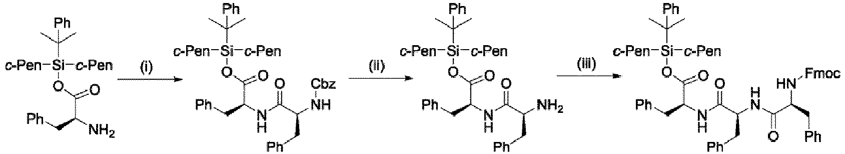

(i) H-Phe-OCPCS (0.75 g, 1.67 mmol) was dissolved in methylene chloride (11.1 g), Cbz-Phe-OH (0.60 g, 2.00 mmol) and 1-ethyl-3-(3-dimethylaminopropyl)carbodiimide hydrochloride (0.38 g, 2.00 mmol) were added thereto under ice-cooling and the mixture was stirred for 20 minutes. After returning to room temperature and stirring for further 1 hour, methylene chloride (18.0 g) and water (24.1 g) were added thereto and the liquids were separated. The organic layer was washed with an aqueous saturated sodium hydrogen carbonate solution (20.5 g) and an aqueous saturated sodium chloride solution (18.4 g) in this order, and the obtained organic layer was concentrated and purified by silica gel column chromatography to obtain Cbz-Phe-Phe-OCPCS (1.20 g, Yield: 99%) as a white liquid.
   MASS (ESI+) m/z; 731.4 (M+H)+
(ii) Cbz-Phe-Phe-OCPCS (1.20 g, 1.64 mmol) was mixed with 2,2,2-trifluoroethanol (6.1 g) and methylene chloride (1.5 g), 10% by mass Pd-C (0.18 g, 0.16 mmol) and triethylsilane (0.76 g, 6.57 mmol) were added thereto and the mixture was stirred at room temperature for 1 hour. The reaction mixture was filtered, and the obtained filtrate was concentrated to obtain H-Phe-Phe-OCPCS (1.49 g) as a crude product.
   MASS (ESI+) m/z; 597.4 (M+H)+
(iii) H-Phe-Phe-OCPCS (1.49 g) was dissolved in methylene chloride (10.9 g), Fmoc-Phe-OH (0.76 g, 1.97 mmol) and 1-ethyl-3-(3-dimethylaminopropyl)carbodiimide hydrochloride (0.38 g, 1.97 mmol) were added thereto under ice-cooling and the mixture was stirred for 20 minutes. After returning to room temperature and stirring for further 1 hour, methylene chloride (34.7 g) and water (30.0 g) were added thereto and the liquids were separated. The organic layer was washed with an aqueous saturated sodium hydrogen carbonate solution (29.0 g) and an aqueous saturated sodium chloride solution (17.7 g) in this order, and the organic layer was concentrated. The obtained solid was washed with 40% by mass ethyl acetate/hexane solution twice to obtain Fmoc-Phe-Phe-Phe-OCPCS (1.50 g, 2-Step Yield: 95%) as a white solid.
   MASS (ESI+) m/z; 966.9 (M+H)+

### Synthetic Example 56: Synthesis of Boc-Phe-Phe-Phe-Phe-OCPCS

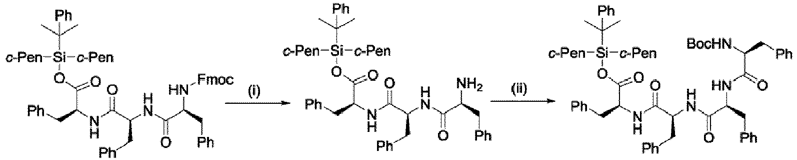

(i) Fmoc-Phe-Phe-Phe-OCPCS (1.25 g, 1.29 mmol) was mixed with methylene chloride (17.2 g) at room temperature, diethylamine (0.95 g, 12.9 mmol) was added thereto and the mixture was stirred for 3 hours. To the reaction mixture was added 8% by mass aqueous hydrogen chloride solution (5.0 g), the mixture was diluted with methylene chloride (37.4 g) and the liquids were separated. The organic layer was washed with an aqueous saturated sodium chloride solution (19.5 g), and the obtained organic layer was concentrated and purified by silica gel column chromatography to obtain H-Phe-Phe-Phe-OCPCS (0.84 g, Yield: 87%) as a white solid.
   MASS (ESI+) m/z; 744.5 (M+H)+
ii) H-Phe-Phe-Phe-OCPCS (0.84 g, 1.12 mmol) was dissolved in methylene chloride (7.5 g), Boc-Phe-OH (0.33 g, 1.24 mmol) and 1-ethyl-3-(3-dimethylaminopropyl)-carbodiimide hydrochloride (0.24 g, 1.24 mmol) were added thereto under ice-cooling and the mixture was stirred for 20 minutes. After returning to room temperature and stirring for further 1 hour, methylene chloride (26.6 g) and water (18.0 g) were added thereto and the liquids were separated. The organic layer was washed with an aqueous saturated sodium hydrogen carbonate solution (22.4 g) and an aqueous saturated sodium chloride solution (15.2 g) in this order, and the obtained organic layer was concentrated and purified by silica gel column chromatography to obtain Boc-Phe-Phe-Phe-Phe-OCPCS (1.01 g, Yield: 91%) as a white solid.
   MASS (ESI+) m/z; 991.7 (M+H)+

### Synthetic Example 57: Synthesis of Cbz-Phe-Phe-Phe-Phe-Phe-OCPCS

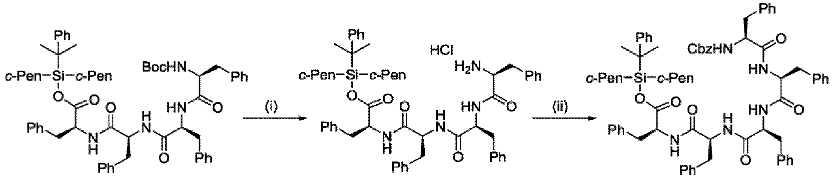

(i) Boc-Phe-Phe-Phe-Phe-OCPCS (0.90 g, 0.91 mmol) was dissolved in methylene chloride (6.0 g), 15% by mass hydrogen chloride-1,4-dioxane (6.8 g, 27.2 mmol) was added thereto under ice-cooling and the mixture was stirred for 20 minutes. After returning to room temperature and stirring for further 90 minutes, the obtained reaction mixture was concentrated and subjected to azeotropic distillation with toluene (7.0 g) twice to obtain H-Phe-Phe-Phe-Phe-OCPCS hydrochloride (0.87 g) as a crude product.
   MASS (ESI+) m/z; 891.6 (M+H-HCl)+
(ii) H-Phe-Phe-Phe-Phe-OCPCS hydrochloride (0.10 g) was dissolved in methylene chloride (0.7 g) and N,N-dimethylformamide (0.5 g), Cbz-Phe-OH (0.036 g, 0.12 mmol), O-(7-azabenzotriazol-1-yl)-N,N,N' ,N' -tetramethyluronium hexafluorophosphate (0.045 g, 0.12 mmol) and N,N-diisopropylethylamine (0.028 g, 0.22 mmol) were added thereto under ice-cooling and the mixture was stirred for 30 minutes. After returning to room temperature and stirring for further 1 hour, methylene chloride (14.0 g) and 8% by mass aqueous hydrogen chloride solution (7.0 g) were added thereto and the liquids were separated. The organic layer was washed with an aqueous saturated sodium hydrogen carbonate solution (13.2 g) and an aqueous saturated sodium chloride solution (10.0 g) in this order, and the obtained organic layer was concentrated and purified by silica gel column chromatography to obtain Cbz-Phe-Phe-Phe-Phe-Phe-OCPCS (0.091 g, 2-Step Yield: 72%) as a white solid.
   MASS (ESI+) m/z; 1173.7 (M+H)+

### Reference Synthetic Example 9: Synthesis of CHCS-OTf

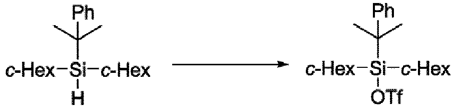

Di-cyclohexylcumylsilane (2.00 g, 6.36 mmol) was dissolved in methylene chloride (5.3 g), trifluoromethanesulfonic acid (0.95 g, 6.36 mmol) was added dropwise thereto under ice-cooling and the mixture was stirred for 40 minutes. The formed di-cyclohexylcumylsilyltriflate (2.94 g, 6.36 mmol) was used in the next reaction as a methylene chloride solution without isolation.

### Synthetic Example 58: Synthesis of Cbz-Phe-OCHCS

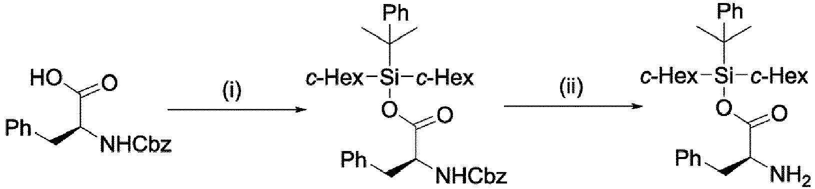

(i) Cbz-Phe-OH (1.27 g, 4.24 mmol) and di-cyclohexylcumylsilyltriflate (2.94 g, 6.36 mmol) were mixed with methylene chloride (26.6 g), the mixture was cooled to 0°C, N,N-diisopropylethylamine (1.10 g, 8.48 mmol) was added dropwise thereto and the mixture was stirred at room temperature for 2 hours. The obtained reaction mixture was washed with an aqueous saturated ammonium chloride solution (30.0 g), and then, washed with an aqueous saturated sodium hydrogen carbonate solution (30.0 g), and the obtained organic layer was concentrated to obtain Cbz-Phe-OCHCS (3.43 g) as a crude product.
   MASS (ESI+) m/z; 612.5 (M+H)+
(ii) Cbz-Phe-OCHCS (3.43 g) was mixed with 2,2,2-trifluoroethanol (8.3 g) and methylene chloride (5.3 g), 10% by mass Pd-C (0.45 g, 0.42 mmol) and triethylsilane (2.96 g, 25.4 mmol) were added thereto and the mixture was stirred at room temperature for 4 hours. The reaction mixture was filtered, the obtained filtrate was concentrated and the concentrate was purified by silica gel column chromatography to obtain H-Phe-OCHCS (1.81 g, 2-Step Yield: 89%) as a colorless liquid.
   MASS (ESI+) m/z; 478.4 (M+H)+

### Synthetic Example 59: Synthesis of Fmoc-Phe-Phe-Phe-OCHCS

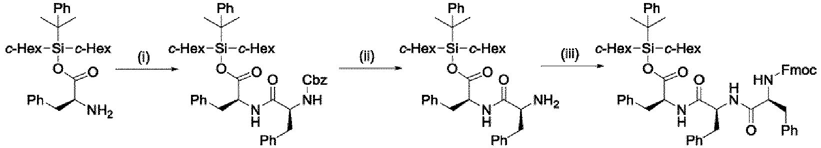

(i) H-Phe-OCHCS (0.60 g, 1.26 mmol) was dissolved in methylene chloride (8.0 g), Cbz-Phe-OH (0.45 g, 1.51 mmol) and 1-ethyl-3-(3-dimethylaminopropyl)carbodiimide hydrochloride (0.29 g, 1.51 mmol) were added thereto under ice-cooling and the mixture was stirred for 20 minutes. After returning to room temperature and stirring for further 1 hour, methylene chloride (8.0 g) and water (16.0 g) were added thereto and the liquids were separated. The organic layer was washed with an aqueous saturated sodium hydrogen carbonate solution (25.0 g) and an aqueous saturated sodium chloride solution (25.0 g) in this order, and the obtained organic layer was concentrated and purified by silica gel column chromatography to obtain Cbz-Phe-Phe-OCHCS (0.87 g, Yield: 91%) as a colorless liquid.
   MASS (ESI+) m/z; 759.5 (M+H)+
(ii) Cbz-Phe-Phe-OCHCS (0.87 g, 1.14 mmol) was mixed with 2,2,2-trifluoroethanol (4.2 g) and methylene chloride (2.7 g), 10% by mass Pd-C (0.12 g, 0.11 mmol) and triethylsilane (0.80 g, 6.85 mmol) were added thereto and the mixture was stirred at room temperature for 4 hours. The reaction mixture was filtered, and the obtained filtrate was concentrated to obtain H-Phe-Phe-OCHCS (0.94 g) as a crude product.
   MASS (ESI+) m/z; 625.5 (M+H)+
(iii) H-Phe-Phe-OCHCS (0.94 g) was dissolved in methylene chloride (6.7 g), Fmoc-Phe-OH (0.49 g, 1.26 mmol) and 1-ethyl-3-(3-dimethylaminopropyl)carbodiimide hydrochloride (0.24 g, 1.26 mmol) were added thereto under ice-cooling and the mixture was stirred for 20 minutes. After returning to room temperature and stirring for further 1 hour, methylene chloride (6.7 g) and water (10.0 g) were added thereto and the liquids were separated. The organic layer was washed with an aqueous saturated sodium hydrogen carbonate solution (15.0 g) and an aqueous saturated sodium chloride solution (10.0 g) in this order, and the obtained organic layer was concentrated and purified by silica gel column chromatography to obtain Fmoc-Phe-Phe-Phe-OCHCS (0.84 g, 2-Step Yield: 74%) as a white solid.
   MASS (ESI+) m/z; 994.7 (M+H)+

### Synthetic Example 60: Synthesis of Boc-Phe-Phe-Phe-Phe-OCHCS

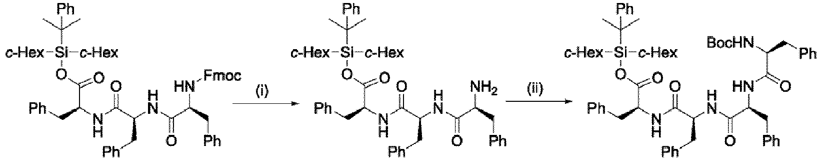

(i) Fmoc-Phe-Phe-Phe-OCHCS (0.70 g, 0.70 mmol) was mixed with methylene chloride (6.3 g) at room temperature, diethylamine (1.03 g, 14.0 mmol) was added thereto and the mixture was stirred for 4 hours. To the reaction mixture was added 8% by mass aqueous hydrogen chloride solution (10.0 g), the mixture was diluted with methylene chloride (6.7 g) and the liquids were separated. The organic layer was washed with an aqueous saturated sodium chloride solution (10.0 g), and the obtained organic layer was concentrated and purified by silica gel column chromatography to obtain H-Phe-Phe-Phe-OCHCS (0.30 g, Yield: 55%) as a white solid.
   MASS (ESI+) m/z; 772.6 (M+H)+
(ii) H-Phe-Phe-Phe-OCHCS (0.060 g, 0.078 mmol) was dissolved in methylene chloride (2.7 g), Boc-Phe-OH (0.023 g, 0.085 mmol), O-(7-azabenzotriazol-1-yl)-N,N,N',N'-tetramethyluronium hexafluorophosphate (0.033 g, 0.085 mmol) and N,N-diisopropylethylamine (0.014 g, 0.11 mmol) were added thereto under ice-cooling and the mixture was stirred for 20 minutes. After returning to room temperature and stirring for further 1 hour, methylene chloride (2.7 g) and water (10.0 g) were added thereto and the liquids were separated. The organic layer was washed with an aqueous saturated sodium hydrogen carbonate solution (15.0 g) and an aqueous saturated sodium chloride solution (10.0 g) in this order, and the obtained organic layer was concentrated and purified by silica gel column chromatography to obtain Boc-Phe-Phe-Phe-Phe-OCHCS (0.074 g, Yield: 94%) as a white solid.
   MASS (ESI+) m/z; 1019.7 (M+H)+

### Synthetic Example 61: Synthesis of Cbz-Phe-Phe-Phe-Phe-Phe-OCHCS

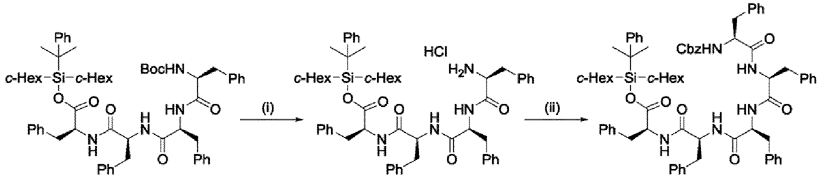

(i) Boc-Phe-Phe-Phe-Phe-OCHCS (0.074 g, 0.073 mmol) was dissolved in methylene chloride (2.7 g), 15% by mass hydrogen chloride-1,4-dioxane (2.0 g, 8.03 mmol) was added thereto under ice-cooling and the mixture was stirred for 10 minutes. After returning to room temperature and stirring for further 1 hour, the obtained reaction mixture was concentrated and subjected to azeotropic distillation with toluene (8.7 g) twice to obtain H-Phe-Phe-Phe-Phe-OCHCS hydrochloride (0.058 g) as a crude product.
   MASS (ESI+) m/z; 919.7 (M+H-HCl)+
(ii) H-Phe-Phe-Phe-Phe-OCHCS hydrochloride (0.058 g) was dissolved in methylene chloride (2.7 g), Cbz-Phe-OH (0.021 g, 0.069 mmol), O-(7-azabenzotriazol-1-yl)-N,N,N',N'-tetramethyluronium hexafluorophosphate (0.026 g, 0.069 mmol) and N,N-diisopropylethylamine (0.016 g, 0.126 mmol) were added thereto under ice-cooling and the mixture was stirred for 10 minutes. After returning to room temperature and stirring for further 1 hour, methylene chloride (2.7 g) and 10% by mass aqueous citric acid solution (10.0 g) were added thereto and the liquids were separated. The organic layer was washed with an aqueous saturated sodium hydrogen carbonate solution (10.0 g) and an aqueous saturated sodium chloride solution (10.0 g) in this order, and the obtained organic layer was concentrated and purified by silica gel column chromatography to obtain Cbz-Phe-Phe-Phe-Phe-Phe-OCHCS (0.040 g, 2-Step Yield: 54%) as a white solid.
   MASS (ESI+) m/z; 1201.8 (M+H)+

### Reference Synthetic Example 10: Synthesis of SBCS-OTf

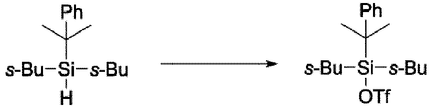

Di-s-butylcumylsilane (2.00 g, 7.62 mmol) was dissolved in methylene chloride (5.1 g), trifluoromethanesulfonic acid (1.14 g, 7.62 mmol) was added dropwise thereto under ice-cooling and the mixture was stirred for 30 minutes. The formed di-s-butylcumylsilyltriflate (3.13 g, 7.62 mmol) was used in the next reaction as a methylene chloride solution without isolation.

### Synthetic Example 62: Synthesis of Cbz-Phe-OSBCS

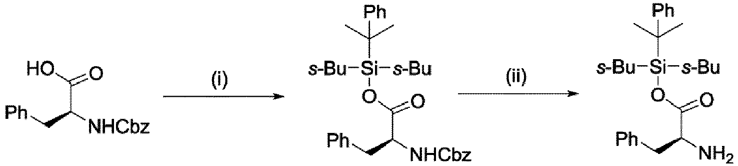

(i) Cbz-Phe-OH (1.52 g, 5.08 mmol) and di-s-butylcumylsilyltriflate (3.13 g, 7.62 mmol) were mixed with methylene chloride (25.3 g), the mixture was cooled to 0°C, N,N-diisopropylethylamine (1.31 g, 10.2 mmol) was added dropwise thereto and the mixture was stirred at room temperature for 2 hours. The obtained reaction mixture was washed with an aqueous saturated ammonium chloride solution (21.1 g), and then, washed with an aqueous saturated sodium hydrogen carbonate solution (25.8 g), and the obtained organic layer was concentrated to obtain Cbz-Phe-OSBCS (3.18 g) as a crude product.
   MASS (ESI+) m/z; 560.4 (M+H)+
(ii) Cbz-Phe-OSBCS (3.18 g) was mixed with 2,2,2-trifluoroethanol (14.1 g) and methylene chloride (3.4 g), 10% by mass Pd-C (0.54 g, 0.51 mmol) and triethylsilane (2.36 g, 20.3 mmol) were added thereto and the mixture was stirred at room temperature for 1 hour. The reaction mixture was filtered, the obtained filtrate was concentrated, and the concentrate was purified by silica gel column chromatography to obtain H-Phe-OSBCS (2.05 g, 2-Step Yield: 95%) as a colorless liquid.
   MASS (ESI+) m/z; 426.4 (M+H)+

### Synthetic Example 63: Synthesis of Fmoc-Phe-Phe-Phe-OSBCS

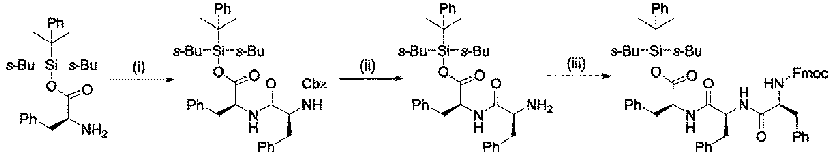

(i) H-Phe-OSBCS (0.60 g, 1.41 mmol) was dissolved in methylene chloride (9.4 g), Cbz-Phe-OH (0.51 g, 1.69 mmol) and 1-ethyl-3-(3-dimethylaminopropyl)carbodiimide hydrochloride (0.32 g, 1.69 mmol) were added thereto under ice-cooling and the mixture was stirred for 20 minutes. After returning to room temperature and stirring for further 1 hour, methylene chloride (16.9 g), water (20.1 g) were added thereto and the liquids were separated. The organic layer was washed with an aqueous saturated sodium hydrogen carbonate solution (17.3 g) and an aqueous saturated sodium chloride solution (15.0 g) in this order, and the obtained organic layer was concentrated and purified by silica gel column chromatography to obtain Cbz-Phe-Phe-OSBCS (0.94 g, Yield: 95%) as a colorless liquid.
   MASS (ESI+) m/z; 707.5 (M+H)+
(ii) Cbz-Phe-Phe-OSBCS (0.94 g, 1.34 mmol) was mixed with 2,2,2-trifluoroethanol (4.9 g) and methylene chloride (1.2 g), 10% by mass Pd-C (0.14 g, 0.13 mmol) and triethylsilane (0.62 g, 5.34 mmol) were added thereto and the mixture was stirred at room temperature for 1 hour. The reaction mixture was filtered, and the obtained filtrate was concentrated to obtain H-Phe-Phe-OSBCS (1.05 g) as a crude product. MASS (ESI+) m/z; 573.5 (M+H)+
(iii) H-Phe-Phe-OSBCS (1.05 g) was dissolved in methylene chloride (8.9 g), Fmoc-Phe-OH (0.62 g, 1.60 mmol) and 1-ethyl-3-(3-dimethylaminopropyl)carbodiimide hydrochloride (0.31 g, 1.60 mmol) were added thereto under ice-cooling and the mixture was stirred for 20 minutes. After returning to room temperature and stirring for further 1 hour, methylene chloride (22.4 g) and water (19.4 g) were added thereto and the liquids were separated. The organic layer was washed with an aqueous saturated sodium hydrogen carbonate solution (20.0 g) and an aqueous saturated sodium chloride solution (11.1 g) in this order, and the obtained organic layer was concentrated and purified by silica gel column chromatography to obtain Fmoc-Phe-Phe-Phe-OSBCS (1.22 g, 2-Step Yield: 97%) as a white solid.
   MASS (ESI+) m/z; 942.7 (M+H)+

### Synthetic Example 64: Synthesis of Boc-Phe-Phe-Phe-Phe-OSBCS

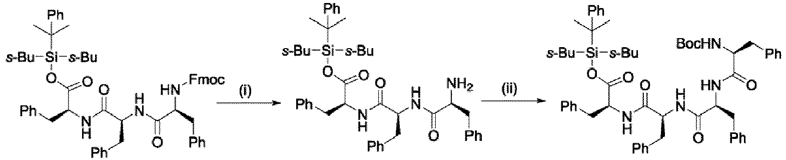

(i) Fmoc-Phe-Phe-Phe-OSBCS (1.00 g, 1.06 mmol) was mixed with methylene chloride (14.1 g) at room temperature, diethylamine (0.78 g, 10.6 mmol) was added thereto and the mixture was stirred for 3 hours. To the reaction mixture was added 8% by mass aqueous hydrogen chloride solution (6.0 g), the mixture was diluted with methylene chloride (26.6 g) and the liquids were separated. The organic layer was washed with an aqueous saturated sodium chloride solution (15.0 g), and the obtained organic layer was concentrated and purified by silica gel column chromatography to obtain H-Phe-Phe-Phe-OSBCS (0.62 g, Yield: 80%) as a white solid.
   MASS (ESI+) m/z; 720.8 (M+H)+
ii) H-Phe-Phe-Phe-OSBCS (0.45 g, 0.63 mmol) was dissolved in methylene chloride (4.2 g), Boc-Phe-OH (0.18 g, 0.69 mmol) and 1-ethyl-3-(3-dimethylaminopropyl)-carbodiimide hydrochloride (0.13 g, 0.69 mmol) were added thereto under ice-cooling and the mixture was stirred for 20 minutes. After returning to room temperature and stirring for further 1 hour, methylene chloride (13.3 g) and water (10.6 g) were added thereto and the liquids were separated. The organic layer was washed with an aqueous saturated sodium hydrogen carbonate solution (15.3 g) and an aqueous saturated sodium chloride solution (8.0 g) in this order, and the obtained organic layer was concentrated and purified by silica gel column chromatography to obtain Boc-Phe-Phe-Phe-Phe-OSBCS (0.58 g, Yield: 96%) as a white solid.
   MASS (ESI+) m/z; 967.7 (M+H)+

### Synthetic Example 65: Synthesis of Cbz-Phe-Phe-Phe-Phe-Phe-OSBCS

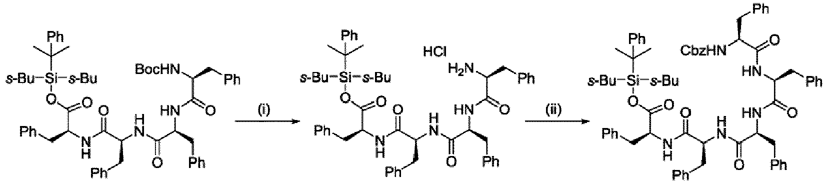

(i) Boc-Phe-Phe-Phe-Phe-OSBCS (0.58 g, 0.60 mmol) was dissolved in methylene chloride (4.0 g), 15% by mass hydrogen chloride-1,4-dioxane (4.5 g, 18.0 mmol) was added thereto under ice-cooling and the mixture was stirred for 20 minutes. After returning to room temperature and stirring for further 1 hour, the obtained reaction mixture was concentrated and subjected to azeotropic distillation with toluene (6.0 g) twice to obtain H-Phe-Phe-Phe-Phe-OSBCS hydrochloride (0.58 g) as a crude product.
   MASS (ESI+) m/z; 867.6 (M+H-HCl)+
(ii) H-Phe-Phe-Phe-Phe-OSBCS hydrochloride (0.10 g) was dissolved in methylene chloride (0.7 g) and N,N-dimethylformamide (0.5 g), Cbz-Phe-OH (0.036 g, 0.12 mmol), O-(7-azabenzotriazol-1-yl)-N,N,N' ,N' -tetramethyluronium hexafluorophosphate (0.046 g, 0.12 mmol) and N,N-diisopropylethylamine (0.029 g, 0.22 mmol) were added thereto under ice-cooling and the mixture was stirred for 30 minutes. After returning to room temperature and stirring for further 1 hour, methylene chloride (11.8 g) and 8% by mass aqueous hydrogen chloride solution (6.1 g) were added thereto and the liquids were separated. The organic layer was washed with an aqueous saturated sodium hydrogen carbonate solution (12.0 g) and an aqueous saturated sodium chloride solution (9.7 g) in this order, and the obtained organic layer was concentrated and purified by silica gel column chromatography to obtain Cbz-Phe-Phe-Phe-Phe-Phe-OSBCS (0.12 g, 2-Step Yield: 96%) as a white solid.
   MASS (ESI+) m/z; 1148.8 (M+H)+

### Synthetic Example 66: Synthesis of H-Phe-OSi(tBu)₃

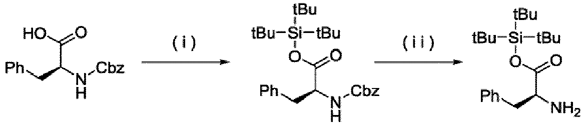

(i) Tri-tert-butylsilane (0.24 g, 1.2 mmol) was mixed with methylene chloride (4.0 g), the mixture was cooled to 0°C, trifluoromethanesulfonic acid (0.20 g, 1.3 mmol) was added dropwise thereto, and the mixture was stirred at room temperature for 1 hour. The obtained reaction mixture was cooled to 0°C, a methylene chloride (2.0 g) solution of Cbz-Phe-OH (0.40 g, 1.3 mmol) and imidazole (0.14 g, 2.1 mmol) was added dropwise thereto, and the mixture was stirred at 40°C for 15 hours. The obtained reaction mixture was diluted with chloroform, and washed with 10% by mass aqueous ammonium chloride solution (2 mL) and water (2 mL) in this order. The obtained organic layer was concentrated and purified by silica gel column chromatography to obtain Cbz-Phe-OSi(tBu)₃(0.17 g, Yield: 28%) as a colorless liquid.
   MASS (ESI+) m/z; 498.4 (M+H)+
(ii) Cbz-Phe-OSi(tBu)₃ (0.16 g, 0.32 mmol) was mixed with 2,2,2-trifluoroethanol (3.2 g), 10% by mass Pd-C (48 mg) was added thereto and the mixture was stirred under a hydrogen gas atmosphere at room temperature for 4 hours. After filtering the reaction mixture, the obtained filtrate was concentrated to obtain H-Phe-OSi(tBu)₃ (0.12 g, Yield: 100%) as a pale brownish liquid.
   MASS (ESI+) m/z; 364.4 (M+H)+

### Synthetic Example 67: Synthesis of Boc-Ala-Val-Pro-Phe-OSi(tBu)₃

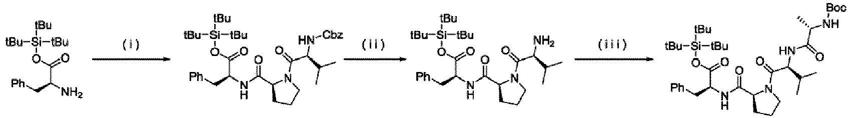

(i) H-Phe-OSi(tBu)₃ (120 mg, 0.33 mmol) and Cbz-Val-Pro-OH (138 mg, 0.40 mmol) were dissolved in methylene chloride (2.4 g), 1-ethyl-3-(3-dimethylaminopropyl)-carbodiimide hydrochloride (76 mg, 0.40 mmol) was added to the mixture under ice-cooling and the mixture was stirred for 2 hours. The obtained reaction mixture was diluted with chloroform (3 mL), and then, washed with 10% by mass aqueous ammonium chloride solution (2 mL) and the liquids were separated, and the aqueous layer was again extracted with chloroform. The obtained organic layer was concentrated and purified by silica gel column chromatography to obtain Cbz-Val-Pro-Phe-OSi(tBu)₃ (218 mg, Yield: 95%) as a colorless liquid.
   MASS (ESI+) m/z; 694.6 (M+H)+
(ii) Cbz-Val-Pro-Phe-OSi(tBu)₃ (0.19 g, 0.27 mmol) was mixed with 2,2,2-trifluoroethanol (3.8 g), 10% by mass Pd-C (57 mg) was added thereto and the mixture was stirred under a hydrogen gas atmosphere at room temperature for 3 hours. Further, 10% by mass Pd-C (19 mg) was added thereto and the mixture was stirred at room temperature for 17 hours. After filtering the reaction mixture, the obtained filtrate was concentrated to obtain H-Val-Pro-Phe-OSi(tBu)₃ (153 mg, Yield: 100%) as a brown solid.
   MASS (ESI+) m/z; 560.5 (M+H)+
(iii) H-Val-Pro-Phe-OSi(tBu)₃ (138 mg, 0.25 mmol) and Boc-Ala-OH (56 mg, 0.30 mmol) were dissolved in methylene chloride (1.4 g), 1-ethyl-3-(3-dimethylaminopropyl)carbodiimide hydrochloride (57 mg, 0.30 mmol) was added to the mixture under ice-cooling and the mixture was stirred for 1.5 hours. Further, Boc-Ala-OH (19 mg, 0.10 mmol) and 1-ethyl-3-(3-dimethylaminopropyl)carbodiimide hydrochloride (19 mg, 0.10 mmol) were added thereto, and the mixture was stirred for 7 hours. The obtained reaction mixture was diluted with chloroform, washed with 10% by mass aqueous ammonium chloride solution and 5% by mass aqueous sodium hydrogen carbonate solution in this order. The obtained organic layer was concentrated and purified by silica gel column chromatography to obtain Boc-Ala-Val-Pro-Phe-OSi(tBu)₃ (153 mg, Yield: 85%) as a white solid.
   MASS (ESI+) m/z; 731.6 (M+H)+

### Synthetic Example 68: Synthesis of H-Phe-Ala-Val-Pro-Phe-OSi(tBu)₃

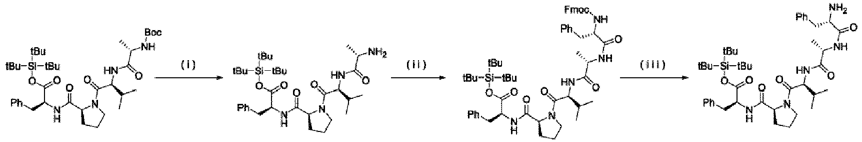

(i) Boc-Ala-Val-Pro-Phe-OSi(tBu)₃ (120 mg, 0.16 mmol) was mixed with methylene chloride (2.4 g), 15% by mass hydrogen chloride-1,4-dioxane (1.2 g) was added thereto and the mixture was stirred at room temperature for 23 hours. The obtained reaction mixture was concentrated to obtain H-Ala-Val-Pro-Phe-OSi(tBu)₃ hydrochloride (107 mg, Yield: 100%) as a white solid.
   MASS (ESI+) m/z; 631 (M+H)+
(ii) H-Ala-Val-Pro-Phe-OSi(tBu)₃ hydrochloride (107 mg, 0.15 mmol), Fmoc-Phe-OH (69 mg, 0.18 mmol) and diisopropylethylamine (25 mg, 0.19 mmol) were dissolved in methylene chloride (1.4 g), 1-ethyl-3-(3-dimethylaminopropyl)carbodiimide hydrochloride (34 mg, 0.18 mmol) were added thereto under ice-cooling and the mixture was stirred at room temperature for 2 hours. The obtained reaction mixture was diluted with chloroform, and then, washed with 10% by mass aqueous ammonium chloride solution and 5% by mass aqueous sodium hydrogen carbonate solution in this order. The obtained organic layer was concentrated and purified by silica gel column chromatography to obtain Fmoc-Phe-Ala-Val-Pro-Phe-OSi(tBu)₃ (146 mg, Yield: 99%) as a white solid.
   MASS (ESI+) m/z; 1000 (M+H)+
(iii) Fmoc-Phe-Ala-Val-Pro-Phe-OSi(tBu)₃ (130 mg, 0.13 mmol) was mixed with methylene chloride (2.0 g), the mixture was cooled to 0°C, diethylamine (0.19 g, 2.6 mmol) was added thereto and the mixture was stirred at room temperature for 3 hours, and at 35°C for 3 hours. After cooling to room temperature, the mixture was diluted with methylene chloride, and washed with 10% by mass aqueous ammonium chloride solution (2 mL) and water in this order. The obtained organic layer was concentrated and purified by silica gel column chromatography to obtain H-Phe-Ala-Val-Pro-Phe-OSi(tBu)₃ (97 mg, Yield: 97%) as a white solid.
   MASS (ESI+) m/z; 778 (M+H)+

### Synthetic Example 69: Synthesis of Cbz-Phe-Phe-Ala-Val-Pro-Phe-OH

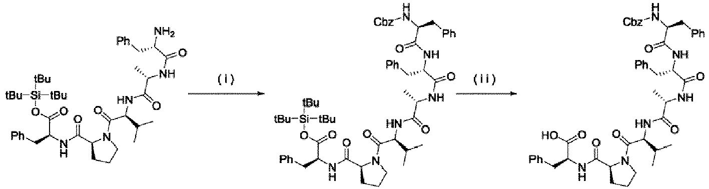

(i) H-Phe-Ala-Val-Pro-Phe-OSi(tBu)₃ (90 mg, 0.12 mmol) and Cbz-Phe-OH (42 mg, 0.14 mmol) were dissolved in methylene chloride (1.8 g), 1-ethyl-3-(3-dimethylaminopropyl)carbodiimide hydrochloride (27 mg, 0.14 mmol) was added thereto under ice-cooling and the mixture was stirred at room temperature for 0.5 hour. The obtained reaction mixture was diluted with chloroform, and then, washed with 10% by mass aqueous ammonium chloride solution and 5% by mass aqueous sodium hydrogen carbonate solution in this order. The obtained organic layer was concentrated and purified by silica gel column chromatography to obtain Cbz-Phe-Phe-Ala-Val-Pro-Phe-OSi(tBu)₃ (109 mg, Yield: 89%) as a white solid.
   MASS (ESI+) m/z; 1059.7 (M+H)+
(ii) Cbz-Phe-Phe-Ala-Val-Pro-Phe-OSi(tBu)₃ (55 mg, 0.052 mmol) was mixed with methanol (0.28 g) and tetrahydrofuran (0.83 g), potassium fluoride (6.0 mg, 0.10 mmol) was added thereto at 0°C and the mixture was stirred at 30°C for 22 hours. Potassium fluoride (12 mg, 0.20 mmol) and methanol (1 mL) were added thereto, and the mixture was further stirred at 30°C for 4 hours. The obtained reaction mixture was concentrated, and then, diluted with chloroform and washed with 5% by mass aqueous sodium chloride solution. The obtained organic layer was concentrated and purified by silica gel column chromatography to obtain Cbz-Phe-Phe-Ala-Val-Pro-Phe-OH (39 mg, Yield: 88%) as a white solid.
   MASS (ESI+) m/z; 861.5 (M+H)+

Synthetic Example 70: Synthesis of H-Phe-OSi(tBu)₂(CH₂TMS) 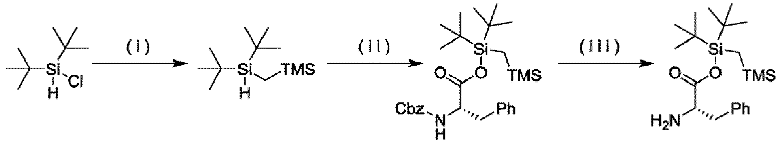
(i) To a tetrahydrofuran (2.5mL) solution of magnesium (0.17 g, 7.0 mmol) were added iodine (10 mg) and dibromoethane (10 µL) and (chloromethyl)trimethylsilane (0.86 g, 7.0 mmol) was added dropwise thereto at 30°C, and the mixture was stirred at 65°C for 2 hours. The obtained reaction mixture was cooled to room temperature, and then, it was added dropwise to a tetrahydrofuran (3 mL) solution of di-tert-butylchlorosilane (0.25 g, 1.4 mmol) and the mixture was stirred at 65°C for 5 hours. The obtained reaction mixture was cooled to room temperature, diluted with hexane, and then, the liquids were separated by 10% by mass aqueous ammonium chloride solution and 5% by mass aqueous sodium chloride solution in this order. The obtained organic layer was concentrated and purified by silica gel column chromatography to obtain di-tert-butylsilylmethyltrimethylsilane (0.24 g, Yield: 37%) as a colorless liquid.
   ¹H-NMR (CDCl₃)
   δ ppm: -0.34 (2H, d, J=3.0Hz), 0.07 (9H, s), 0.98 (18H, s), 3.42 (1H, m)
(ii) Di-tert-butylsilylmethyltrimethylsilane (0.17 g, 0.74 mmol) and dichloromethane (2.0 g) were mixed, trifluoromethanesulfonic acid (0.11 g, 0.73 mmol) was added dropwise thereto under ice-cooling and the mixture was stirred at room temperature for 1 hour. The obtained reaction mixture was cooled to 0°C, then, a methylene chloride (2.0 g) solution of Cbz-Phe-OH (0.20 g, 0.67 mmol) and imidazole (91 mg, 1.3 mmol) was added dropwise thereto, and the mixture was stirred at room temperature for 2 hours. The obtained reaction mixture was diluted with chloroform, and washed with 10% by mass aqueous ammonium chloride solution (2 mL) and 5% by mass sodium hydrogen carbonate (2 mL) in this order. The obtained organic layer was concentrated and purified by silica gel column chromatography to obtain Cbz-Phe-OSi(tBu)₂(CH₂TMS) (0.10 g, Yield: 29%) as a colorless liquid.
   MASS (ESI+) m/z; 528.4 (M+H)+
(iii) Cbz-Phe-OSi(tBu)₂(CH₂TMS) (95 mg, 0.18 mmol) was dissolved in 2,2,2-trifluoroethanol (1.9 g), 10% by mass Pd-C (29 mg) was added thereto and the mixture was stirred under a hydrogen gas atmosphere at 30°C for 5 hours. The reaction mixture was filtered, and the obtained filtrate was concentrated to obtain H-Phe-OSi(tBu)₂(CH₂TMS) (71 mg, Yield: 100%) as a colorless liquid.
   MASS (ESI+) m/z; 394.4 (M+H)+

### Synthetic Example 71: Synthesis of Fmoc-Phe-Phe-Phe-OSi(tBu)₂(CH₂TMS)

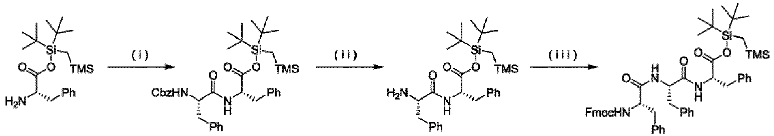

(i) H-Phe-OSi(tBu)₂(CH₂TMS) (71 mg, 0.18 mmol) and Cbz-Phe-OH (65 mg, 0.22 mmol) were mixed with methylene chloride (1.4 g), 1-ethyl-3-(3-dimethylaminopropyl)carbodiimide hydrochloride (42 mg, 0.22 mmol) was added thereto and the mixture was stirred for 0.5 hour. The obtained reaction mixture was diluted with chloroform (3 mL), and then, washed with 10% by mass aqueous ammonium chloride solution (2 mL), 5% by mass aqueous sodium hydrogen carbonate solution (2 mL) in this order. The obtained organic layer was concentrated and purified by silica gel column chromatography to obtain Cbz-Phe-Phe-OSi(tBu)₂(CH₂TMS) (97 mg, Yield: 80%) as a colorless liquid.
   MASS (ESI+) m/z; 675.5 (M+H)+
(ii) Cbz-Phe-Phe-OSi(tBu)₂(CH₂TMS) (81 mg, 0.12 mmol) was dissolved in 2,2,2-trifluoroethanol (1.6 g), 10% by mass Pd-C (24 mg) was added thereto and the mixture was stirred under a hydrogen gas atmosphere at room temperature for 4 hours. The reaction mixture was filtered and the obtained filtrate was concentrated to obtain H-Phe-Phe-OSi(tBu)₂(CH₂TMS) (65 mg, Yield: 100%) as a brown solid.
   MASS (ESI+) m/z; 541.4 (M+H)+
(iii), H-Phe-Phe-OSi(tBu)₂(CH₂TMS) (65 mg, 0.12 mmol) and Fmoc-Phe-OH (56 mg, 0.14 mmol) were dissolved in methylene chloride (1.3 g), 1-ethyl-3-(3-dimethylaminopropyl)carbodiimide hydrochloride (28 mg, 0.15 mmol) was added thereto under ice-cooling and the mixture was stirred for 30 minutes. The obtained reaction mixture was diluted with chloroform, and then, washed with 10% by mass aqueous ammonium chloride solution and 5% by mass aqueous sodium hydrogen carbonate solution in this order. The obtained organic layer was concentrated and purified by silica gel column chromatography to obtain Fmoc-Phe-Phe-Phe-OSi(tBu)₂(CH₂TMS) (99 mg, Yield: 100%) as a white solid.
   MASS (ESI+) m/z; 822.5 (M+H)+

### Synthetic Example 72: Synthesis of H-Phe-Phe-Phe-Phe-OSi(tBu)₂(CH₂TMS)

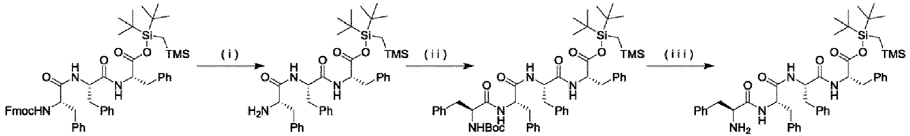

(i) Fmoc-Phe-Phe-Phe-OSi(tBu)₂(CH₂TMS) (99 mg, 0.12 mmol) was mixed with methylene chloride (2.0 g), diethylamine (0.18 g, 2.5 mmol) was added thereto at room temperature and the mixture was stirred at 40°C for 3 hours. After cooling to room temperature, the mixture was diluted with chloroform, 10% by mass aqueous ammonium chloride solution (3 mL) was added thereto and the liquids were separated. The obtained organic layer was concentrated and purified by silica gel column chromatography to obtain H-Phe-Phe-Phe-OSi(tBu)₂(CH₂TMS) (74 mg, Yield: 89%) as a white solid.
   MASS (ESI+) m/z; 688.5 (M+H)+
(ii) H-Phe-Phe-Phe-OSi(tBu)₂(CH₂TMS) (55 mg, 0.08 mmol) and Boc-Phe-OH (25 mg, 0.094 mmol) were dissolved in methylene chloride (1.1 g), 1-ethyl-3-(3-dimethylaminopropyl)carbodiimide hydrochloride (19 mg, 0.099 mmol) was added thereto under ice-cooling and the mixture was stirred for 30 minutes. The obtained reaction mixture was diluted with chloroform and washed with 5% by mass aqueous sodium hydrogen carbonate solution and water in this order. The obtained organic layer was concentrated and purified by silica gel column chromatography to obtain Boc-Phe-Phe-Phe-Phe-OSi(tBu)₂(CH₂TMS) (74 mg, Yield: 99%) as a white solid. MASS (ESI+) m/z; 935.7 (M+H)+
(iii) Boc-Phe-Phe-Phe-Phe-OSi(tBu)₂(CH₂TMS) (74 mg, 0.079 mmol) was dissolved in methylene chloride (1.5 g), 15% by mass hydrogen chloride-1,4-dioxane (0.74 g) was added thereto under ice-cooling and the mixture was stirred at room temperature for 4 hours. The obtained reaction mixture was diluted with chloroform and washed with 5% by mass aqueous sodium hydrogen carbonate solution and water in this order. The obtained organic layer was concentrated to obtain H-Phe-Phe-Phe-Phe-OSi(tBu)₂(CH₂TMS) (65 mg, Yield: 98%) as a white solid.
   MASS (ESI+) m/z; 835.6 (M+H)+

### Test Example 1: Comparison of protection reaction at C-terminus due to difference in silylating agent

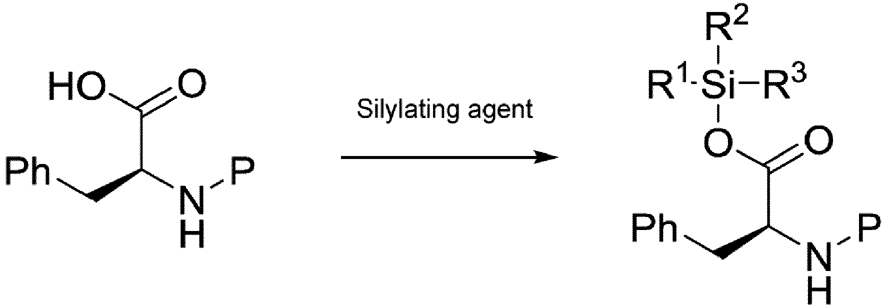

### [Test compounds]

As the silylating agent, trimethylsilyl chloride (TMS-C1), t-butyldimethylsilyl chloride (TBS-C1) and di-t-butylisobutylsilyltriflate (BIBS-OTf) were used.

As the N-protected amino acid, commercially available Boc-Phe-OH, Fmoc-Phe-OH and Cbz-Phe-OH were used.

### [Test method]

N-protected amino acid and imidazole (1.5 equivalents) were mixed with methylene chloride (20-fold by mass), the mixture was cooled to 0°C, a silylating agent (1.2 equivalents) was added dropwise thereto and the mixture was stirred at room temperature for 3 hours. The obtained reaction mixture was washed with an aqueous saturated ammonium chloride solution and water, the obtained organic layer was concentrated, and the presence or absence of formation of the objective material (crude material) was confirmed by ¹H-NMR and LC-MS. Further, a compound which was capable of carrying out was purified by silica gel column chromatography.

### [Test results]

When TMS-C1 was used, formation of the objective material could not be confirmed in either of the N-protected amino acids. In addition, when TBS-C1 was used, formation of the objective material could be confirmed as the crude material in any of the N-protected amino acids, but in the subsequent silica gel column chromatography, the objective material was decomposed. On the other hand, when BIBS-OTf was used, the objective material could be obtained with good yield in any of the N-protected amino acids.

**[Table 1]**

| | | | |
|---|---|---|---|
| Silylating agent | TMS-Cl | TBS-Cl | BIBS-OTf |
| Objective material | Boc-Phe-OTMS | Boc-Phe-OTBS | Boc-Phe-OBIBS |
| With or without purification | None | None | Done |
| Results | No objective material obtained | Objective material was obtained as crude material but decomposed by silica gel column chromatography | Yield: 89% |

**[Table 2]**

| | | | |
|---|---|---|---|
| Silylating agent | TMS-Cl | TBS-Cl | BIBS-OTf |
| Objective material | Cbz-Phe-OTMS | Cbz-Phe-OTBS | Cbz-Phe-OBIBS |
| With or without purification | None | None | Done |
| Results | No objective material obtained | Objective material was obtained as crude material but decomposed by silica gel column chromatography | Yield: 91% |

**[Table 3]**

| | | | |
|---|---|---|---|
| Silylating agent | TMS-Cl | TBS-Cl | BIBS-OTf |
| Objective material | Fmoc-Phe-OTMS | Fmoc-Phe-OTBS | Fmoc-Phe-OBIBS |
| With or without purification | None | None | Done |
| Results | No objective material obtained | Objective material was obtained as crude material but decomposed by silica gel column chromatography | Yield: 99% |

### Boc-Phe-OTBS

¹H-NMR (CDCl₃)
δ ppm: 0.25 (3H, s), 0.26 (3H, s), 0.91 (9H, s), 1.42 (9H, s), 3.03-3.17 (2H, m), 4.52-4.58 (1H, m), 4.98 (1H, d, J=8.1Hz), 7.13-7.31 (10H, m)
MASS (ESI+) m/z; 380.22 (M+H)+

### Boc-Phe-OBIBS

¹H-NMR (CDCl₃)
δ ppm: 0.86 (2H, d, J=6.6Hz), 0.96 (3H, d, J=6.6Hz), 0.97 (3H, d, J=6.6Hz), 1.04 (9H, s), 1.05 (9H, s), 1.38 (9H, s), 1.94-2.07 (1H, m), 2.98 (1H, dd, J=7.4, 14.0Hz), 3.22 (1H, dd, J=5.5, 14.0Hz), 4.53-4.60 (1H, m), 4.93 (1H, d, J=8.5Hz), 7.18-7.31 (10H, m)
MASS (ESI+) m/z; 464.31 (M+H)+

### Cbz-Phe-OTBS

¹H-NMR (CDCl₃)
δ ppm: 0.25 (3H, s), 0.26 (3H, s), 0.90 (9H, s), 3.01-3.20 (2H, m), 4.59-4.66 (1H, m), 5.09 (2H, d, J=2.9Hz), 5.22 (1H, d, J=8.1Hz), 7.10-7.39 (10H, m)
MASS (ESI+) m/z; 414.3 (M+H)+

### Cbz-Phe-OBIBS

¹H-NMR (CDCl₃)
δ ppm: 0.87 (2H, d, J=6.6Hz), 0.96 (3H, d, J=6.6Hz), 0.97 (3H, d, J=6.3Hz), 1.04 (9H, s), 1.05 (9H, s), 1.94-2.01 (1H, m), 3.01 (1H, dd, J=7.4, 14.0Hz), 3.25 (1H, dd, J=5.2, 14.0Hz), 4.60-4.65 (1H, m), 5.06 (2H, d, J=2.9Hz), 5.15 (1H, d, J=8.5Hz), 7.15-7.37

### (10H, m)

MASS (ESI+) m/z; 498.5 (M+H)+

### Fmoc-Phe-OTBS

¹H-NMR (CDCl₃)
δ ppm: 0.27 (3H, s), 0.27 (3H, s), 0.92 (9H, s), 3.01-3.22 (2H, m), 4.21 (1H, t, J=7.0Hz), 4.31 (1H, dd, J=7.0, 10.3Hz), 4.44 (1H, dd, J=7.0, 10.3Hz), 4.61-4.68 (1H, m), 5.27 (1H, d, J=8.1Hz), 7.12 (2H, dd, J=1.8, 7.7Hz), 7.24-7.33 (5H, m), 7.40 (2H, t, J=7.4Hz), 7.57 (2H, dd, J=3.7, 7.4Hz), 7.76 (2H, d, J=7.7Hz)
MASS (ESI+) m/z; 502.3 (M+H)+

### Fmoc-Phe-OBIBS

¹H-NMR (CDCl₃)
δ ppm: 0.87 (2H, d, J=6.6Hz), 0.97 (3H, d, J=6.6Hz), 0.98 (3H, d, J=6.6Hz), 1.05 (9H, s), 1.06 (9H, s), 1.95-2.08 (1H, m), 3.04 (1H, dd, J=7.0, 14.0Hz), 3.29 (1H, dd, J=5.5, 14.0Hz), 4.18 (1H, t, J=7.0Hz), 4.26 (1H, dd, J=7.4, 10.3Hz), 4.39 (1H, dd, J=7.4, 10.3Hz), 4.61-4.68 (1H, m), 5.23 (1H, d, J=8.1Hz), 7.18-7.31 (7H, m), 7.39 (2H, t, J=7.4Hz), 7.53 (2H, t, J=7.0Hz), 7.75 (2H, d, J=7.0Hz)
MASS (ESI+) m/z; 586.4 (M+H)+

### Test Example 2: Comparison of deprotection reaction of protective group at N-terminus due to difference in silyl protective group

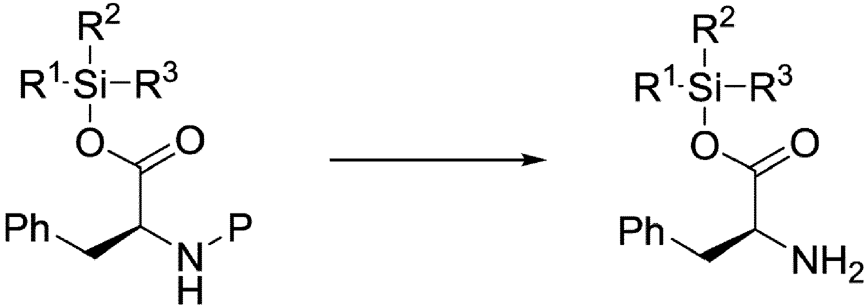

### [Test compounds]

The protected amino acids (Boc-Phe-OTBS, Boc-Phe-OBIBS, Cbz-Phe-OTBS, Cbz-Phe-OBIBS, Fmoc-Phe-OTBS and Fmoc-Phe-OBIBS) synthesized in Test Example 1 were used as the test compounds.

### [Test Method 1: Deprotection reaction of Boc group]

Boc-Phe-OTBS and Boc-Phe-OBIBS were each mixed with methylene chloride (20-fold by mass), the mixture was cooled to 0°C, 15% by mass hydrogen chloride-1,4-dioxane (10-fold by mass) was added thereto and the mixture was stirred at room temperature for 5 to 7 hours. The obtained reaction mixture was diluted with chloroform, and washed with a saturated aqueous sodium carbonate solution and water. The obtained organic layer was concentrated, and the presence or absence of formation of the objective material was confirmed by ¹H-NMR and LC-MS.

### [Test Results 1]

In the case of Boc-Phe-OTBS, formation of the objective material could not be confirmed. On the other hand, in Boc-Phe-OBIBS, the objective material could be obtained with good yield.

**[Table 4]**

| | | |
|---|---|---|
| Protected amino acid | Boc-Phe-OTBS | Boc-Phe-OBIBS |
| Objective material | H-Phe-OTBS | H-Phe-OBIBS |
| With or without purification | None | None |
| Results | No objective material could be obtained | Yield: 100% |

### H-Phe-OBIBS

¹H-NMR (CDCl₃)
δ ppm: 0.89 (2H, d, J=7.7Hz), 0.99 (6H, d, J=6.6Hz), 1.07 (9H, s), 1.07 (9H, s), 1.98-2.11 (1H, m), 2.74 (1H, dd, J=9.2, 13.3Hz), 3.24 (1H, dd, J=4.4, 13.6Hz), 3.71 (1H, dd, J=4.4, 9.2Hz), 7.19-7.34 (5H, m)
MASS (ESI+) m/z; 364.3 (M+H)+

### [Test Method 2: Deprotection reaction of Cbz group]

Cbz-Phe-OTBS and Cbz-Phe-OBIBS were each mixed with 2,2,2-trifluoroethanol (20-fold by mass), 10% by mass Pd-C (0.2-fold by mass) was added thereto and the mixture was stirred under a hydrogen gas atmosphere at room temperature for 24 hours. The reaction mixture was filtered and the obtained filtrate was concentrated, and the presence or absence of formation of the objective material was confirmed by ¹H-NMR and LC-MS.

### [Test Results 2]

In the case of Cbz-Phe-OTBS, formation of the objective material could not be confirmed. On the other hand, in Cbz-Phe-OBIBS, the objective material could be obtained with good yield.

**[Table 5]**

| | | |
|---|---|---|
| Protected amino acid | Cbz-Phe-OTBS | Cbz-Phe-OBIBS |
| Objective material | H-Phe-OTBS | H-Phe-OBIBS |
| With or without purification | None | None |
| Results | No objective material could be obtained | Yield: 100% |

Incidentally, ¹H-NMR and LC-MS of H-Phe-OBIBS in Test Results 2 were accorded with ¹H-NMR and LC-MS in Test Results 1.

### [Test Method 3: Deprotection reaction of Fmoc group]

Fmoc-Phe-OTBS and Fmoc-Phe-OBIBS were each mixed with methylene chloride (20-fold by mass), the mixture was cooled to 0°C, diethylamine (15-fold by mass) was added thereto and the mixture was stirred at room temperature for 5 to 7 hours. The obtained reaction mixture was diluted with chloroform, and washed with an aqueous saturated ammonium chloride solution and water. The obtained organic layer was concentrated and purified by silica gel column chromatography, and the presence or absence of formation of the objective material was confirmed by ¹H-NMR and LC-MS.

### [Test Results 3]

In the case of Fmoc-Phe-OTBS, formation of the objective material could not be confirmed. On the other hand, in Fmoc-Phe-OBIBS, the objective material could be obtained with good yield.

**[Table 6]**

| | | |
|---|---|---|
| Protected amino acid | Fmoc-Phe-OTBS | Fmoc-Phe-OBIBS |
| Objective material | H-Phe-OTBS | H-Phe-OBIBS |
| With or without purification | None | None |
| Results | No objective material could be obtained | Yield: 87% |

Incidentally, ¹H-NMR and LC-MS of H-Phe-OBIBS in Test Results 3 were accorded with ¹H-NMR and LC-MS in Test Results 1.

### Test Example 3: Comparison of condensing step of each N-protected amino acid and H-Phe-OBIBS

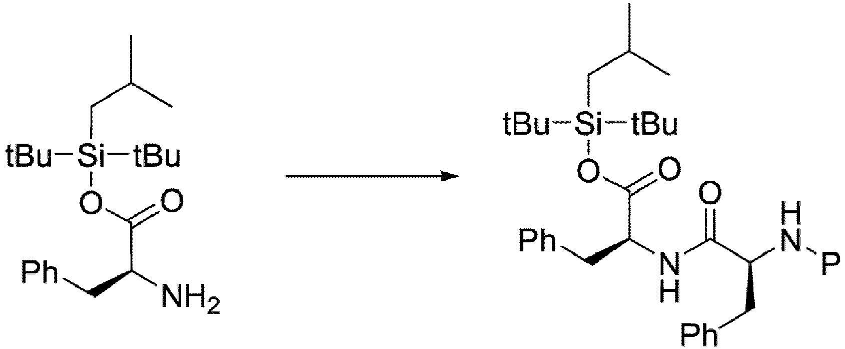

### [Test compounds]

H-Phe-OBIBS synthesized in Test Example 2 and the N-protected amino acids (Boc-Phe-OH, Fmoc-Phe-OH and Cbz-Phe-OH) were used.

### [Test method]

H-Phe-OBIBS and each N-protected amino acid (1.2 equivalents) were mixed with methylene chloride (20-fold by mass), the mixture was cooled to 0°C, 1-ethyl-3-(3-dimethylaminopropyl)carbodiimide hydrochloride (1.2 equivalents) were added thereto and the mixture was stirred for 3 hours. The obtained reaction mixture was diluted with chloroform, and then, washed with an aqueous saturated sodium hydrogen carbonate solution, an aqueous saturated ammonium chloride solution and water in this order. The obtained organic layer was concentrated and purified by silica gel column chromatography, and the presence or absence of formation of the objective material was confirmed by ¹H-NMR and LC-MS.

### [Test results]

In each N-protected amino acid, the objective material could be obtained with good yield.

**[Table 7]**

| | | | |
|---|---|---|---|
| N-Protected amino acid | Boc-Phe-OH | Cbz-Phe-OH | Fmoc-Phe-OH |
| Objective material | Boc-Phe-Phe-OBIBS | Cbz-Phe-Phe-OBIBS | Fmoc-Phe-Phe-OBI BS |
| With or without purification | Done | Done | Done |
| Results | Yield: 93% | Yield: 86% | Yield: 91% |

### Boc-Phe-Phe-OBIBS

¹H-NMR (CDCl₃)
δ ppm: 0.85 (2H, d, J=6.6Hz), 0.97 (6H, d, J=6.6Hz), 1.03 (9H, s), 1.04 (9H, s), 1.39 (9H, s), 1.93-2.06 (1H, m), 2.96-3.09 (3H, m), 3.22 (1H, dd, J=5.5, 14.0Hz), 4.28-4.34
(1H, m), 4.72-4.79 (1H, m), 4.86 (1H, brs), 6.34 (1H, d, d=8.5Hz), 7.05-7.08 (2H, m), 7.14-7.29 (8H, m)
MASS (ESI+) m/z; 611.38 (M+H)+

### Cbz-Phe-Phe-OBIBS

¹H-NMR (CDCl₃)
δ ppm: 0.86 (2H, d, J=6.6Hz), 0.97 (6H, d, J=6.3Hz), 1.04 (9H, s), 1.05 (9H, s), 1.94-2.07 (1H, m), 2.92-3.01 (3H, m), 3.23 (1H, dd, J=5.5, 14.0Hz), 4.35-4.42 (1H, m), 4.72-4.79 (1H, m), 5.06 (1H, s), 5.14 (1H, d, d=7.4Hz), 6.28 (1H, d, J=7.4Hz), 7.03-7.38 (15H, m)
MASS (ESI+) m/z; 645.5 (M+H)+

### Fmoc-Phe-Phe-OBIBS

¹H-NMR (CDCl₃)
δ ppm: 0.86 (2H, d, J=6.7Hz), 0.97 (6H, d, J=6.4Hz), 1.04 (9H, s), 1.05 (9H, s), 1.93-2.04 (1H, m), 2.92-3.05 (3H, m), 3.23 (1H, dd, J=5.5, 14.1Hz), 4.17 (1H, t, J=7.0Hz), 4.28 (1H, m), 4.38 (1H, m), 4.43 (dd, J=6.7, 10.4Hz), 4.75 (1H, dd, J=7.0, 13.2Hz), 5.15 (1H, d, J=8.0Hz), 6.24 (1H, d, J=7.0Hz), 7.01-7.04 (2H, m), 7.13-7.33 (10H, m), 7.40 (2H, t, J=7.7Hz), 7.45-7.54 (2H, m), 7.77 (2H, d, J=7.7Hz)
MASS (ESI+) m/z; 733.6 (M+H)+

### UTILIZABLE FIELD IN INDUSTRY

According to the present invention, a method for producing a peptide with high efficiency can be provided.

## Claims

1. A method for producing an amino acid or a peptide which comprises a step of removing a protective group at an N-terminus of an amino acid or a peptide represented by the formula (I):
P-AA-O-SiR¹R²R³ (I)
wherein, AA represents a group derived from an amino acid or a peptide, P represents a protective group at the N-terminus, R¹R²R³Si represents a protective group at the C-terminus,
R¹, R² and R³ each independently represent an aliphatic hydrocarbon group which may have a substituent(s), an aromatic hydrocarbon group which may have a substituent(s) or -OR⁴, where R⁴ represents an aliphatic hydrocarbon group which may have a substituent(s) or an aromatic hydrocarbon group which may have a substituent(s),
two of R¹, R² and R³ may form a 5- to 7-membered ring together with the Si atom to which they are bonded, and
a total number of the carbon atoms in the R¹R²R³Si group is 8 or more.

2. A method for producing a peptide which comprises the following steps (1) and (2):
(1) a step of condensing an N-protected amino acid or an N-protected peptide to an N-terminus of a C-protected amino acid or a C-protected peptide represented by the formula (II): 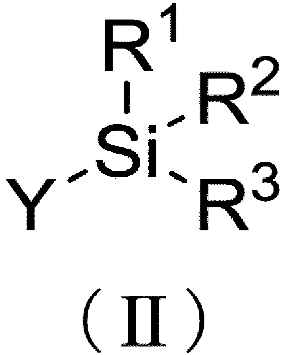 wherein,
Y represents an amino acid an N-terminus of which is unprotected or a peptide an N-terminus of which is unprotected,
R¹, R² and R³ each independently represent an aliphatic hydrocarbon group which may have a substituent(s), an aromatic hydrocarbon group which may have a substituent(s) or -OR⁴, where R⁴ represents an aliphatic hydrocarbon group which may have a substituent(s) or an aromatic hydrocarbon group which may have a substituent(s),
two of R¹, R² and R³ may form a 5- to 7-membered ring together with the Si atom to which they are bonded,
a total number of the carbon atoms in the R¹R²R³Si group is 8 or more, and the R¹R²R³Si group is bonded to a C-terminus of an amino acid or a peptide in Y; and
(2) a step of removing the protective group at the N-terminus of the peptide obtained in the step (1).

3. The method for producing a peptide according to Claim 2, which comprises the following steps (1) and (2):
(1) a step of condensing an N-protected amino acid or an N-protected peptide to an N-terminus of a C-protected amino acid or a C-protected peptide represented by the formula (II): 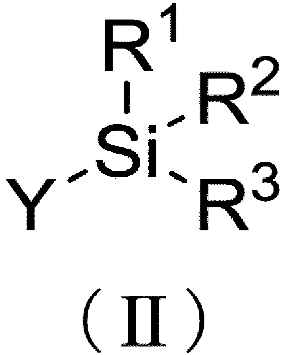 wherein,
Y represents an amino acid an N-terminus of which is unprotected or a peptide an N-terminus of which is unprotected,
R¹, R² and R³ each independently represent an aliphatic hydrocarbon group which may have a substituent(s),
a total number of the carbon atoms in the R¹R²R³Si group is 8 or more, and
the R¹R²R³Si group is bonded to a C-terminus of an amino acid or a peptide in Y;
(2) a step of removing the protective group at the N-terminus of the peptide obtained in the step (1).

4. A method for producing a peptide which comprises the following steps (1) to (3):
(1) a step of condensing an N-protected amino acid or an N-protected peptide to an N-terminus of a C-protected amino acid or a C-protected peptide represented by the formula (II): 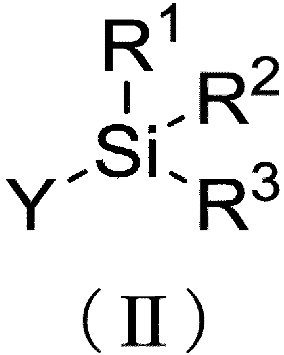 wherein,
Y represents an amino acid an N-terminus of which is unprotected or a peptide an N-terminus of which is unprotected,
R¹, R² and R³ each independently represent an aliphatic hydrocarbon group which may have a substituent(s), an aromatic hydrocarbon group which may have a substituent(s) or -OR⁴, where R⁴ represents an aliphatic hydrocarbon group which may have a substituent(s) or an aromatic hydrocarbon group which may have a substituent(s),
two of R¹, R² and R³ may form a 5- to 7-membered ring together with the Si atom to which they are bonded,
a total number of the carbon atoms in the R¹R²R³Si group is 8 or more, and
the R¹R²R³Si group is bonded to a C-terminus of an amino acid or a peptide in Y;
(2) a step of removing the protective group at the N-terminus of the peptide obtained in the step (1);
(3) a step of condensing an N-protected amino acid or an N-protected peptide to the N-terminus of the peptide obtained in the step (2).

5. The method for producing a peptide according to Claim 4, which comprises the following steps (1) to (3):
(1) a step of condensing an N-protected amino acid or an N-protected peptide to an N-terminus of a C-protected amino acid or a C-protected peptide represented by the formula (II): 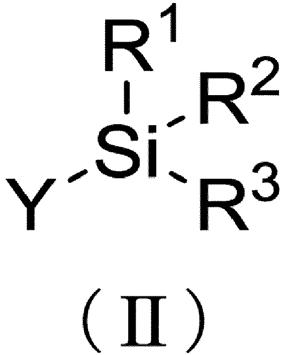 wherein,
Y represents an amino acid an N-terminus of which is unprotected or a peptide an N-terminus of which is unprotected,
R¹, R² and R³ each independently represent an aliphatic hydrocarbon group which may have a substituent(s),
a total number of the carbon atoms in the R¹R²R³Si group is 8 or more, and
the R¹R²R³Si group is bonded to a C-terminus of an amino acid or a peptide in Y;
(2) a step of removing the protective group at the N-terminus of the peptide obtained in the step (1);
(3) a step of condensing an N-protected amino acid or an N-protected peptide to the N-terminus of the peptide obtained in the step (2).

6. The method for producing a peptide according to Claim 4 or 5, which comprises a step of removing the protective group at the C-terminus of the peptide obtained in the step (3).

7. The method for producing a peptide according to Claim 4 or 5, which further comprises one or more repeating of the following steps (4) and (5):
(4) a step of removing the protective group at the N-terminus of the peptide obtained in the step (3) or the step (5).
(5) a step of condensing an N-protected amino acid or an N-protected peptide to an N-terminus of the peptide obtained in the step (4).

8. The method for producing a peptide according to Claim 7, which comprises a step of removing the protective group at the C-terminus of the peptide obtained in the step (5).

9. The producing method according to any one of Claims 1 to 8, wherein a total number of the carbon atoms in the R¹R²R³Si group is 10 to 100.

10. The producing method according to any one of Claims 1 to 8, wherein a total number of the carbon atoms in the R¹R²R³Si group is 10 to 40.

11. The producing method according to any one of Claims 1 to 10, wherein among R¹, R² and R³, two or three of them each independently represent a secondary or tertiary aliphatic hydrocarbon group.

12. The producing method according to Claim 11, wherein among R¹, R² and R³, two of them each independently represent a secondary aliphatic hydrocarbon group, and the remaining one represents a secondary or tertiary aliphatic hydrocarbon group which may have a substituent(s) and a group different from the above two.

13. The producing method according to Claim 11, wherein among R¹, R² and R³, two of them each independently represent a tertiary aliphatic hydrocarbon group.

14. The producing method according to any one of Claims 1 to 10, wherein among R¹, R² and R³, two or three of them each independently represent a secondary or tertiary C₃₋₆ alkyl group or a C₃₋₆ cycloalkyl group.

15. The producing method according to Claim 14, wherein among R¹, R² and R³, two of them each independently represent a secondary C₃₋₆ alkyl group or a C₃₋₆ cycloalkyl group, and the remaining one represents a secondary or tertiary C₃₋₆ alkyl group or a C₃₋₆ cycloalkyl group, which may have a substituent(s), and is different from the above two.

16. The producing method according to Claim 14, wherein among R¹, R² and R³, two of them each independently represent a tertiary C₄₋₆ alkyl group.

17. The producing method according to any one of Claims 1 to 10, wherein among R¹, R² and R³, two or three of them each independently represent a t-butyl group, an i-propyl group, an s-butyl group, a cyclopentyl group or a cyclohexyl group.

18. The producing method according to Claim 17, wherein among R¹, R² and R³, one of them represents a t-butyl group or a cumyl group, and the remaining two are each independently represent a t-butyl group, an i-propyl group, an s-butyl group, a cyclopentyl group or a cyclohexyl group.

19. The producing method according to Claim 17, wherein among R¹, R² and R³, two of them are t-butyl groups, and the remaining one is an i-butyl group, a benzyl group, an octadecyl group or a (trimethylsilyl)methyl group.

20. The producing method according to any one of Claims 1 to 10, wherein the R¹R²R³Si group is a di-s-butyl-t-butylsilyl group, a di-t-butylisobutylsilyl group, a dit-butyloctadecylsilyl group, a benzyl-di-t-butylsilyl group, a tri-t-butylsilyl group, a di-i-propyl-t-butylsilyl group, a di-i-propylcumylsilyl group, a di-cyclopentyl-cumylsilyl group, a di-cyclohexylcumylsilyl group, a di-s-butylcumylsilyl group or a di-t-butyl { (trimethylsilyl)methyl}silyl group.

21. The producing method according to any one of Claims 1, 2, 4 and 6 to 10, wherein the R¹R²R³Si group is a di-t-butylphenethoxysilyl group or a di-t-butylphenylsilyl group.

22. The producing method according to any one of Claims 1, 2, 4 and 6 to 10, wherein R¹, R² and R³ are -OR⁴, where R⁴ represents a tertiary aliphatic hydrocarbon group.

23. The producing method according to any one of Claims 1, 2, 4 and 6 to 10, wherein the R¹R²R³Si group is a tri-t-butoxysilyl group.

24. The producing method according to any one of Claims 1 to 23, wherein the amino acid or the peptide is constituted by an α-amino acid.

25. The producing method according to any one of Claims 1 to 24, wherein the protective group at the N-terminus of the N-protected amino acid or the N-protected peptide is a carbamate protective group.

26. The producing method according to any one of Claims 1 to 24, wherein the protective group at the N-terminus of the N-protected amino acid or the N-protected peptide is a benzyloxycarbonyl group, a 9-fluorenylmethoxycarbonyl group or a t-butoxycarbonyl group.

27. Use of a group represented by the following formula (III): 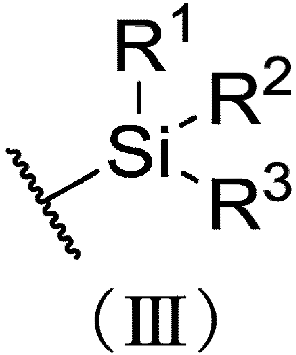 wherein, R¹, R² and R³ each independently represent an aliphatic hydrocarbon group which may have a substituent(s), an aromatic hydrocarbon group which may have a substituent(s) or -OR⁴, where R⁴ represents an aliphatic hydrocarbon group which may have a substituent(s) or an aromatic hydrocarbon group which may have a substituent(s), two of R¹, R² and R³ may form a 5- to 7-membered ring together with the Si atom to which they are bonded, a total number of the carbon atoms in the R¹R²R³Si group is 8 or more, and a wavy line is a bonding position to a residue at the C-terminus of the amino acid or the peptide,
as a protective group of a C-terminus of an amino acid or a peptide in an N-terminal elongation reaction of a peptide.
